# FASCICULE DE BREVET EUROPEEN

(11) **EP 3 824 286 B1**
(45) Date de publication et mention de la délivrance du brevet: **24.12.2025**
(21) Numéro de dépôt: 19761730.1
(22) Date de dépôt: 17.07.2019
(51) Int. Cl.: G01N 33/558, G01N 33/58, G01N 21/64, C09K 11/77, G01N 33/543

(54) **TEST À DIFFUSION CAPILLAIRE METTANT EN OEUVRE DES NANOPARTICULES INORGANIQUES PHOTOLUMINESCENTES**
PRÜFUNG DER KAPILLARWIRKUNG UNTER VERWENDUNG VON PHOTOLUMINESZENTEN ANORGANISCHEN NANOPARTIKELN
CAPILLARY ACTION TEST USING PHOTOLUMINESCENT INORGANIC NANOPARTICLES

(30) Priorité: 18.07.2018 FR 1856651
(43) Date de publication de la demande: 26.05.2021
(73) Titulaire: Ecole Polytechnique, 91128 Palaiseau Cedex (FR); Centre National de la Recherche Scientifique, 75794 Paris Cedex 16 (FR)
(72) Inventeur: PREIRA, Pascal, 91600 SAVIGNY-SUR-ORGE (FR); RICHLY, Maximilian, 75006 PARIS (FR); BOUZIGUES, Cédric, 75016 PARIS (FR); ALEXANDROU, Antigoni, 91120 PALAISEAU (FR); GACOIN, Thierry, 91440 BURES SUR YVETTE (FR)
(74) Mandataire: Ipsilon
(86) Numéro de dépôt international: PCT/EP2019/069263
(87) Numéro de publication internationale: WO 2020/016308

(56) Documents cités:
- WO-A1-2019/025618
- JP-A- 2012 032 263
- MOUNA ABDESSELEM ET AL: "Multifunctional Rare-Earth Vanadate Nanoparticles: Luminescent Labels, Oxidant Sensors, and MRI Contrast Agents", ACS NANO, vol. 8, no. 11, 25 November 2014 (2014-11-25), US, pages 11126 - 11137, XP055275081, ISSN: 1936-0851, DOI: 10.1021/nn504170x
- CASANOVA DIDIER ET AL: "Optical in situ size determination of single lanthanide-ion doped oxide nanoparticles", APPLIED PHYSICS LETTERS, A I P PUBLISHING LLC, US, vol. 89, no. 25, 18 December 2006 (2006-12-18), pages 253103 - 253103, XP012087778, ISSN: 0003-6951, DOI: 10.1063/1.2405871
- LUO MIN ET AL: "Synthesis of carboxyl-capped and bright YVO4:Eu,Bi nanoparticles and their applications in immunochromatographic test strip assay", MATERIALS RESEARCH BULLETIN, vol. 48, no. 11, 29 July 2013 (2013-07-29), pages 4454 - 4459, XP028730320, ISSN: 0025-5408, DOI: 10.1016/J.MATERRESBULL.2013.07.051
- CEDRIC BOUZIGUES ET AL: "Biological Applications of Rare-Earth Based Nanoparticles", ACS NANO, vol. 5, no. 11, 22 November 2011 (2011-11-22), US, pages 8488 - 8505, XP055587451, ISSN: 1936-0851, DOI: 10.1021/nn202378b
- ANDREW S. PATERSON ET AL: "Persistent Luminescence Strontium Aluminate Nanoparticles as Reporters in Lateral Flow Assays", ANALYTICAL CHEMISTRY, vol. 86, no. 19, 23 September 2014 (2014-09-23), US, pages 9481 - 9488, XP055427256, ISSN: 0003-2700, DOI: 10.1021/ac5012624
- SILVAN TRKCAN ET AL: "Observing the Confinement Potential of Bacterial Pore-Forming Toxin Receptors Inside Rafts with Nonblinking Eu-Doped Oxide Nanoparticles", BIOPHYSICAL JOURNAL, ELSEVIER, AMSTERDAM, NL, vol. 102, no. 10, 23 March 2012 (2012-03-23), pages 2299 - 2308, XP028488196, ISSN: 0006-3495, [retrieved on 20120407], DOI: 10.1016/J.BPJ.2012.03.072

## Description

La présente invention concerne le domaine de la bioanalyse et du diagnostic *in vitro.* Elle a plus particulièrement pour objet un procédé *in vitro* de détection et/ou de quantification d'une substance d'intérêt biologique ou chimique, par exemple de protéines, d'anticorps, de toxines et autres composés, dans un échantillon liquide, par un test à diffusion capillaire mettant en œuvre, à titre de sondes, des nanoparticules inorganiques photoluminescentes à propriétés optiques et physico-chimiques contrôlées.

Les tests à diffusion capillaire, tels que par exemple les tests à flux latéral (LFA ou « Lateral-flow assays » en langue anglaise), plus connus sous la dénomination « tests sur bandelettes », sont couramment utilisés aux fins d'analyses cliniques, pharmaceutiques, alimentaires ou chimiques. Ils peuvent être employés pour détecter la présence de nombreux types d'analytes tels que des anticorps, des antigènes, des protéines, des biomarqueurs, des molécules chimiques, des acides nucléiques, etc. ([1]). Quand les molécules de reconnaissance mises en œuvre dans le test à flux latéral sont des anticorps, on parle plus communément de test immunochromatographique (« Lateral Flow Immunoassay » (LFIA)).

Les tests à diffusion capillaire sont particulièrement appréciés pour leur simplicité d'utilisation, leur rapidité (détection dans une durée inférieure ou égale à 15 minutes) et leur faible coût.

D'une manière générale, les dispositifs de test à diffusion capillaire mettent en œuvre un moyen de diffusion capillaire sous la forme d'un support solide poreux (par exemple une membrane de nitrocellulose), au sein duquel l'échantillon à tester, déposé au niveau de l'une des extrémités du support solide et les réactifs, intégrés au niveau du dispositif commercialisé, migrent par diffusion capillaire.

Typiquement, le support solide poreux des dispositifs de test à diffusion capillaire comporte une zone de marquage (« Conjugate Pad » en terminologie anglo-saxonne) portant, sous forme liquide, lyophilisée ou déshydratée, un réactif de liaison spécifique de la substance à analyser (ou « analyte »), conjugué à une sonde (ou espèce révélatrice), et une zone de détection (« Detection Pad » en terminologie anglo-saxonne) sur laquelle est immobilisé un réactif de capture spécifique de l'analyte.

Le réactif de liaison spécifique de l'analyte est immobilisé sous forme lyophilisée mais devient mobile dans le support solide à l'état humide. Ainsi, lorsque le support solide est mis en contact avec un échantillon liquide, ce dernier migre par diffusion capillaire dans ce support entraînant le réactif de liaison spécifique de l'analyte conjugué à la sonde. L'échantillon et le réactif de liaison spécifique de l'analyte migrent par diffusion capillaire dans le support solide jusqu'à la zone de détection portant un réactif de capture immobilisé, spécifique de l'analyte.

Dans les tests à diffusion capillaire les plus courants, de type « sandwich », le réactif de liaison conjugué à la sonde se lie à l'analyte contenu dans l'échantillon, lorsque ceux-ci se rencontrent, puis l'analyte est immobilisé sur le support solide par le réactif de capture. La présence ou l'absence de l'analyte dans l'échantillon est ainsi mesurée *via* la détection de la sonde immobilisée au niveau de la zone de détection par l'intermédiaire de l'analyte.

Ces dispositifs comportent également une zone dite de contrôle, située en aval de la zone de détection par rapport à la direction de diffusion capillaire, dans laquelle est immobilisé un deuxième réactif de capture spécifique du réactif de ciblage marqué. Après avoir migré jusqu'à la zone de détection, le réactif de liaison couplé à la sonde en excès, n'ayant pas réagi avec l'analyte, migre jusqu'à la zone de contrôle, et se lie au deuxième réactif de capture. L'utilisateur dispose ainsi d'un témoin positif permettant de vérifier la migration de l'échantillon et des réactifs dans le dispositif, et donc de vérifier le bon fonctionnement du test.

La détermination de l'analyte dans l'échantillon est donc opérée par la détection de la présence, ou non, de la sonde au niveau de la zone de détection et, éventuellement, de la zone de contrôle.

Les sondes les plus couramment employées dans les tests à diffusion capillaire sont des nanoparticules d'or ([1], [2]). Celles-ci absorbent la lumière à des longueurs d'onde caractéristiques qui correspondent à leur fréquence de plasmon de surface. La fréquence de plasmon de surface des nanoparticules dépend de leur taille et de leur état d'agrégation. Lors de l'immobilisation des nanoparticules d'or au niveau de la zone de détection et/ou de la zone de contrôle, l'absorption liée à la fréquence du plasmon de surface des nanoparticules, qui se retrouvent proches les unes aux autres, permet ainsi de conférer auxdites zones une couleur caractéristique, typiquement bleu/violet, visible à l'œil nu. Ces tests permettent avantageusement d'obtenir rapidement le résultat de l'analyse de l'échantillon, typiquement en quelques minutes, comparativement à la durée requise pour des techniques d'immuno-détection classiques, tel qu'un essai d'immuno-absorption enzymatique (ELISA, pour « Enzyme-Linked ImmunoSorbent Assay » en terminologie anglo-saxonne), typiquement en quelques heures pour les tests ELISA. Egalement, ils ne nécessitent pas d'équipements préparatoires ou analytiques, coûteux et encombrants.

Cependant, ces tests ont l'inconvénient majeur de présenter une sensibilité de détection faible et une limite de quantification moins bonne. Ils fournissent ainsi généralement une information uniquement qualitative, ou semi-quantitative. En particulier, ces tests sur bandelette présentent une sensibilité de détection bien plus faible que celle pouvant être obtenue par des tests d'immuno-détection classiques, par exemple de type ELISA. Ainsi, les tests sur bandelettes à base de nanoparticules d'or permettent typiquement de détecter des concentrations de l'ordre de quelques ng/mL, tandis qu'un test ELISA permet une détection de quelques pg/mL, typiquement 2 à 3 ordres de grandeur plus sensible. Par exemple, la société Cortez Diagnostics propose un test sur bandelette pour la détection de la troponine I (biomarqueur de l'infarctus du myocarde) présentant une sensibilité de 1 ng/ml ([3]), tandis que la société Abcam propose un test ELISA (ab200016) avec une sensibilité de 7 pg/mL.

En vue d'améliorer la sensibilité des tests sur bandelette, autrement dit d'abaisser la limite de détection de ces tests, différents matériaux ont été proposés comme sondes, en alternative des nanoparticules d'or, dans des tests à diffusion capillaire, et notamment des nanoparticules d'or modifiées, des particules magnétiques, des nanocristaux de semi-conducteurs ou « quantum dots », des phosphores à conversion ascendante (« up-conversion »), des fluorophores organiques, etc. ([1], [4]).

Ainsi, plusieurs nanomatériaux à base de nanoparticules d'or ont été explorés comme sondes pour des dispositifs de test à diffusion capillaire, comme par exemple des microsphères magnétiques comprenant un cœur formé d'une particule nanométrique de Fe₂O₃ enrobé de nanoparticules d'or ([5]), des nanotubes de silice portant des nanoparticules d'or ([6]), ou encore des nanofleurs d'or multi-branchées (GNFs) ([7]). On peut encore citer Der-Jiang *et al* ([8]) qui proposent de procéder au dépôt d'une couche d'argent sur les nanoparticules d'or. Cependant, ces approches requièrent des voies de synthèse de ces sondes souvent hautement complexes. Qui plus est, l'argent s'oxydant plus facilement que l'or, les sondes, associant à l'or de l'argent, présentent une stabilité moindre pour une application dans un test à diffusion capillaire en milieu aqueux.

La mise en œuvre de sondes photoluminescentes (également appelées plus simplement par la suite « sondes luminescentes »), telles que des fluorophores organiques et des « quantum dots », a permis d'accroître la sensibilité des tests à diffusion capillaire, comparativement aux tests à base de nanoparticules d'or. De fait, la détection d'une émission de lumière (luminescence) est, en général, plus sensible que la détection d'une absorption (cas des nanoparticules d'or), cette dernière étant réalisée sur le fond élevé de la lumière transmise (par exemple, concernant les fluorophores organiques [9], [10] et [11] ; concernant les QDs : [11]-[17], [50]).

Malheureusement, ces sondes photoluminescentes présentent plusieurs désavantages qui ne permettent pas d'exploiter pleinement leur potentiel en tant que sondes dans les tests sur bandelettes. Parmi ces inconvénients, on peut citer par exemple le phénomène de photoblanchiment dans le cas des fluorophores organiques qui, suite à des modifications structurales irréversibles induites par l'illumination, se traduit par une disparition de la fluorescence, ou encore le phénomène de clignotement de l'émission pour les nanocristaux de semi-conducteurs, ou « quantum dots », les sondes cessant alors périodiquement d'émettre et étant, par conséquent, inadaptées pour produire un signal constant et reproductible. D'autres inconvénients résultent par exemple de la largeur du spectre d'émission des sondes luminescentes. De fait, un spectre d'émission trop large rend difficile le filtrage du signal de fond éventuellement présent, ce qui a une incidence sur la qualité du signal et, en particulier, sur le rapport signal à bruit. Il convient également de prendre en compte, en plus des facteurs optiques qui contribuent à l'efficacité de la sonde dans un test biologique, le caractère pratique et la facilité d'utilisation de la sonde. Ainsi, certaines particules, comme c'est le cas des nanocristaux de semi-conducteurs, perdent leurs caractéristiques de luminescence après congélation, ce qui représente un inconvénient pour le stockage des nanocristaux de semi-conducteurs bio-conjugués. La facilité de couplage des sondes au composé moléculaire permettant de cibler les molécules souhaitées est également un aspect à prendre en considération pour le choix de la sonde adéquate. Ainsi, un certain nombre de particules, dont les nanocristaux de semi-conducteurs, sont synthétisées dans des solvants organiques. Il en découle qu'une utilisation pour des applications biologiques nécessite des étapes supplémentaires de préparation de la surface pour réaliser une dispersion dans l'eau de ces particules, processus qui peut être complexe à mettre en œuvre et peu stable dans le temps ([18]). En effet, les fonctionnalisations de surface utilisées pour les nanocristaux de semi-conducteurs n'impliquent pas de liaisons covalentes avec la surface des nanocristaux. Les molécules de fonctionnalisation peuvent ainsi se détacher et causer la dissociation du réactif de liaison spécifique de l'analyte (anticorps ou autre) du nanocristal qui sert de sonde. Ainsi, la conjugaison de ces nanocristaux aux réactifs de liaison spécifiques de l'analyte peut durer de quelques semaines à quelques mois, selon le type de fonctionnalisation. Cependant, une utilisation commerciale d'un test à diffusion capillaire nécessite une stabilité de l'ordre de deux ans après dépôt des conjugués sonde-réactif de liaison sur la bandelette du test.

Un autre inconvénient de ces sondes luminescentes réside dans le fait que l'excitation, nécessaire à la détection de la luminescence, peut provoquer l'émission de lumière parasite, ce qui a pour conséquence d'augmenter le signal de fond et, par conséquence, de diminuer le rapport signal à bruit. Différentes approches ont été proposées pour éliminer, ou tout au moins diminuer, ce signal d'émission parasite comme, par exemple, la mise en œuvre de nanoparticules luminescentes contenant des chélates ou des complexes d'ions lanthanides, combinée à une détection retardée de la luminescence ; de nanoparticules à conversion ascendante ; ou encore de nanoparticules à luminescence persistante.

Ainsi, des particules luminescentes, chargées en chélates ou complexes de lanthanides, ont déjà été proposées à titre de sondes luminescentes dans des tests à diffusion capillaire.

Par exemple, Zhang *et al.* ([19]) proposent d'utiliser, comme sondes pour la détection de la bactérie *Pantoea stewartii subsp. stewartii (Pss)* dans un test de bandelettes à migration, des nanoparticules de silice chargées avec des chélates de lanthanide (Eu). Il est indiqué que ces sondes permettent d'accéder à une limite de détection 100 fois plus faible que celle pouvant être obtenue avec des tests classiques mettant en œuvre des particules d'or. De même, Xia *et al.* ([20]) utilisent des particules de silice chargées en chélate d'europium dans un test à flux latéral pour la détection d'un antigène de surface de l'hépatite B (HBsAg).

On peut encore citer les publications Liang *et al.* ([21]) et Juntunen *et al.* ([22]), qui proposent d'utiliser des microparticules de polystyrène chargées en chélate d'europium, dans un test à flux latéral, pour la détection de l'alpha-fetoprotéine (AFP) dans un échantillon de sérum, ou encore pour la détection de l'antigène prostatique spécifique (PSA) et de l'albumine de sérum bovin biotinylée (biotine-BSA). Les documents WO 2013/013214 et WO 2014/146215 proposent également la mise en œuvre de nanosphères de polystyrène chargées en chélates de terbium et/ou d'europium à titre de sondes dans une bandelette de test à flux latéral.

Il a également été proposé, dans le document WO 2014/146215, d'exploiter l'émission à durée de vie longue (de l'ordre de 100 µs) de ces nanoparticules contenant des chélates ou complexes d'ions lanthanides, pour mettre en œuvre une détection retardée permettant de s'affranchir des émissions parasites dont la durée de vie est généralement de l'ordre de 1-10 ns.

Cependant, les particules luminescentes chargées en chélates ou complexes de lanthanides, mises en œuvre comme sondes luminescentes, ne contiennent typiquement qu'un unique ion lanthanide par chélate ou complexe. Chaque chélate ou complexe prend une place non négligeable au sein de la nano- ou micro-particule, ce qui limite de façon conséquente le nombre d'ions émetteurs pour une taille de particule donnée. Par exemple, une nanoparticule de 45 nm ne contient que de l'ordre de 1000 chélates et ions émetteurs [47]. Par ailleurs, la synthèse de ce type de particules contenant des complexes ou des chélates d'ions lanthanides comprend au moins deux étapes : la synthèse du complexe ou chélate, puis la synthèse de la particule contenant les chélates. Ainsi, la synthèse de ce type de particules est complexe et, de ce fait, relativement onéreuse. Enfin, la stabilité de ce type de particules a également été mise en cause ([23]).

Ces dernières années, un autre type de nanoparticules luminescentes à base de terres rares a été proposé à titre de sonde luminescente dans des applications en bio-imagerie, et notamment dans des tests à diffusion capillaire : il s'agit de nanoparticules de phosphores à conversion ascendante (« up-conversion »), qui émettent de la lumière visible sous excitation par des sources infra-rouge ou proche-infrarouge (par exemple, [24]-[29]). Dans le cadre de la mise en œuvre de ces nanoparticules à conversion ascendante, deux photons sont absorbés par la nanoparticule avant d'observer l'émission de luminescence, qui correspond au signal détecté. A titre d'exemple, on peut citer la publication de Niedbala *et al.* ([30]) qui propose des bandelettes de test à flux latéral mettant en œuvre des sondes de type UPT (« Up-converting Phosphor Technology »), permettant d'atteindre une sensibilité de détection plus élevée que les tests d'immuno-absorption enzymatique. De telles sondes luminescentes à « up-conversion » sont par exemple mises en œuvre dans le dispositif de test à flux latéral proposé dans le document US 2014/0170674.

Ces phosphores à « up-conversion » ont notamment comme avantage, comparativement aux sondes luminescentes précitées, de présenter une résistance au phénomène de photoblanchiment et un faible niveau de fluorescence parasite causant le bruit de fond. En effet, comme l'excitation se fait à une longueur d'onde plus faible que la longueur d'onde de détection, l'émission, due aux substances annexes contenues dans l'échantillon ou au support solide poreux, est pratiquement inexistante.

Malheureusement, ces phosphores à « up-conversion » présentent l'inconvénient majeur de présenter des rendements quantiques faibles, et leur efficacité décroît considérablement pour des densités de puissance optique de la source d'excitation faibles, la luminescence étant proportionnelle au carré de la densité de puissance de l'excitation. De plus, une zone assez large comportant la bande de test, et éventuellement la bande de contrôle, doit être excitée, ce qui diminue la densité de puissance de l'excitation pour une puissance donnée. Par conséquent, la lecture des tests mettant en œuvre de telles sondes luminescentes nécessite un équipement complexe, combinant des diodes laser pour l'excitation, avec d'autres éléments tels que des lentilles, filtres, photomultiplicateurs, préamplificateurs, etc.

Enfin, des nanoparticules inorganiques émettant une luminescence persistante ont également été proposées pour s'affranchir de la luminescence parasite induite par l'excitation. Ces nanoparticules inorganiques sont formées d'une matrice cristalline contenant des ions lanthanides comme dopants. La particularité des nanoparticules à luminescence persistante réside dans le fait que les dopants introduisent des états piège dans la structure électronique du cristal, et les charges excitées s'y trouvent piégées. Ainsi, l'émission de luminescence par ces nanoparticules ne peut avoir lieu qu'après libération des charges de ces états pièges, libération qui a lieu par activation thermique ([31]). Selon l'énergie de ces états pièges dans la structure électronique, i.e. selon la profondeur du piège, l'activation thermique, et ainsi la durée de vie de l'émission, peut atteindre des heures voire des jours. Il est ainsi possible d'exciter les nanoparticules, puis d'insérer le dispositif de test à diffusion capillaire dans un lecteur adapté après arrêt de l'excitation, et de lire le test en détectant la luminescence en l'absence d'excitation, et donc en l'absence d'émissions parasites. Par exemple, Paterson *et al.* ([32]) ont obtenu une sensibilité de détection significativement améliorée par rapport à celle obtenue avec des nanoparticules d'or (limite de détection environ 10 fois en-dessous de celle obtenue avec des nanoparticules d'or). Cela permet également de s'affranchir de l'utilisation d'un filtre d'émission pour la lecture.

Cependant, ce système présente l'inconvénient de nécessiter une longue durée d'acquisition du signal de l'émission. De fait, l'émission par ces nanoparticules ayant lieu pendant plusieurs minutes, en particulier pendant plusieurs heures, voire des jours, selon le cas, il est nécessaire d'attendre l'équivalent de cette durée de vie pour collecter une fraction non-négligeable du nombre de photons émis. Ainsi, par unité de temps, par exemple par seconde, le nombre de photons émis sera faible, ce qui requiert en conséquence d'allonger la durée de l'acquisition de la luminescence pour atteindre un niveau de sensibilité élevé. Une telle durée d'acquisition est contraire à l'objectif des tests à diffusion capillaire qui est de fournir un diagnostic rapide. Pour contrecarrer ce problème, il est possible d'augmenter la puissance d'excitation ; cela implique cependant un appareillage complexe, ce qui ne permet pas de satisfaire l'exigence d'un système de test compact et peu onéreux.

Enfin, dans une autre variante de test à diffusion capillaire, des nanoparticules YVO₄ codopées à l'europium et au bismuth ont été mises en œuvre ([33]). La présence de bismuth permet de produire un décalage de l'absorption de la matrice YVO₄ qui présente un pic d'absorption à 280 nm due à la transition de transfert de charge O²⁻-V⁵⁺ à l'intérieur des ions vanadate VO₄³⁻, vers le visible, du fait de l'apparition de la transition de transfert de charge Bi³⁺-V⁵⁺, autorisant ainsi l'utilisation de sources d'excitation plus courantes, autour de 350 nm. L'excitation est ensuite transférée vers les ions Eu³⁺. Cette approche présente néanmoins l'inconvénient d'une synthèse complexe de ces nanoparticules. En particulier, il est difficile de réaliser une solution solide homogène de YVO₄ et de BiVO₄ dont les structures cristallines sont différentes ([34]). Cela nécessite de recourir, soit à des synthèses hydrothermales à haute température requérant un appareillage plus complexe (autoclave) ([33]), soit à des processus de murissement ([34]).

La demande JP-A-2012-032263 décrit un kit de dosage fluoroimmunologique utilisant une particule fine fluorescente dont le dopage est réalisé à raison de 0,01 % à 6 % molaire de terre rare.

Ainsi, aucune des sondes proposées jusqu'ici pour des tests à diffusion capillaire ne donne entière satisfaction. En particulier, il demeure un besoin de disposer d'une sonde, compatible avec une mise en œuvre dans un dispositif de test à diffusion capillaire, permettant de combiner les avantages d'une sonde très lumineuse, peu complexe et peu onéreuse à synthétiser, qui autorise une détection bien plus sensible que les systèmes à diffusion capillaire à base de nanoparticules d'or, avec un système de lecture à l'œil nu ou avec un lecteur compact et peu onéreux.
La présente invention a précisément pour objet de proposer de nouvelles sondes luminescentes pouvant être mises en œuvre dans un test à diffusion capillaire, et répondant à ce besoin.

Plus particulièrement, elle propose l'utilisation, à titre de sondes dans une méthode d'analyse par un test à diffusion capillaire, de nanoparticules luminescentes dopées par des ions terres rares, aux propriétés optiques et physico-chimiques contrôlées.

Plus particulièrement, l'invention concerne, selon un premier de ses aspects, un procédé *in vitro* de détection et/ou de quantification d'une substance d'intérêt biologique ou chimique dans un échantillon liquide, par un test à diffusion capillaire de type bandelette de migration, mettant en œuvre, à titre de sondes, des nanoparticules inorganiques photoluminescentes, de formule (II) suivante :

A₁₋ₓLnₓVO_{4(1-y)}(PO₄)_{y} (II)

dans laquelle :
. A est choisi parmi l'yttrium (Y), le gadolinium (Gd), le lanthane (La), le lutécium (Lu), et leurs mélanges ;
. Ln est choisi parmi l'europium (Eu), le dysprosium (Dy), le samarium (Sm), le néodyme (Nd), l'erbium (Er), l'ytterbium (Yb), le thulium (Tm), le praséodymium (Pr), l'holmium (Ho) et leurs mélanges ;
0,1 ≤ x ≤ 0,9, en particulier 0,2 ≤ x ≤ 0,6 et plus particulièrement x vaut 0,4 ; et
. 0 ≤ y < 1, en particulier y vaut 0 ;
ledit procédé mettant en œuvre la détection de la luminescence, d'une durée de vie d'émission plus brève que 100 ms, des nanoparticules, après une absorption à un photon, par excitation de la matrice à une longueur d'onde inférieure ou égale à 320 nm.

En particulier, la détection de la luminescence est avantageusement opérée par excitation de la matrice AVO_{4(1-y)}(PO₄)_{y}, par exemple YVO₄, à une longueur d'onde inférieure ou égale à 300 nm, en particulier comprise entre 250 et 300 nm.

Selon le procédé de l'invention, le signal détecté correspond ainsi à l'émission de luminescence par les nanoparticules photoluminescentes après absorption d'un unique photon, autrement dit de l'émission à une longueur d'onde plus grande que celle de l'excitation. L'émission de luminescence après absorption d'un unique photon s'oppose en particulier au cas de la détection de l'émission de luminescence par des particules pour une absorption à deux photons, comme c'est le cas des particules à « up-conversion » évoquées précédemment.

Ces nanoparticules sont fonctionnalisées avec des molécules de reconnaissance (anticorps, acides nucléiques, peptides, aptamères, etc.) qui sont capables de reconnaitre la substance à analyser tel que cela est décrit par la suite.

Au sens de l'invention, l'« analyse » de la substance dans un échantillon couvre l'aspect détection ou encore caractérisation qualitative de la présence ou non de ladite substance, et également l'aspect dosage ou encore caractérisation quantitative de ladite substance.

L'échantillon liquide peut être notamment un échantillon biologique, en particulier tout fluide biologique ou corporel. Il peut s'agir d'un échantillon de prélèvement humain, par exemple choisi parmi le sang, le sérum, le plasma, la salive, l'expectoration, le frottis nasal, l'urine, la matière fécale diluée, le frottis vaginal ou encore le liquide cérébro-spinal.

Il peut encore s'agir d'une solution contenant des molécules biologiques, des molécules chimiques ou encore des virus ou bactéries pathogènes, comme par exemple des échantillons environnementaux ou issus de produits agro-alimentaires.

Le procédé de l'invention peut notamment être mis en œuvre pour la détection et/ou la quantification de molécules, de protéines, d'acides nucléiques, de toxines, de virus, de bactéries ou de parasites, dans un échantillon, en particulier dans un échantillon biologique.

Il peut par exemple être utilisé pour détecter la présence de biomarqueurs, d'anticorps, d'ADN et/ou d'ARN, d'immunoglobulines (IgG, IgM, etc.), d'antigènes, les antigènes pouvant également être des biomolécules composant un virus, une bactérie ou un parasite, dans un échantillon biologique.

Il peut également s'agir par exemple d'une molécule d'intérêt pour des enquêtes policières scientifiques, par exemple d'une substance chimique illicite telle qu'une drogue, ou d'une substance d'intérêt pour la défense (agents de bioterrorisme).

Il peut encore s'agir d'une substance d'intérêt pour la sécurité alimentaire (bactéries pathogènes comme des *Salmonella,* des *Listeria,* ou des *Escherichia coli,* ou encore des virus comme le norovirus ou des allergènes), ou pour l'environnement, par exemple d'un polluant (pesticides).

La substance d'intérêt biologique ou chimique que l'on cherche à analyser *via* le test à diffusion capillaire selon l'invention est désignée, plus simplement par la suite, par les expressions « substance à analyser » ou « analyte ».

L'utilisation des nanoparticules inorganiques luminescentes selon l'invention comme sondes dans un dispositif de test à diffusion capillaire, dans une bandelette de test, s'avère particulièrement avantageuse à plusieurs titres.

Tout d'abord, les nanoparticules dopées par des ions de terres rares, mises en œuvre selon l'invention, de formule (A₁₋ₓLnₓ)ₐ(MₚO_{q}) (I) décrites plus précisément dans la suite du texte, par exemple des nanoparticules de type YVO₄ :Eu ou GdVO₄ :Eu, YAG :Ce, en particulier les nanoparticules de formule A₁₋ₓLnₓVO_{4(1-y)}(PO₄)_{y} (II), présentent des propriétés particulièrement avantageuses, notamment au regard de leur excellente photostabilité, qui autorise l'acquisition d'un signal constant et prolongé, et une absence de phénomène de clignotement de l'émission.

Par ailleurs, ces nanoparticules ne perdent pas leur luminescence après congélation.

Des nanoparticules à base de vanadate d'yttrium dopé par des terres rares ont par exemple été décrites de façon détaillée par Riwotzki *et al.* ([45]) et Huignard *et al.* ([46]). Quant au document EP 1 282 824, il décrit la mise en œuvre de nanoparticules luminescentes inorganiques, modifiées en surface, à titre de sondes pour détecter une substance biologique ou autre substance organique.

Toutefois, à la connaissance des inventeurs, il n'a jamais été proposé de tirer profit de nanoparticules inorganiques photoluminescentes telles que définies précédemment, dopées par des ions de terres rares, distinctes de particules à luminescence persistante, et émettant après absorption d'un unique photon, pour leur utilisation à titre de sondes luminescentes dans un test à diffusion capillaire.

Il n'était nullement prévisible que ces nanoparticules à base d'ions lanthanides puissent être utilisées pour la détection et la quantification de substances chimiques ou biologiques, en particulier dans un test à diffusion capillaire et, qui plus est, qu'elles conduisent à des performances améliorées en termes de sensibilité du test.

En effet, en dehors de l'absence de clignotement, les propriétés de luminescence des nanoparticules à base de terres rares sont considérées comme inférieures à celles des Quantum Dots. Dans ces nanoparticules dopées par des ions de terres rares, en particulier celles constituées d'une matrice d'oxyde métallique, l'excitation de la luminescence peut se faire soit par excitation directe de la matrice, soit, plus rarement, par excitation directe dans le visible des ions de terres rares luminescents. Le coefficient d'extinction pour l'absorption directe des ions de terres rares est en général très faible, mais le coefficient d'extinction pour une excitation de la matrice cristalline est bien plus élevé ([35]).

Cependant, la bande d'absorption de la matrice cristalline se situe en général dans l'UV, ce qui présente un inconvénient majeur : les biomolécules ainsi que les différentes composantes du dispositif à diffusion capillaire (par exemple, la membrane de diffusion capillaire) absorbent également fortement dans l'UV. Par exemple, dans le cas d'une matrice à base d'ions vanadate VO₄³⁻, le pic d'absorption à 280 nm coïncide avec l'absorption des protéines, et en particulier des acides aminés tryptophane. Par conséquent, l'excitation de la luminescence de nanoparticules à base d'une matrice cristalline dopée en ions de terres rares, en particulier dans l'UV, est susceptible de produire des signaux d'émission annexe importants. Une telle émission parasite est incompatible avec l'objectif d'un test de diagnostic *in vitro* tel qu'un test sur bandelettes à diffusion capillaire, qui vise précisément à identifier un signal de faible intensité à partir d'un mélange complexe de molécules.

Contre toute attente, les inventeurs ont découvert qu'il est possible d'utiliser de telles nanoparticules photoluminescentes à base de terres rares selon l'invention, à titre de sondes dans une technique de diagnostic *in vitro* de type test à diffusion capillaire, même dans des conditions d'excitation dans l'UV et en particulier pour une excitation en dessous de 350 nm, avantageusement en-dessous de 320 nm, plus avantageusement entre 250 et 320 nm et en particulier entre 250 et 300 nm.

Sans vouloir être lié par la théorie, les inventeurs ont constaté qu'une détection, dans un test à diffusion capillaire, de la luminescence des nanoparticules excitées dans l'UV s'avère possible, malgré la présence d'un fort signal d'émissions parasites, du fait de trois propriétés optiques, spécifiques aux nanoparticules mises en œuvre : i) un grand nombre d'ions lanthanides luminescents contenus dans les nanoparticules de l'invention sans être obligé de recourir à des nanoparticules de taille très importante, ii) un spectre d'émission des ions de terres rares étroit, ce qui permet d'éliminer efficacement les émissions parasites qui, en général, sont très larges spectralement et iii) un grand décalage de Stokes (décalage entre le pic d'absorption et le pic d'émission), typiquement de l'ordre de 350 nm pour les nanoparticules YVO₄ ou GdVO₄ dopées à l'Eu (pic d'absorption à 280 nm pour la matrice de vanadate et pic d'émission de l'Eu à 617 nm), ce qui permet une réjection efficace des longueurs d'onde d'excitation et des émissions parasites dues au support de migration ou à l'échantillon contenant la substance à analyser qui, en général, présentent un faible décalage de Stokes. En particulier, ce grand décalage de Stokes permet d'utiliser pour la détection un simple filtre passe-haut, moins onéreux qu'un filtre interférentiel.

Dès lors, il est possible d'obtenir une élimination efficace des émissions parasites, et l'acquisition d'un signal avec un rapport signal à bruit suffisant pour atteindre la sensibilité de détection souhaitée.

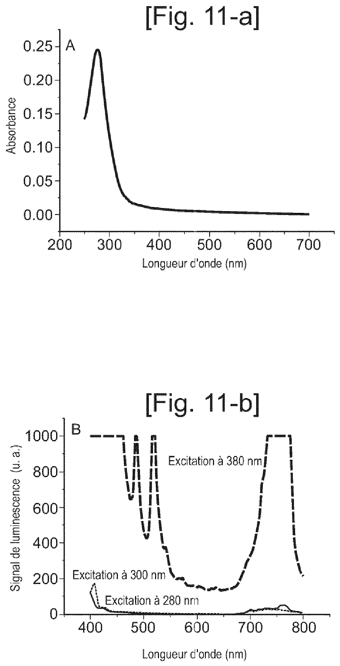

Par ailleurs, il s'est avéré contre toute attente qu'une excitation en dessous de 320 nm et en particulier aux alentours de 280 nm où se situe le pic d'absorption de la matrice de vanadate (voir Figure 11(A)) ou à 300 nm, induit des émissions parasites, liées à la membrane de nitrocellulose typiquement utilisée pour les tests à flux latéral, bien plus faibles qu'une excitation à 380 nm (voir Figure 11(B)). Sachant que les molécules chélatant ou complexant les ions lanthanides absorbent typiquement entre 320 nm ([47]) et 400 nm ([48]), l'utilisation d'une matrice absorbant entre 250 et 300 nm, en particulier entre 260 et 300 nm, présente un avantage supplémentaire.

En particulier, en dehors de l'absorption de transfert de charge O²⁻-V⁵⁺ à l'intérieur des ions vanadate VO₄³⁻ dans un cristal de YVO₄ ou GdVO₄, qui est centrée à 280 nm, ou dans un cristal de LaVO₄, centrée autour de 300 nm ([40]), dans le cas de nanoparticules contenant de l'Eu, une autre absorption, liée à un transfert de charge O²⁻-Eu³⁺, est possible et mène à une absorption centrée autour de 260 nm. Celle-ci a par exemple été observée dans des nanoparticules La₂Hf₂O₇:Eu, A₂Hf₂O₇ (A = Y, Gd, Lu) et La₂Zr₂O₇:Eu ([41]).

Qui plus est, comme illustré dans les exemples qui suivent, le procédé de l'invention permet d'atteindre une performance du test à diffusion capillaire, en termes de sensibilité de détection, améliorée d'au moins un ordre de grandeur voire bien plus.

Il autorise en particulier, non seulement des analyses qualitatives (présence ou absence de l'analyte dans l'échantillon), mais également des analyses semi-quantitatives et quantitatives.

Ainsi, ce document concerne encore l'utilisation, à titre de sondes dans un dispositif de test à diffusion capillaire, de nanoparticules telles que définies précédemment, pour augmenter la sensibilité de détection dudit dispositif de test à diffusion capillaire.

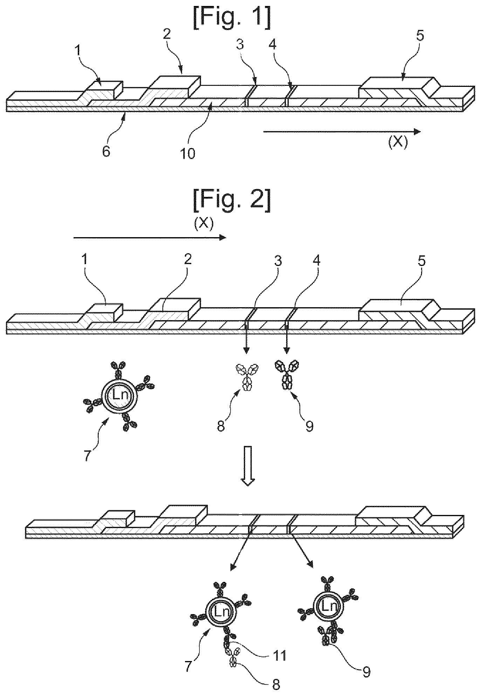

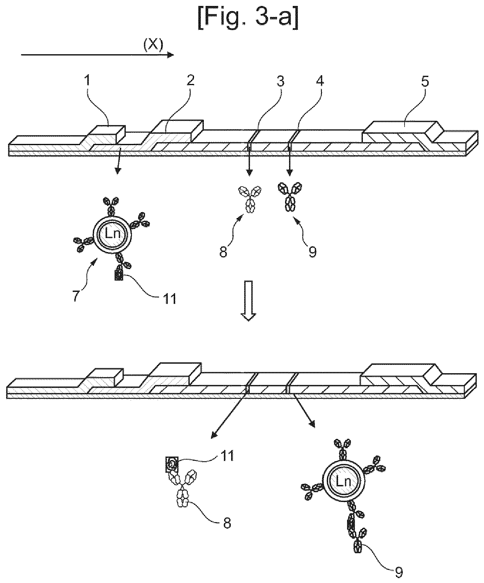

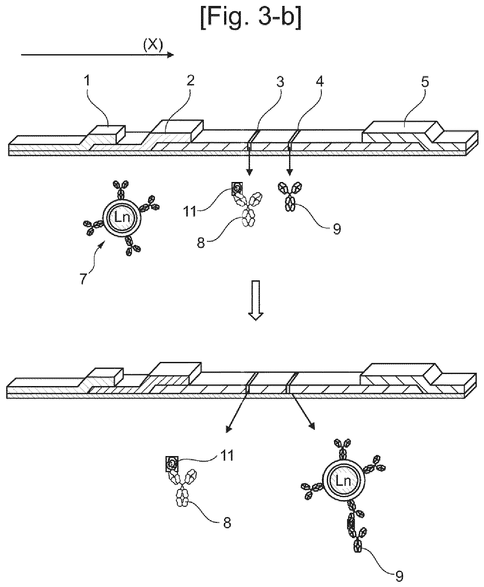

Les nanoparticules photoluminescentes peuvent être mises en œuvre, à titre de sondes, dans tout type de test à diffusion capillaire connu, par exemple des tests à flux latéral, qu'il s'agisse d'un test dit « sandwich » comme représenté schématiquement en Figure 2, ou encore d'un test dit « de compétition » comme représenté en Figure 3. En particulier, elles sont adaptées à une mise en œuvre dans des dispositifs de tests à diffusion capillaire proposés jusqu'à présent avec, comme sondes luminescentes, des nanoparticules d'or, sans nécessiter de modifier les caractéristiques du support du dispositif de test à diffusion capillaire.

En particulier, comme illustré dans les exemples qui suivent, les nanoparticules photoluminescentes selon l'invention peuvent, par exemple, présenter une taille moyenne similaire à celle des nanoparticules d'or, de l'ordre de 30 à 50 nm et, par conséquent, compatible avec les moyens de diffusion capillaire, typiquement une membrane de nitrocellulose, adaptés à la migration des particules de cette taille.

Alternativement, les nanoparticules photoluminescentes mises en œuvre selon l'invention peuvent présenter des tailles plus importantes, permettant ainsi d'optimiser le signal de luminescence. De fait, le nombre d'ions lanthanides augmente avec le volume de la nanoparticule et ainsi le signal de luminescence émis augmente avec le cube du rayon d'une particule sphérique. Dans ce cas, le support de migration du test à flux latéral peut contenir des pores adaptés à la taille des nanoparticules choisies. Des membranes avec des tailles de pores variables sont par exemple proposées dans le commerce (par exemple, les membranes à tailles de pores variables, commercialisées sous les références HF075, HF090, HF120, HF135, HF180, par la société MerckMillipore).

Des nanoparticules de taille plus importante peuvent par exemple être obtenues par tri en taille par centrifugation des particules telles qu'exemplifiées, pour ne conserver, dans la distribution de tailles, que les particules de tailles les plus importantes, ou encore être obtenues par broyage du matériau massif. Toute autre technique connue par l'homme du métier peut être également utilisée.

Par ailleurs, comme illustré dans les exemples, tout en conservant une taille de particules similaire à celle des particules d'or, les nanoparticules mises en œuvre selon l'invention présentent un nombre d'ions à l'origine de la luminescence important, en particulier significativement plus élevé que dans le cas de particules à base de chélates ou complexes de lanthanides, et permettent, dès lors, de produire un signal d'émission d'intensité élevée et, par conséquent, d'accéder à une sensibilité améliorée.

De manière avantageuse, le procédé de détection selon l'invention rend possible une mesure qualitative jusqu'à 10 fois, en particulier jusqu'à 100 fois, voire jusqu'à 1000 fois, en-dessous de la limite de détection d'un même test mettant en œuvre, à titre de sondes, des nanoparticules d'or.

Enfin, la mise en œuvre des nanoparticules mises en œuvre selon l'invention à titre de sondes dans un test à diffusion capillaire conduit même à des performances, en termes de sensibilité, améliorées comparativement aux résultats obtenus avec des particules chargées en chélates de lanthanides.

L'invention concerne, selon un autre de ses aspects, un dispositif de test à diffusion capillaire de type bandelette de migration selon la revendication 14.

Ainsi, le dispositif de test à diffusion capillaire selon l'invention comprend des nanoparticules inorganiques photoluminescentes de formule (II) dont on détecte la luminescence, d'une durée de vie d'émission plus brève que 100 ms, après absorption à un photon, par excitation de la matrice à une longueur d'onde inférieure ou égale à 320 nm, en particulier inférieure ou égale à 300 nm et plus particulièrement entre 250 et 300 nm.

Plus précisément, à l'instar des sondes, par exemple des nanoparticules d'or, classiquement mises en œuvre dans les dispositifs connus de test à flux latéral, les nanoparticules photoluminescentes selon l'invention sont présentes au niveau d'une zone du dispositif de test, dite « zone de marquage » (plus couramment appelée « Conjugate Pad » en terminologie anglo-saxonne), sous une forme couplée avec au moins un réactif de liaison spécifique de la substance à analyser, tel qu'un anticorps.

Dans la suite du texte, l'invention est plus particulièrement décrite en référence à un dispositif de test à diffusion capillaire classique de type bandelette de migration (connu sous l'appellation en langue anglaise « Lateral Flow Strip »), comme représenté schématiquement en Figure 1. Bien entendu, le procédé de l'invention peut mettre en œuvre toute autre variante de dispositif de test à diffusion capillaire, pour autant qu'elle soit adaptée à la mise en œuvre, à titre de sondes, des nanoparticules photoluminescentes mises en œuvre selon l'invention.

Typiquement, un dispositif de test à diffusion capillaire selon l'invention peut ainsi comprendre un moyen de diffusion capillaire dans une direction de référence, en particulier un support solide poreux, tel qu'une membrane de nitrocellulose, comprenant :
- une zone de dépôt de l'échantillon liquide ;
- une zone, disposée en aval de la zone de dépôt de l'échantillon, dite zone de marquage ou zone de couplage, chargée avec les nanoparticules inorganiques photoluminescentes selon l'invention (sondes), couplées à au moins un « réactif de liaison » (molécule de reconnaissance), par exemple un anticorps, spécifique de la substance à analyser ;
- une zone réactionnelle, dite encore « zone de détection », disposée en aval de la zone de marquage, dans laquelle est immobilisé au moins un « réactif de capture » (molécule de reconnaissance), tel qu'un anticorps, spécifique de la substance à analyser ;
- une zone de contrôle de migration des réactifs, située en aval de la zone de détection ; et
optionnellement, un tampon absorbant, disposé en aval de la zone de contrôle.

Des exemples de dispositifs de test à diffusion capillaire seront plus particulièrement détaillés dans la suite du texte.

L'échantillon liquide peut être analysé directement à l'aide du dispositif de test à diffusion capillaire selon l'invention. L'analyse d'un échantillon liquide selon le procédé de l'invention comprend typiquement :
(i) l'application de l'échantillon liquide à analyser, et éventuellement d'un diluant, au niveau de la zone de dépôt du dispositif de test à diffusion capillaire ;
(ii) l'incubation du dispositif jusqu'à la détection dans la zone réactionnelle de la luminescence générée par les nanoparticules photoluminescentes et/ou jusqu'à la détection de la luminescence dans la zone de contrôle de migration ; et
(iii) la lecture et l'interprétation des résultats.

Selon un autre de ses aspects, la présente invention concerne l'utilisation d'un dispositif de test à diffusion capillaire selon l'invention pour détecter et/ou quantifier une substance d'intérêt biologique ou chimique dans un échantillon liquide, en particulier un échantillon biologique.

Le dispositif de test à diffusion capillaire peut être couplé avec un lecteur permettant de fournir le résultat du test.

Comme détaillé dans la suite du texte, la lecture des résultats comprend la détection de la luminescence générée par les nanoparticules immobilisées au niveau de la zone de détection, et le cas échéant au niveau de la zone de contrôle, du dispositif de test à diffusion capillaire.

Elle est plus particulièrement opérée par :
- excitation des nanoparticules photoluminescentes immobilisées ; et
- détection de l'émission de luminescence.

De manière particulièrement avantageuse, il est possible de procéder à une lecture du dispositif de test à diffusion capillaire, à l'œil nu, en utilisant uniquement un filtre adapté.

Alternativement, la lecture de la luminescence peut être opérée à l'aide d'un appareillage de détection simple, par exemple à l'aide d'un filtre d'émission et un détecteur tel qu'une caméra.

Le filtre d'émission peut être un filtre interférentiel mais également un simple filtre passe-haut. En effet, grâce au grand décalage de Stokes lié à l'émission de ces particules, toute émission parasite, qui présente en général un faible décalage de Stokes, se situera à plus courte longueur d'onde que l'émission de ces nanoparticules.

Enfin, le dispositif de test à diffusion capillaire selon l'invention est adapté à une détection multiplexée, autrement dit à la détection simultanée par le même test à diffusion capillaire, de plusieurs substances dans un même échantillon.

L'invention concerne encore, selon un autre de ses aspects, l'utilisation d'un procédé de détection tel que défini précédemment, ou d'un dispositif de test à diffusion capillaire tel que défini précédemment, à des fins de diagnostic *in vitro.* Avantageusement, la possibilité, par le test à diffusion capillaire selon l'invention, de détecter des teneurs faibles de certaines substances dans des échantillons biologiques permet, par exemple, l'utilisation du procédé de l'invention pour une détection plus précoce des maladies, ou encore un diagnostic sur l'évolution d'une maladie ou sur l'effet d'un traitement thérapeutique.

Les maladies pouvant être diagnostiquées par un test à diffusion capillaire selon l'invention ne sont pas limitées et comprennent toutes maladies révélées par la présence d'un marqueur spécifique de la maladie, du type molécule d'intérêt biologique (protéine, acide nucléique, anticorps, ...), pour lequel il existe un ou des partenaire(s) de liaison spécifique (ligand, anticorps, antigènes, acides nucléiques complémentaires, aptamères, ...).

A titre d'exemples, on peut citer les maladies infectieuses (bactériennes, parasitaires, ou virales, telles que le SIDA), les maladies inflammatoires et auto-immunes, les maladies cardiologiques, neurologiques, ou oncologiques (par exemple, les cancers solides tels que le cancer du sein ou de la prostate).

Les gains en sensibilité les plus importants (d'un facteur de 100 ou 1000) permettent d'atteindre des performances proches d'un test ELISA ou d'une des variantes d'ELISA. Ainsi, le procédé de l'invention est particulièrement adapté dans les cas où une détection sensible de type ELISA est nécessaire, mais non accessible.

Il rend ainsi possible un diagnostic rapide, au point d'intervention (POC pour « Point Of Care » en langue anglaise).

Le procédé de l'invention peut être ainsi utile pour le diagnostic de maladies infectieuses ou autres maladies courantes, par exemple dans les pays en développement, dans des zones rurales et/ou reculées pour le diagnostic de maladies infectieuses ou autres maladies courantes.

Il peut encore s'avérer particulièrement utile dans le cadre d'interventions en urgence (SAMU, SMUR), pour permettre un diagnostic d'urgence, notamment dans le cas où le pronostic vital du patient peut être en danger (défaillance cardiaque, thrombose veineuse, syndrome inflammatoire, infection bactérienne systémique (sepsis), pancréatite aigüe). Dans de telles situations, il peut être mis en œuvre pour réaliser un test rapide avec une sensibilité comparable à celle d'un test ELISA avant l'arrivée à l'hôpital, permettre de gagner du temps dans la pose du diagnostic et dans la prise en charge du patient, et ainsi améliorer le pronostic vital du patient.

Par ailleurs, un diagnostic par un test à diffusion capillaire, utilisant des particules selon l'invention comme sondes, peut être particulièrement utile pour des patients qui doivent faire régulièrement des tests de diagnostic pour ajuster la dose de médicaments administrée (par exemple dans le cas d'immunomodulateurs ou d'immunosuppresseurs). En effet, la réalisation d'un test sur bandelette, plutôt qu'un diagnostic par prise de sang ou autre examen plus invasif, permet avantageusement d'améliorer le confort du patient, de diminuer les frais du diagnostic, d'effectuer une détection/quantification plus rapprochée dans le temps, et ainsi de permettre un meilleur ajustement des doses de médicaments administrées.

Bien entendu, le procédé de l'invention n'est pas limité aux applications précitées. Il peut ainsi être mis en œuvre pour une détection d'acides nucléiques (OGM dans des semences par exemple), ou pour la détection d'un polluant ou d'un pathogène dans l'environnement, par exemple dans de l'eau, ou dans des aliments destinés à la consommation humaine ou animale.

Les applications du procédé de l'invention peuvent ainsi s'étendre des domaines immunologiques à la génétique moléculaire ou à la détection d'ADN et d'ARN. Il peut être utilisé pour marquer un ou plusieurs brins d'ARN d'un prélèvement biologique, avec un fragment partiellement complémentaire lié à une nanoparticule, puis les détecter par hybridation sur des fragments complémentaires d'une autre région greffés sur le substrat d'une bandelette, suivant une approche voisine des puces à ADN de type Affymetrix. Un intérêt de l'invention réside alors dans l'absence d'étape d'amplification habituellement nécessaire pour ces approches.

Le procédé de l'invention peut encore être mis en œuvre pour la détection *in vitro* de substances chimiques illicites, par exemple de drogues ou toute autre substance d'intérêt pour la police ou la défense.

Il peut encore être mis en œuvre pour la détection et/ou quantification d'une substance d'intérêt, notamment d'un pathogène, dans un produit agroalimentaire ou dans l'environnement.

D'autres caractéristiques, variantes et avantages du procédé et du dispositif de test à diffusion capillaire selon l'invention ressortiront mieux à la lecture de la description, des exemples et des figures qui suivent, donnés à titre illustratif et non limitatif de l'invention.

Dans la suite du texte, les expressions « compris entre ... et ... », « allant de ... à ... » et « variant de ... à ... » sont équivalentes et entendent signifier que les bornes sont incluses, sauf mention contraire.

Sauf indication contraire, l'expression « comprenant un(e) » doit être comprise comme « comportant au moins un(e) ».

### NANOPARTICULES INORGANIQUES PHOTOLUMINESCENTES

Comme indiqué précédemment, le procédé de l'invention met en œuvre, à titre de sondes dans un dispositif de test à diffusion capillaire de type bandelette de migration, des nanoparticules inorganiques photoluminescentes présentant des propriétés optiques et physico-chimiques spécifiques.

Les nanoparticules photoluminescentes mises en œuvre selon l'invention sont formées d'une matrice cristalline dopée par des ions de terres rares. La « matrice cristalline » est caractéristique d'un solide cristallin, dans lequel certains atomes sont remplacés par d'autres atomes, appelés « ions substitués ». Les ions substitués permettent de modifier une propriété chimique ou physique de la matrice cristalline, notamment de conférer une qualité d'émission optique à la nanoparticule.

Les ions de terres rares dans les nanoparticules ne sont pas sous forme de complexes ou chélates d'ions de terres rares, ces derniers étant formés d'ions de terres rares en combinaison avec des ligands organiques appropriés, comme par exemple décrits dans la publication Yuan *et al.* ([36]).

Les nanoparticules peuvent être dopées par des ions de terres rares de même nature ou de natures différentes.

De manière avantageuse, la cristallinité imparfaite des nanoparticules comme décrit plus précisément par la suite, permet de s'affranchir de l'effet de « quenching ».

Selon une autre des caractéristiques des nanoparticules photoluminescentes mises en œuvre selon l'invention, elles sont aptes à émettre de la luminescence après absorption d'un unique photon, qui correspond au signal détecté.

Par ailleurs, l'émission de luminescence par les nanoparticules, à la différence des nanoparticules dites à luminescence persistante ([32]), n'implique pas d'états « pièges ».

Ainsi, les nanoparticules mises en œuvre selon l'invention présentent une durée de vie d'émission de la luminescence plus courte que 100 ms, autrement dit strictement inférieure à 100 ms ([35], [39], [49]).
La durée de vie d'émission s'entend comme la durée de vie de l'état excité de la nanoparticule émettrice, et plus spécifiquement des ions de terres rares émetteurs, et est déterminée en pratique par la durée de l'émission de photons de luminescence après arrêt de l'excitation, soit le temps caractéristique du déclin exponentiel de luminescence après arrêt de l'excitation.

La durée de vie d'émission d'une nanoparticule émettrice est distincte de la durée d'émission de la luminescence avant photodégradation ou photoblanchiment des nanoparticules.

Les nanoparticules mises en œuvre selon l'invention présentent une durée de vie d'émission inférieure à 100 ms, voire inférieure à 10 ms, voire inférieure à 1 ms.

De manière avantageuse, les nanoparticules mises en œuvre selon l'invention présentent une durée de vie d'émission supérieure ou égale à 5 µs, en particulier supérieure ou égale à 10 µs, notamment supérieure ou égale à 20 µs, voire supérieure ou égale à 50 µs.

Il est possible de tirer profit de la durée de vie d'émission des particules de l'invention (quelques centaines de µs dans le cas des particules Y₁₋ₓEuₓVO₄, en comparaison aux durées de vie des fluorophores usuels de l'ordre de la nanoseconde), pour effectuer une détection résolue en temps, en particulier une détection retardée de l'émission, avec une résolution temporelle suffisante (de l'ordre de 10 µs voire de l'ordre de 100 µs), en utilisant du matériel peu sophistiqué et peu onéreux. Par exemple, il est possible de moduler le courant d'une LED (« Light Emitting Diode ») qui peut être utilisée pour l'excitation et d'enregistrer une série d'images de la bandelette au lieu d'une seule et ensuite analyser le signal en fonction du temps de sorte à éliminer toute émission parasite résiduelle de durée de vie courte (de l'ordre de 10 ns ou moins).
, les nanoparticules photoluminescentes mises en œuvre selon l'invention sont de formule (II) suivante :

   A₋ₓLnₓVO_{4(1-y)}(PO₄)_{y} (II)
dans laquelle :
   . A est choisi parmi l'yttrium (Y), le gadolinium (Gd), le lanthane (La), le lutécium (Lu) et leurs mélanges, en particulier A représente Y ;
   . Ln est choisi parmi l'europium (Eu), le dysprosium (Dy), le samarium (Sm), le néodyme (Nd), l'erbium (Er), l'ytterbium (Yb), le thulium (Tm), le praséodynium (Pr), l'holmium (Ho) et leurs mélanges, de préférence Ln représente Eu ;
   . 0,1 ≤ x ≤ 0,9, en particulier 0,2≤x≤0,6 et plus particulièrement x vaut 0,4 ; et
   . 0 ≤ y < 1, en particulier y vaut 0
ledit procédé mettant en œuvre la détection de la luminescence, d'une durée de vie plus courte que 100 ms, par les nanoparticules, après une absorption à un photon.

Selon un mode de réalisation particulier, les nanoparticules mises en œuvre selon l'invention répondent à la formule (II) précitée dans laquelle y vaut 0. Autrement dit, les nanoparticules peuvent être de formule A₁₋ₓLnₓVO₄ (III), dans laquelle A, Ln et x sont tels que définis précédemment.

A, dans la formule (II) ou (III), précitée peut être plus particulièrement choisi parmi l'yttrium (Y), le gadolinium (Gd), le lanthane (La) et leurs mélanges. En particulier, A représente Y ou Gd. Selon un mode de réalisation particulier, A dans la formule (II) ou (III) précitée représente l'yttrium (Y).

Ln, dans la formule (II) ou (III), précitée peut être plus particulièrement choisi parmi l'europium (Eu), le dysprosium (Dy), le samarium (Sm), l'ytterbium (Yb), l'erbium (Er), le néodyme (Nd) et leurs mélanges. En particulier, Ln est choisi parmi Eu, Dy, Sm et leurs mélanges. Selon un autre mode de réalisation particulier, Ln dans la formule (II) ou (III) précitée représente Eu.

Ainsi, selon une variante de réalisation, les nanoparticules mises en œuvre comme sondes luminescentes selon l'invention sont de formule Y₁₋ₓEuₓVO₄ (IV) dans laquelle 0,1 ≤ x ≤ 0,9, en particulier 0,2≤x≤0,6 et plus particulièrement x vaut 0,4.

Selon une variante de réalisation, il est possible d'exploiter l'absorption directe des ions terres rares, dans les cas où la transition électronique correspondante est permise. Dans ces cas, l'absorption directe est plus forte que lorsque la transition électronique correspondant est interdite même si elle reste en général moins forte que l'absorption de la matrice d'oxyde. Deux exemples d'ions terres rares qui se trouvent dans ce cas sont l'ion Eu²⁺ et Ce³⁺. Ces ions peuvent par exemple se trouver comme constituants des nanoparticules inorganiques suivantes : LaPO₄ ou YAG dans le cas de Ce³⁺ et Sr₂O₄ dans le cas de l'Eu²⁺.

Les nanoparticules photoluminescentes mises en œuvre selon l'invention peuvent présenter une taille moyenne supérieure ou égale à 5 nm et strictement inférieure à 1 µm, en particulier comprise entre 10 nm et 500 nm, de préférence entre 20 nm et 200 nm et notamment entre 20 nm et 100 nm.

Les nanoparticules photoluminescentes mises en œuvre selon l'invention présentent ainsi un volume suffisant pour contenir un grand nombre d'ions de terres rares, et donc émettre un signal luminescent suffisant pour permettre la détection de faibles concentrations en analyte.

De préférence, les nanoparticules mises en œuvre selon l'invention comprennent au moins 10³ ions de terres rares, notamment entre 1000 et 6 000 000 ions de terres rares, en particulier entre 5 000 et 500 000 et plus particulièrement entre 20 000 et 100 0000 ions de terres rares.

A titre d'exemple, une nanoparticule sphérique Y_{0.6}Eu_{0,4}VO₄ de diamètre de 30 nm contient 70 000 ions Eu³⁺ (calcul du nombre d'ions selon la référence Casanova *et al.* [37])*.*

La taille moyenne peut être mesurée par microscopie électronique à transmission. Les images de microscopie électronique à transmission permettent de déterminer la forme des nanoparticules (sphérique, ellipsoïde) et de déduire les dimensions moyennes des nanoparticules. Dans le cas de particules globalement sphériques, la taille moyenne s'entend du diamètre moyen des particules. Dans le cas de particules de forme ellipsoïde, la taille moyenne s'entend de la taille moyenne d'une sphère de même volume que l'ellipsoïde. On suppose en général que le troisième axe de l'ellipsoïde, non visible dans les images de transmission qui sont des projections 2D, est de longueur égale à l'axe de plus petite taille.

Selon un mode de réalisation particulier, les nanoparticules sont de forme globalement ellipsoïdale allongée (« prolate » en langue anglaise).

Elles peuvent plus particulièrement présenter une longueur du grand axe, notée *a,* comprise entre 20 et 60 nm ; et une longueur du petit axe, notée b, comprise entre 10 et 30 nm. En particulier, les nanoparticules peuvent présenter une valeur moyenne de longueur du grand axe, a, de 40 nm et une valeur moyenne de longueur du petit axe, b, de 20 nm.

De manière avantageuse, les nanoparticules mises en œuvre selon l'invention présentent une faible polydispersité. Il est préférable que l'indice de polydispersité, qui peut être déduit des mesures de diffusion dynamique de la lumière, soit strictement inférieur à 0,2. Lorsque cela n'est pas le cas à l'issue de la synthèse ou de la fonctionnalisation des particules, une polydispersité plus faible peut être obtenue par tri en taille par centrifugation ou par toute autre technique connue par l'homme du métier.

Selon un mode de réalisation particulier, le produit entre le taux de dopage en ions de terres rares, par exemple en europium (Eu), et le rendement quantique de l'émission par la nanoparticule est maximisé.

Cette maximisation du produit entre le taux de dopage x en ions Ln et le rendement quantique peut être effectuée en utilisant un fort dopage en ions Ln, par exemple entre 0,2 et 0,6, et notamment de 0,4, sans pour autant diminuer le rendement quantique, en particulier en limitant les processus de transfert entre ions de dopage conduisant à une extinction de concentration. En particulier, afin de maintenir un rendement quantique élevé, la nanoparticule présente une cristallinité imparfaite. En effet, une excellente cristallinité favorise les processus de transfert entres ions de dopage, en particulier lorsque ceux-ci sont à proximité immédiate entre eux, comme cela est le cas pour des dopages élevés et, par conséquent, favorise les processus de désexcitation des ions par des processus non radiatifs, liés à la surface et à la présence du solvant. En particulier, un procédé de synthèse à température ambiante, ou tout au moins à température n'excédant pas 600°C, est favorable pour la cristallinité imparfaite requise pour ces nanoparticules.

La cristallinité des nanoparticules est considérée comme étant « imparfaite » lorsque la longueur de cohérence, déterminée par le diffractogramme des rayons X dans au moins une direction cristallographique donnée, est inférieure à 80 % de la taille de la particule dans cette direction-là telle que mesurée à partir des images de microscopie électronique à transmission. Différents types de cristallinité imparfaite peuvent être considérés : polycristallinité, défauts, porosité, etc.

De manière avantageuse, les nanoparticules mises en œuvre selon l'invention sont aptes à émettre chacune plus de 10⁸ photons avant l'arrêt de l'émission, en particulier plus de 10⁹, voire plus de 10¹⁰ photons. Dans de nombreux cas, en particulier dans le cas de particules YVO₄ ou GdVO₄ dopées à l'Eu, aucun arrêt (extinction) de l'émission n'est constaté sous illumination continue. Autrement dit, de manière avantageuse, les nanoparticules selon l'invention ne présentent pas de phénomènes de photodégradation ou photoblanchiment irréversible.

De manière avantageuse, les nanoparticules mises en œuvre selon l'invention présentent une bonne stabilité colloïdale en solution.

La stabilité des nanoparticules en solution est particulièrement déterminante pour répondre aux exigences en termes de reproductibilité des résultats de détection à partir de l'utilisation de ces particules à titre de sondes dans un dispositif de test à diffusion capillaire.

En particulier, une bonne stabilité colloïdale des nanoparticules permet d'assurer, lors de la migration de l'échantillon liquide dans le support poreux du test à diffusion capillaire, la migration des nanoparticules luminescentes, le cas échéant, liées à la substance à analyser, jusqu'à la zone de détection et, éventuellement, jusqu'à la zone de contrôle du dispositif.

Le « potentiel zêta » est un des éléments représentatifs de la stabilité d'une suspension. Il peut être par exemple directement mesuré à l'aide d'un équipement de type Zetasizer Nano ZS de la société Malvern. Cet équipement mesure, à l'aide de dispositifs optiques, les vitesses de déplacement des particules en fonction du champ électrique qui leur est appliqué.

En particulier, les nanoparticules mises en œuvre selon l'invention présentent avantageusement, à l'issue de leur synthèse, un potentiel zêta, noté ζ, inférieur ou égal à -28 mV, en milieu aqueux à pH ≥ 5. En particulier, les nanoparticules présentent un potentiel zêta ζ, en milieu aqueux à pH ≥ 6,5, en particulier à pH ≥ 7, et notamment à pH ≥ 8, inférieur ou égal à - 30 mV.

Le « potentiel zêta », noté ζ, peut être défini comme la différence de potentiel existant entre le sein de la solution, et le plan de cisaillement de la particule. Il est représentatif de la stabilité d'une suspension. Le plan de cisaillement (ou rayon hydrodynamique) correspond à une sphère imaginaire autour de la particule dans laquelle le solvant bouge avec la particule lorsque les particules se déplacent dans la solution. Le potentiel zêta peut être déterminé par des méthodes connues de l'homme du métier, par exemple par déplacement de la particule avec sa couche de solubilisation dans un champ électrique.

Ce potentiel zêta négatif des nanoparticules, inférieur ou égal à -28 mV à pH ≥ 5, accroît les phénomènes de répulsion électrostatique des nanoparticules en solution aqueuse les unes par rapport aux autres, ce qui permet ainsi de supprimer les phénomènes de floculation. Il est en effet connu de façon empirique de l'homme du métier qu'un potentiel zêta de valeur absolue élevée, en particulier supérieure à 28 mV, permet en général de supprimer les effets de floculation dans des milieux à faible force ionique.

Il est entendu que les mesures du potentiel zêta sont effectuées après purification de la suspension aqueuse des particules, et donc pour une suspension aqueuse présentant une conductivité ionique strictement inférieure à 100 µS.cm⁻¹. La conductivité ionique de la suspension, permettant une appréciation du taux d'ions présents dans ladite suspension, peut être mesurée, à une température ambiante (25°C), par tout conductimètre connu.

Selon un mode de réalisation particulier, les nanoparticules luminescentes mises en œuvre selon l'invention peuvent présenter une ou plusieurs molécules de surface, permettant de favoriser leur maintien en suspension, grâce à un potentiel zêta élevé.

Selon un mode de réalisation particulier, les nanoparticules mises en œuvre selon l'invention peuvent présenter en surface des cations tétraalkylammonium. De telles nanoparticules, et leur procédé de synthèse, sont par exemple décrits dans la demande déposée sous le n° FR1754416.

A titre d'exemple, les nanoparticules photoluminescentes mises en œuvre selon l'invention peuvent être de formule Y_{0,6}Eu_{0,4}VO₄ à la surface de laquelle sont éventuellement immobilisés des cations tétraméthylammonium.

Les nanoparticules mises en œuvre selon l'invention, de formule (II) précitée, sont de nature majoritairement cristalline et polycristalline, en particulier de taille moyenne de cristallites, déduite par diffraction de rayons X, comprise entre 3 et 40 nm.

### Préparation des nanoparticules

Les nanoparticules photoluminescentes à matrice cristalline dopée par des ions de terres rares mises en œuvre selon l'invention peuvent être préparées par toute méthode conventionnelle connue de l'homme du métier.

En particulier, elles peuvent être préparées par voie de synthèse colloïdale. Les méthodes de synthèse colloïdale aqueuse sont bien connues de l'homme du métier (Bouzigues et al., ACS Nano 5, 8488-8505 (2011) [49]). Ces synthèses en milieu aqueux présentent l'avantage de s'affranchir de toute étape ultérieure de transfert de solvant.

A titre d'exemple, les nanoparticules de formule A₁₋ₓLnₓVO_{4(1-y)}(PO₄)_{y}(II) peuvent être formées par réaction de co-précipitation, en milieu aqueux, à partir de précurseurs desdits éléments A et Ln, et à partir de précurseurs d'ions orthovanadate (VO₄³⁻) et éventuellement d'ions phosphate (PO₄³⁻).

Les précurseurs des éléments A et Ln peuvent se présenter, de manière classique, sous la forme de sels desdits éléments, par exemple de nitrates, chlorures, perchlorates ou acétates, en particulier de nitrates. Les précurseurs des éléments A et Ln, et leur quantité, sont bien entendu choisis de manière adéquate au regard de la nature de la nanoparticule souhaitée.

Par exemple, la synthèse de nanoparticules de formule Y₁₋ₓEuₓVO₄(IV) peut mettre en œuvre, à titre de composés précurseurs de l'yttrium et de l'europium, les nitrates d'yttrium (Y(NO₃)₃) et d'europium (Eu(NO₃)₄).

Un tel procédé de synthèse par voie colloïdale de nanoparticules photoluminescentes mises en œuvre selon l'invention est par exemple décrit dans la demande déposée sous le n° FR1754416. De manière avantageuse, comme décrit dans la demande n° FR1754416, la réaction de co-précipitation peut être réalisée en présence d'une quantité efficace de cations tétraalkylammonium.

La synthèse de nanoparticules luminescentes mises en oeuvre selon l'invention, en particulier de tailles plus importantes, supérieures à quelques dizaines de nanomètres, peut être opérée par toute autre approche connue de l'homme du métier, par exemple par broyage.

### FONCTIONNALISATION DE SURFACE DES NANOPARTICULES LUMINESCENTES

### Couplage des nanoparticules avec un réactif de liaison

A l'instar des sondes classiquement mises en œuvre dans des dispositifs de test à flux latéral, les nanoparticules luminescentes mises en œuvre selon l'invention sont couplées à au moins un réactif de liaison spécifique de la substance à analyser.

La fonction, et par conséquent la nature, du réactif de liaison couplé aux sondes luminescentes varient selon la nature du test à diffusion capillaire, en particulier à flux latéral mis en œuvre, comme détaillé dans la suite du texte, notamment selon qu'il s'agisse d'un test dit « sandwich » ou d'un test « par compétition ».

Ainsi, par « réactif de liaison », on entend tout composé chimique, biochimique ou biologique, susceptible de se lier spécifiquement avec la substance d'intérêt biologique ou chimique dont l'identification est recherchée, dans le cadre d'un test de type « sandwich », ou avec le réactif de capture de la zone de détection en compétition avec la substance d'intérêt biologique ou chimique dont l'identification est recherchée dans le cadre d'un test « par compétition ». Comme détaillé dans la suite du texte, le réactif de liaison est également susceptible de se lier spécifiquement avec le deuxième réactif de capture immobilisé dans la zone de contrôle du dispositif de test à flux latéral.

Par « se lier » ou « liaison », on entend toute liaison forte, par exemple covalente, ou, de préférence, un ensemble de liaisons faibles, par exemple du type antigène/anticorps.

La nature du réactif de liaison couplé aux nanoparticules luminescentes mises en œuvre à titre de sondes selon l'invention est bien entendu choisie au regard de la substance à analyser dans l'échantillon.

De manière avantageuse, les nanoparticules photoluminescentes mises en œuvre selon l'invention sont parfaitement adaptées à une grande diversité de ciblages biologiques, les spécificités étant dépendantes de la nature du ou des réactifs de liaison greffés à la surface de la nanoparticule.

Le réactif de liaison peut être plus particulièrement choisi parmi un anticorps polyclonal ou monoclonal, un fragment d'anticorps, un nanobody, un antigène, un oligonucléide, un peptide, une hormone, un ligand, une cytokine, un peptidomimétique, une protéine, un carbohydrate, une protéine modifiée chimiquement, un acide nucléique modifié chimiquement, un carbohydrate modifié chimiquement qui cible une protéine de surface cellulaire connue, un aptamère, un assemblage de protéines et d'ADN/ARN ou un chloroalcane utilisé par les marquages de type HaloTag. Une approche de type SNAP-Tag ou CLIP-Tag peut également être utilisée.

Selon un mode de réalisation particulier, il s'agit d'un anticorps ou fragment d'anticorps, un peptide, un acide nucléique modifié chimiquement ou un aptamère, en particulier un anticorps.

Des fragments d'anticorps appropriés comprennent au moins un domaine variable d'une immunoglobuline, tels que des domaines variables simples Fv, scFv, Fab, (Fab')² et autres fragments protéolytiques ou des « nanobody » (anticorps à domaine unique tels que les fragments V_{H}H obtenus à partir d'anticorps de camélidés ou V_{NAR} obtenus à partir d'anticorps de poissons cartilagineux).

Le terme « anticorps » selon l'invention inclut des anticorps chimériques, des anticorps humains ou humanisés, des anticorps recombinants et modifiés, des anticorps conjugués, et leurs fragments.

Le réactif de liaison peut également être dérivé d'une molécule connue pour lier un récepteur de surface cellulaire. Par exemple, le fragment de ciblage peut dériver de lipoprotéines de faible densité, transferrine, EGF, insuline, PDGF, enzymes fibrinolytiques, anti-HER2, anti-HER3, anti-HER4, annexines, interleukines, interférons, érythropoïétines ou facteurs stimulant les colonies.

### Couplage de la particule avec le réactif de liaison

Il appartient à l'homme du métier de mettre en œuvre les méthodes de couplage/greffage adaptées pour préparer de manière adéquate les particules couplées à un ou plusieurs réactifs de liaison. La quantité de réactif(s) de liaison mise en œuvre pour la fonctionnalisation de surface des nanoparticules luminescentes est ajustée au regard de la quantité de particules.

Typiquement, il est souhaitable que chaque nanoparticule soit couplée à plusieurs réactifs de liaison, de préférence au moins cinq réactifs de liaison, et plus préférentiellement au moins dix réactifs de liaison.

Le réactif de liaison peut être greffé directement, ou *via* un espaceur (encore désigné sous les termes « linker » ou « spacer »), à la nanoparticule.

Les méthodes de couplage (encore appelé greffage) des particules à des biomolécules sont bien connues de l'homme de l'art. Il s'agit en général d'un couplage par liaison covalente, par complexation de surface, par interactions électrostatiques, par encapsulation, ou par adsorption.

Dans certains cas, dont le cas d'un couplage par liaison covalente, les particules peuvent être préalablement fonctionnalisées par des groupements chimiques capables ensuite de réagir avec un autre groupement chimique porté par le réactif de liaison pour former une liaison covalente.

Comme exemples de groupements chimiques pouvant être présents à la surface des nanoparticules, on peut citer les groupements carboxyle, amino, thiol, aldéhyde et époxy.

Des groupements amino peuvent être apportés par des molécules comme les organosilane aminés, tel que l'aminotriéthoxysilane (APTES). L'avantage de l'APTES réside dans le fait qu'il forme *via* des liaisons covalentes une capsule autour de la nanoparticule. Les amines apportés par l'APTES sont ainsi très stables dans le temps. Les groupements amino peuvent être transformés en groupements carboxyle par réaction avec de l'anydride succinique.

Des groupements carboxyle peuvent être apportés par des molécules telles que l'acide citrique ou un polyacide acrylique (PAA).

Les groupements carboxyle peuvent être activés selon toute technique connue de l'homme de l'art, en particulier par réaction avec le 1-éthyl-3-(3-diméthylaminopropyl)carbodiimide (EDC) et N-hydroxysuccinimide (NHS), pour réagir ensuite avec les fonctions amines à la surface d'un polypeptide et former une liaison amide covalente, lorsque le réactif de liaison est une protéine ou un anticorps.

La fonctionnalisation des nanoparticules par de l'APTES peut se faire avantageusement suite au recouvrement des nanoparticules par une couche de silice.

Dans d'autres cas, les particules peuvent être préalablement couplées à des molécules aptes à permettre un couplage ultérieur avec un réactif de liaison.

Par exemple, les particules peuvent être couplées à la streptavidine apte à permettre un couplage avec un agent de ciblage biotinylé.

A titre d'exemple, la demande n° FR1754416 illustre le couplage de nanoparticules avec des anticorps biotinylés, par couplage des nanoparticules couplées avec la streptavidine avec des anticorps biotinylés. Il peut être également réalisé directement par couplage des anticorps sur des nanoparticules fonctionnalisées avec de l'APTES comme cité ci-dessus (transformation des groupements amino en groupements carboxyle, activation des groupements carboxyle et réaction directe avec les groupements amino à la surface des anticorps).

Le couplage du réactif de liaison sur la surface des nanoparticules peut également se faire par toute autre méthode connue de l'homme du métier.

Il peut également se faire avantageusement par recouvrement des nanoparticules d'une couche de silice, suivi d'une réaction de recouvrement par de l'APTES, dont les fonctions amines servent à réagir avec un agent espaceur bifonctionnel comportant deux fonctions NHS. Par la suite, les nanoparticules couplées aux agents espaceurs bifonctionnels peuvent réagir avec les fonctions amines à la surface d'une protéine (anticorps, streptavidine, etc.). Ce type de procédé de couplage est notamment décrit dans les publications Casanova *et al.* ([38]) et Giaume *et al.* ([39]).

### Agent de migration

Selon la charge et la nature de leur surface, leur forme et leur taille, les nanoparticules luminescentes mises en œuvre selon l'invention peuvent également être enrobées par un agent, appelé dans la suite du texte « agent de migration », qui facilite leur migration au sein du dispositif de test à diffusion capillaire, par exemple au sein de la membrane de nitrocellulose.

L'homme du métier est à même de fonctionnaliser de manière appropriée les nanoparticules avec un ou plusieurs agents de migration. En particulier, il est entendu que cet agent de migration ne doit pas perturber le couplage des nanoparticules avec le réactif de liaison comme décrit précédemment, et en particulier l'aptitude de ce dernier, lors du test à diffusion capillaire, à se lier spécifiquement avec l'analyte ou avec le réactif de capture en compétition avec l'analyte.

Les agents de migration peuvent être notamment choisis parmi des agents de furtivité ou de passivation.

De tels agents peuvent être par exemple des chaines de polyéthylèneglycol (PEG) ou poly(oxyde d'éthylène) (PEO), en particulier silanisées ; de PEO-poly(oxyde de propylène)-PEO ; de chaînes de poly(oxyde d'éthylène) greffées par des chaînes poly(L-lysine) (« poly(L-lysine)-grafted-Poly(Ethylene Glycol) » (PLL-g-PEG)) ; de chaînes de dextrane greffées par du poly(L-lysine) ; de poly(p-xylylène) (parylène) ; des poloxamères (copolymères triblocs dont la partie centrale est un bloc oxyde de propylène et les extrémités des blocs oxyde de polyéthylène, par exemple ceux commercialisés par la société BASF sous la dénomination Pluronic^{®}) ; des poloxamines ; des polysorbates et polysaccharides (par exemple, chitosan, dextrane, acide hyaluronique et héparine), les poly(D,L-lactide-co-glycolide) (PLGA), les acides polylactiques (PLA), les acides polyglutamiques (PGA), poly(caprolactone) (PCL), les copolymères N-(2-hydroxypropyl)-méthacrylate (HPMA) et polyaminoacides ; des tensioactifs anioniques ; des tensioactifs cationiques ; des tensioactifs non ioniques et des détergents zwitterioniques.

De préférence, les agents de migration sont choisis parmi les chaines PEG silanisées, les poloxamères et les acides polylactiques (PLA). Ces agents peuvent être déposés à la surface des nanoparticules par toute approche connue de l'homme du métier. Par exemple, ils peuvent y être adsorbés ou y être fixés de manière covalente.

Le couplage des nanoparticules avec un ou plusieurs agents de migration peut être opéré en même temps que celui du ou des réactif(s) de liaison, en choisissant par exemple un agent de migration portant un groupement amino lorsque le couplage du réactif de liaison aux nanoparticules se fait par réaction sur les groupements amino du réactif de liaison. Dans ce cas, il convient d'ajuster la quantité d'agents de migration par rapport à la quantité de réactifs de liaison, de sorte à ce que la nanoparticule comporte à sa surface un nombre suffisant à la fois d'agents de migration et de réactifs de liaison.

### DISPOSITIF DE TEST A DIFFUSION CAPILLAIRE

Comme indiqué précédemment, les nanoparticules photoluminescentes telles que définies précédemment sont mises en œuvre selon l'invention à titre de sondes dans un test à diffusion capillaire, tel que défini dans les revendications, par exemple dans un test à « flux latéral ».

Le terme « flux latéral » se réfère à un flux liquide dans lequel tous les composés dissous ou dispersés sont transportés par capillarité, de préférence à des vitesses équivalentes et un débit régulier, latéralement à travers un moyen de diffusion.

Le procédé de l'invention peut être mis en œuvre avec tout dispositif de test à diffusion capillaire classique, par exemple connu pour des sondes de type nanoparticules d'or. Le dispositif de test à diffusion capillaire peut notamment adopter n'importe quelle configuration ; il peut ainsi avoir une configuration linéaire, radiale, en T, en L, en croix, etc.

Dans la suite du texte, il est plus particulièrement fait référence aux figures 1 à 3 et 5 annexées, qui se rapportent à un dispositif de test à flux latéral de type bandelette de migration.

Egalement, le dispositif mis en œuvre selon l'invention peut être adapté à un test de type « sandwich » ou, alternativement, à un test « par compétition », comme détaillé dans la suite du texte.

Typiquement, un dispositif de test à diffusion capillaire, en particulier un dispositif de test à flux latéral selon l'invention, comme représenté en Figure 1, comprend un moyen de diffusion capillaire dans une direction de référence (X), en particulier un support solide poreux (10), comprenant :
- une zone (1) de dépôt de l'échantillon liquide, et éventuellement d'un diluant ;
- une zone (2), disposée en aval de la zone de dépôt, dite « zone de marquage », chargée avec les nanoparticules inorganiques photoluminescentes selon l'invention (sondes) couplées avec au moins un réactif de liaison spécifique de la substance à analyser ;
- une zone réactionnelle (3), dite encore « zone de détection », disposée en aval de la zone (2) de marquage, dans laquelle est immobilisé au moins un réactif de capture spécifique de la substance à analyser ; et
- une zone de contrôle (4), située en aval de la zone de détection (3), dans laquelle est immobilisé au moins un deuxième réactif de capture spécifique du réactif de liaison spécifique de l'analyte.

Dans un test « sandwich », le réactif de capture spécifique de l'analyte de la zone de détection et le réactif de liaison couplé à la sonde sont choisis pour se lier respectivement et spécifiquement avec l'analyte, par exemple sur deux sites épitopiques différents de l'analyte.

Dans un test « par compétition », le réactif de liaison couplé à la sonde est identique ou analogue à l'analyte, pour se lier avec le réactif de capture de la zone de détection, en compétition avec l'analyte.

La zone de contrôle (4) de migration indique à l'utilisateur qu'au moins une partie de l'échantillon est bien passée au travers du support solide poreux du dispositif de test.

Le dispositif de test à flux latéral comprend généralement en outre un tampon absorbant (5) (« Absorbent Pad » en terminologie anglo-saxonne), disposé en aval de la zone réactionnelle et de la zone de contrôle, et dont une extrémité est en contact fluidique avec le support poreux. Le tampon absorbant entretient la migration par capillarité et réceptionne l'excès d'échantillon liquide.

Les termes « amont » et « aval » se réfèrent à la direction (X) de diffusion capillaire dans le test, cette migration s'effectuant depuis la zone de dépôt (1) (à l'extrémité fonctionnelle amont) vers la zone de détection (3), et se terminant au niveau du tampon absorbant (5) (à l'extrémité fonctionnelle aval) lorsque ce dernier est présent.

Chacune des différentes zones du support solide poreux du dispositif de test à flux latéral est en communication fluidique avec la ou les zones adjacentes.

Par « contact fluidique » entre deux éléments, on entend signifier, comme cela est usuel pour les dispositifs de test à diffusion capillaire, que les deux éléments sont en contact physique, de manière à permettre la migration d'un liquide du premier élément dans le deuxième. De manière préférée, ce contact est réalisé par chevauchement d'un élément sur l'autre, comme représenté schématiquement en Figure 1.

Par « moyen de diffusion capillaire », on entend plus particulièrement un support solide poreux (10) permettant la migration d'un liquide par simple diffusion capillaire. La porosité de ce support permet la diffusion capillaire (ou migration latérale) de l'échantillon et/ou des réactifs à l'état liquide ou humide. Le support poreux peut être choisi parmi les supports déjà mis en œuvre dans les dispositifs de test à flux latéral connus. A titre d'exemples, il peut être constitué de nitrocellulose, de polyester, des fibres de verre, des fibres de cellulose, de polyéther de sulfone (PES), d'ester de cellulose, de PVDF, etc.

Le moyen de diffusion capillaire peut être constitué d'une ou plusieurs parties distinctes, les différentes parties du support pouvant être constituées de matériaux différents. Lorsque le moyen de diffusion capillaire est constitué de différentes parties ou de différents matériaux, ces éléments sont disposés de façon à permettre la continuité de l'écoulement capillaire dans le moyen de diffusion capillaire.

Typiquement, le moyen de diffusion capillaire est constitué d'un support solide poreux allongé selon la direction (X) de diffusion capillaire.

Avantageusement, il s'agit d'un support poreux (10) en forme de bande ou de bandelette. En particulier, il peut s'agir d'une bandelette immunochromatographique constituée de plusieurs membranes superposées ou chevauchantes.

Selon un mode de réalisation particulier, le support poreux est une membrane de nitrocellulose. A titre d'exemples de membranes de nitrocellulose, on peut citer les membranes Millipore^{™} HF240, Millipore^{™} HF180, Millipore^{™} HF135, Millipore^{™} HF120, Millipore^{™} HF090, Millipore^{™} HF075, Sartorius^{™} CN140, Sartorius^{™} CN150, FF120 HP membranes (GE), FF80HP membranes (GE), AE membranes (GE), Immunopore membranes (GE).

La taille du support solide poreux d'un dispositif de test à flux latéral peut être variable. Par exemple, il peut s'agir d'une bande de 30 à 200 mm, de préférence de 60 à 100mm, de long et de 2 à 10 mm, de préférence de 4 à 5 mm, de large.

Le dispositif de test à flux latéral selon l'invention peut par exemple être constitué d'une bandelette chromatographique fixée sur un support rigide (6).

Le support rigide (6) peut être constitué de matériaux divers tels que du carton, du carton plastifié, ou plus préférentiellement de matières plastiques. De préférence, le support rigide est en polystyrène.

Avantageusement, un matériau spécifique correspond à chaque zone du moyen de diffusion capillaire.

La zone (1) de dépôt (encore connue sous l'appellation « Sample Pad » en terminologie anglo-saxonne) de l'échantillon peut être avantageusement formée en un matériau absorbant poreux. En effet, la zone de dépôt du moyen de diffusion capillaire est destinée à recevoir un échantillon liquide, par exemple être mise en contact avec un flux d'urine ou un échantillon de sang. Ce matériau est choisi parmi des absorbants adaptés connus de l'homme du métier, et déjà mis en œuvre dans les tests à flux latéral classiques.

Les nanoparticules photoluminescentes inorganiques (7) telles que décrites précédemment sont mises en œuvre au niveau de la zone de marquage (2) (encore connue sous l'appellation « Conjugate Pad » en terminologie anglo-saxonne) du moyen de diffusion capillaire, comme représenté en figures 2 et 3.

Comme indiqué précédemment, ces nanoparticules (7) sont couplées avec au moins un réactif de liaison spécifique de la substance à analyser.

Dans le cadre d'un test « sandwich », le réactif de liaison est susceptible de se lier spécifiquement avec l'analyte lors du test à flux latéral. Il peut s'agir par exemple d'un anticorps spécifique de l'analyte.

Dans le cadre d'un test « par compétition » classique, le réactif de liaison est susceptible de se lier spécifiquement avec le réactif de capture de la zone de détection (3), en compétition avec l'analyte. Le réactif de liaison peut ainsi être par exemple l'analyte lui-même ou un analogue approprié. Par analogue approprié, on entend un analogue se liant de manière spécifique au réactif de capture spécifique de l'analyte.

Le réactif de liaison, couplé aux sondes luminescentes, est également susceptible de se lier spécifiquement avec le deuxième réactif de capture (9) immobilisé dans la zone de contrôle.

De préférence, comme indiqué précédemment, les nanoparticules luminescentes présentent, en outre, à leur surface au moins un agent destiné à faciliter leur migration dans le dispositif de test à flux latéral, tel qu'un agent de furtivité ou de passivation, par exemple du polyéthylène glycol. Ces agents vont ainsi faciliter la migration des nanoparticules, le cas échéant, liées par l'intermédiaire du réactif de liaison à l'analyte, dans le support poreux, par exemple dans la membrane de nitrocellulose, jusqu'à la zone de détection (3).

Les nanoparticules photoluminescentes inorganiques couplées à au moins un réactif de liaison spécifique de l'analyte sont immobilisées à l'état sec dans le moyen de diffusion capillaire, mais elles sont libres de migrer par diffusion capillaire à l'état humide.

Ainsi, lors du test, l'échantillon qui migre par diffusion capillaire à travers le moyen de diffusion capillaire entraîne les nanoparticules couplées avec le réactif de liaison spécifique de la substance à analyser.

Un premier réactif de capture, spécifique de la substance à analyser, est immobilisé au niveau de la zone de détection (3) (encore connue sous l'appellation « Detection Pad » en terminologie anglo-saxonne) du moyen de diffusion capillaire du dispositif de test à flux latéral selon l'invention. Il est choisi de manière adéquate pour son aptitude à se lier spécifiquement avec l'analyte.

Dans le cadre d'un test « sandwich » (Figure 2), le réactif de capture de la zone de détection peut être de même nature que les réactifs de liaison tels que décrits précédemment pour le couplage aux nanoparticules photoluminescentes. Il peut s'agir par exemple d'un anticorps (8) ayant une forte affinité pour la substance à analyser.

Dans le cadre d'un test « par compétition », le réactif de capture est apte à se lier également au réactif de liaison couplé aux sondes luminescentes (par exemple identique ou analogue à l'analyte).

L'analyte (11) et le réactif de capture (8) forment typiquement un couple ligand/récepteur, antigène/anticorps, ADN/ARN, ADN/ADN ou ADN/protéine.

Ainsi, si l'analyte est un antigène ou un haptène, le réactif de capture est par exemple un anticorps spécifique de l'analyte ou, si l'analyte est un anticorps, le réactif de capture est l'antigène reconnu par l'anticorps ou un anticorps reconnaissant spécifiquement l'analyte. Si l'analyte est un acide nucléique, le réactif de capture est par exemple une sonde ADN complémentaire.

Les réactifs de capture sont déposés et immobilisés au niveau de la zone de détection, de telle façon qu'ils ne soient pas mobiles à l'état humide. Cette immobilisation peut s'effectuer selon des techniques connues de l'homme du métier, par exemple par interactions électrostatiques dans le cas de membranes de nitrocellulose ou de nylon modifié pour être chargé (« charge-modified nylon » en langue anglaise) ou par interactions hydrophobe dans le cas de membranes de poly(fluorure de vinylidène) (PVDF) ou de polyethersulfone (PES).

Selon un mode de réalisation particulier, la zone de détection (3) peut comprendre une ou plusieurs régions, séparées spatialement, sur le moyen de diffusion capillaire, par exemple sous forme de bandes (une ou plusieurs ligne(s) test « T »), fonctionnalisées avec un ou plusieurs réactifs de capture. La mise en œuvre de plusieurs « lignes test » est particulièrement intéressante dans le cadre de l'utilisation du test pour une détection multiplexée, autrement dit pour la détection simultanée de plusieurs substances dans un même échantillon.

Le dispositif de test à diffusion capillaire comprend typiquement une zone de contrôle (4), située en aval de la zone de détection (3), utilisée pour confirmer la validité du test, dans laquelle est immobilisé au moins un deuxième agent de capture (9), spécifique du réactif de liaison couplé aux sondes.

Ce deuxième agent de capture est choisi de manière appropriée pour son aptitude à se lier spécifiquement au réactif de liaison conjugué aux sondes. Il peut s'agir par exemple d'un anticorps secondaire ou d'un antigène spécifique de l'anticorps mis en œuvre comme réactif de liaison spécifique de l'analyte, dans le cadre d'un test de type « sandwich ».

Comme pour le premier réactif de capture de la zone de détection (3), ce deuxième réactif de capture est immobilisé au niveau de la zone de contrôle (4) de telle façon qu'il ne soit pas mobile à l'état humide.

Le moyen de diffusion capillaire peut être optionnellement fixé à un support solide (6) tel qu'une plaque ou une cassette, généralement en matière plastique.

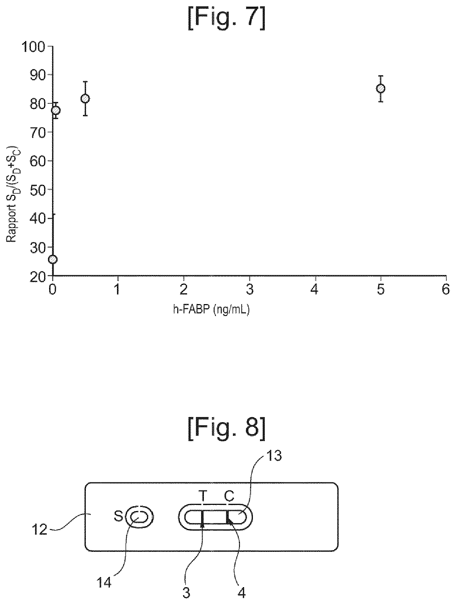

Un dispositif de test à diffusion capillaire selon l'invention peut comprendre en particulier un boîtier (12) dans lequel est placée la bandelette de test, ledit boîtier étant de préférence fermé, sauf au niveau de certaines ouvertures aménagées, comme représenté schématiquement en Figure 8. En particulier, une ouverture (14) est prévue au-dessus de la zone de dépôt de l'échantillon. Une autre ouverture, constituant la fenêtre de lecture (13), peut être par exemple prévue au niveau de la zone de détection (3) et de l'éventuelle zone de contrôle (4). Alternativement, deux fenêtres peuvent être aménagées, pour l'observation de la zone de détection et de la zone de contrôle respectivement.

Alternativement, pour rendre ces zones visibles, le boîtier peut être transparent ou muni d'une ou plusieurs parties transparentes.

Bien entendu, diverses configurations du dispositif, connues pour des dispositifs classiques de test à diffusion capillaire, peuvent être mises en œuvre. Par exemple, le boîtier comportant la bandelette de test peut comporter au niveau d'une face supérieure au moins un relief creux, dont la base vient en appui sur la surface de la bandelette, et formant un puits ou volume de dépôt pour l'échantillon liquide.

Le déroulement du test à diffusion capillaire selon le procédé de l'invention comprend plus particulièrement :
(i) l'application de l'échantillon liquide à analyser, et éventuellement d'un diluant, au niveau de la zone de dépôt (1) du dispositif de test à diffusion capillaire ;
(ii) l'incubation du dispositif jusqu'à la détection dans la zone réactionnelle (3) de la luminescence générée par les nanoparticules photoluminescences et/ou jusqu'à la détection de la luminescence dans la zone de contrôle de migration (4) ; et
(iii) la lecture et l'interprétation des résultats.

L'échantillon liquide à analyser peut être déposé directement sur la zone de dépôt (1) du moyen de diffusion capillaire du dispositif.

Par « échantillon liquide », on entend tout échantillon dans lequel la substance à analyser est en solution ou en suspension. Cet échantillon liquide peut notamment être tout fluide biologique ou corporel. L'échantillon liquide peut également avoir été obtenu à partir d'un fluide biologique ou corporel. Il peut encore s'agir d'un extrait liquide d'un échantillon solide.

Typiquement, l'échantillon liquide est de l'urine, du sang total, du plasma, du sérum,des matières fécales diluées.

Selon un mode de réalisation particulier, un diluant est utilisé avec l'échantillon à analyser, notamment lorsque l'échantillon liquide est du plasma, du sérum, du sang total, du frottis nasal ou vaginal ou de l'expectoration par exemple. Le diluant est déposé au niveau de la zone de dépôt du dispositif.

Il peut être mélangé avec l'échantillon à analyser, préalablement au dépôt de l'échantillon. Alternativement, le diluant peut être déposé avant ou après l'échantillon. Ce diluant migre dans le support poreux entraînant l'échantillon et les sondes couplées au réactif de liaison. Typiquement, ce diluant est composé d'une solution saline tamponnée. Il peut également comprendre un détergent ou tout autre composant nécessaire à la réaction.

Le dispositif de test à diffusion capillaire est ensuite incubé pendant une durée suffisante pour la migration par diffusion capillaire de l'échantillon liquide depuis la zone de dépôt jusqu'à la zone de contrôle.

Plus particulièrement, le déroulement d'un test à flux latéral de type « sandwich », schématisé en figure 2, est le suivant.

Lorsque le support poreux est mis en contact avec l'échantillon liquide contenant l'analyte (11), ce dernier migre par diffusion capillaire dans ce support jusqu'à la zone de marquage où se trouve le réactif de liaison spécifique de l'analyte couplé aux sondes (7). L'analyte (11) se lie ainsi aux sondes luminescentes (7) par l'intermédiaire du réactif de liaison.

En cas de présence de la substance à analyser, celle-ci va ensuite être immobilisée au niveau de la zone de détection (3) du dispositif de test à diffusion capillaire par le premier réactif de capture (8) fixé au niveau de cette zone. Cela va donc entrainer l'immobilisation au niveau de la zone de détection (3) des sondes luminescentes.

La présence ou l'absence de la substance à analyser dans l'échantillon est ainsi mesurée par détection des sondes luminescentes au niveau de la zone de détection (3). Plus particulièrement, la luminescente détectée au niveau de la zone de détection augmente avec, en particulier est proportionnelle à, la concentration de l'analyte dans l'échantillon.

Les sondes en excès, autrement dit les nanoparticules couplées avec un réactif de liaison n'ayant pas réagi avec l'analyte, migrent jusqu'à la zone de contrôle. Dans cette zone de contrôle, le réactif de liaison se lie au deuxième agent de capture (9), entraînant l'immobilisation des sondes en excès au niveau de la zone de contrôle (4). L'utilisateur dispose donc d'un témoin positif permettant de vérifier la migration de l'échantillon et des réactifs dans le dispositif, et donc de vérifier le bon fonctionnement du test.

Selon une autre variante du procédé de l'invention, le test mis en œuvre est de type « test par compétition ». Dans le cadre d'un test par compétition, comme schématisé en Figure 3-a, en cas d'absence de l'analyte dans l'échantillon, les sondes luminescentes seront immobilisées au niveau de la zone de détection par liaison de leur réactif de liaison avec le réactif de capture de la zone de détection. En revanche, en cas de présence de l'analyte, celui-ci se fixera, en compétition avec le réactif de liaison des sondes luminescentes, au réactif de capture de la zone de détection.

Ainsi, dans le cadre d'un test par compétition, la luminescence détectée au niveau de la zone de détection décroît avec, en particulier est inversement proportionnelle à la concentration de l'analyte dans l'échantillon.

Selon encore une autre variante d'un test de type « par compétition », comme représenté en Figure 3-b, l'analyte est déjà immobilisé au niveau des sites de capture de la zone de détection, tandis que le réactif de liaison couplé aux nanoparticules luminescentes de la zone de marquage est apte à se lier spécifiquement avec l'analyte, à l'instar d'un test « sandwich ».

Si l'échantillon contient l'analyte, celui-ci se fixe, à l'instar d'un test sandwich, aux nanoparticules luminescentes *via* le réactif de liaison, et ne peut donc pas se lier au niveau de la zone de détection. En revanche, les sondes couplées au réactif de liaison n'ayant pas réagi avec l'analyte, peuvent se lier à la zone de détection *via* l'analyte immobilisé au niveau des réactifs de capture de la zone de détection. Là encore, le signal de luminescence détecté au niveau de la zone de détection va décroître avec, en particulier sera inversement proportionnel à, la concentration de l'analyte dans l'échantillon.

La lecture des résultats, selon l'une ou l'autre des variantes précitées de test à diffusion capillaire, se fait donc par détection de la luminescence générée par les nanoparticules immobilisées, à l'issue du test, au niveau du dispositif de test à diffusion capillaire, en particulier immobilisées, à l'issue de la migration de l'échantillon au niveau de la zone de détection (3) et, éventuellement, au niveau de la zone de contrôle (4).

Bien entendu, l'invention n'est nullement limitée à la mise en œuvre d'un dispositif de test à diffusion capillaire, tel que schématisé dans les figures annexées.

D'autres variantes de dispositif de test à diffusion capillaire pour la mise en œuvre du procédé selon l'invention peuvent être envisagées, pour autant qu'elles soient adaptées à la mise en œuvre, à titre de sondes de détection, des nanoparticules photoluminescentes mises en œuvre selon l'invention. Par exemple, il peut s'agir de dispositifs de test à diffusion capillaire de type « Dipstick Lateral Flow » ou encore « Vertical Lateral Flow », etc.

Selon une variante de réalisation, il est possible de détecter plusieurs substances de l'échantillon avec un unique test (détection dite « multiplexée »).

Par exemple, dans le cadre d'un test « sandwich », il est possible d'immobiliser, au niveau de la zone réactionnelle (3), au niveau de régions distinctes (par exemple, plusieurs lignes test « T »), plusieurs réactifs de capture spécifiques de chacune des substances à analyser. Dans ce cas, les nanoparticules qui servent de sonde de détection peuvent être couplées aux deux ou plusieurs types de réactifs de capture spécifique de chacune des substances à analyser. En présence des différents analytes, les sondes vont se fixer sur chacune des régions distinctes comportant les réactifs de capture spécifiques à chaque analyte. La présence et la valeur du signal de luminescence sur chacune des zones réactionnelles correspondront à la présence et la concentration de l'analyte correspondant. Il s'agit dans ce cas d'un multiplexage spatial.

Alternativement, il est également possible de mettre en œuvre, au niveau de la zone (2) de marquage, différentes sondes dopées avec différents ions lanthanides émettant à des longueurs d'onde d'émission différentes, chacune des sondes étant couplée avec un réactif de liaison spécifique de chacune des substances à analyser et, au niveau d'une seule zone réactionnelle (3) plusieurs réactifs de capture spécifiques de chacune des substances à analyser. Il s'agit dans ce cas d'un multiplexage utilisant plusieurs couleurs d'émission. Dans ce cas, l'excitation de la matrice cristalline dans l'UV est particulièrement avantageuse car elle permet d'exciter avec la même longueur d'onde d'excitation différents ions lanthanides qui émettent à des longueurs d'onde différentes.

Les deux approches, multiplexage spatial et multiplexage à plusieurs couleurs d'émission, peuvent être combinées pour réaliser, par exemple, la détection de quatre analytes avec deux sondes à deux couleurs d'émission différentes, couplées chacune à deux types de réactifs spécifiques à deux des quatre substances à analyser et deux zones réactionnelles, chacune comportant des réactifs de liaison spécifiques de deux des quatre substances à analyser. Ainsi, le signal aux deux couleurs différentes sur la première zone réactionnelle indiquera la présence et la concentration des deux premiers analytes ; le signal aux deux couleurs différentes sur la deuxième zone réactionnelle indiquera la présence et la concentration des deux autres analytes.

### DETECTION DE LA LUMINESCENCE

A l'instar des tests classiques à diffusion capillaire, l'évaluation du test (détection et/ou quantification) mis en œuvre selon le procédé de l'invention est opérée par observation de la zone de détection et, éventuellement, de la zone de contrôle.

Plus précisément, la lecture des résultats du test se fait par détection de la luminescence générée par les sondes immobilisées au niveau de la zone de détection et/ou de la zone de contrôle, de préférence au niveau de la zone de détection et de la zone de contrôle.

### Dispositif de détection

L'observation des zones de détection et de contrôle du dispositif de test à diffusion capillaire selon l'invention met plus particulièrement en œuvre une étape (i) d'excitation des nanoparticules photoluminescentes et une étape (ii) de détection de l'émission de luminescence.

Selon un autre de ses aspects, l'invention concerne un ensemble de diagnostic *in vitro* comprenant au moins :
- un dispositif de test à diffusion capillaire selon l'invention tel que défini précédemment ; et
- un dispositif de détection de la luminescence générée par les sondes immobilisées au niveau de la zone de détection et, éventuellement, de la zone de contrôle du dispositif.

Un montage de détection simple, comprenant un dispositif d'illumination comportant une source d'excitation, permet ainsi d'observer la présence des sondes luminescentes.

L'excitation doit être compatible avec les caractéristiques d'absorbance des nanoparticules. L'excitation peut être réalisée dans l'UV, dans le visible ou dans le proche infra-rouge.

Elle peut être opérée à l'aide d'une source d'excitation non-cohérente comme une lampe, une diode électroluminescente ou un laser.

La source d'excitation peut exciter directement les ions de terres rares et/ou la matrice des nanoparticules dans laquelle sont incorporés les ions de terres rares. De préférence, les ions de terres rares sont excités par excitation de la matrice (par exemple AVO₄, ou une autre matrice d'oxyde métallique) des nanoparticules photoluminescentes immobilisées au niveau de la zone de détection et, éventuellement, de la zone de contrôle, puis transfert d'énergie ultérieure vers les ions de terres rares desdites nanoparticules. Dans la grande majorité des cas, l'excitation de la matrice est réalisée dans l'UV.

En particulier, dans le cadre de nanoparticules de formule A₁₋ₓLnₓVO_{4(1-y)}(PO₄)_{y} (II) telles que décrites précédemment, en particulier dans laquelle y vaut 0, la détection de la luminescence peut être opérée par excitation de la matrice à une longueur d'onde strictement inférieure à 350 nm, en particulier inférieure ou égale à 320 nm, et plus particulièrement inférieure ou égale à 300 nm. Dans le cadre de l'invention, l'excitation de la matrice se fait à une longueur d'onde inférieure ou égale à 320 nm.

Dans le cas de la matrice AVO₄, en particulier de la matrice YVO₄ des nanoparticules de formule Y₁₋ₓEuₓVO₄, (IV), l'excitation peut se faire à une longueur d'onde comprise entre 230 et 320 nm, en particulier entre 250 et 310 nm et plus particulièrement entre 265 et 295 nm. L'excitation de la matrice des nanoparticules est particulièrement avantageuse étant donné que le coefficient d'absorption de la matrice (ou le coefficient d'extinction pour des nanoparticules en solution) est bien supérieur à celui correspondant à l'excitation directe des ions luminescents. De plus, contre toute attente, le signal de fond parasite engendré par une excitation dans l'UV, n'est pas de nature à empêcher la détection du signal provenant de l'émission des nanoparticules, même lorsque l'analyte est présent en faible concentration, comme illustré dans les exemples qui suivent.

Dans le cas des matrices d'oxyde contenant de l'Eu, l'excitation peut se faire à une longueur d'onde comprise entre 210 et 310 nm, en particulier entre 230 et 290 nm et plus particulièrement entre 245 et 275 nm.

Dans ce cas, l'excitation peut être opérée à l'aide d'une lampe UV, d'une diode électroluminescente (LED) UV ou d'un laser UV. Les puissances et intensités d'excitation nécessaires pour une détection des sondes peuvent être aisément obtenues avec une lampe UV ou une LED UV. De préférence, l'excitation est opérée à l'aide d'une LED, cette dernière étant impliquant peu de pertes d'énergie, et ainsi peu de chaleur à évacuer.

Egalement, de manière avantageuse, l'excitation est réalisée de manière homogène sur la surface de la bandelette, en particulier au niveau des zones de détection et de contrôle. Cette homogénéité peut être atteinte par une lampe UV mais également par plusieurs LED de plus faible puissance disposées autour des zones de détection et de contrôle. A titre d'exemple, comme représenté en Figure 7, quatre groupes de quatre LED peuvent être utilisées. Le schéma de la Figure 7 indique un des schémas possibles pour disposer plusieurs LED autour des zones de détection et de contrôle de la bandelette.

La densité de puissance d'excitation peut être comprise entre 0,5 et 20 mW/cm², en particulier entre 1 et 10 mW/cm².

Dans le cadre d'une application commerciale d'un test à diffusion capillaire selon l'invention, en supposant que la chaleur produite lors de l'excitation peut être efficacement évacuée, un facteur de mérite peut être défini, tenant compte du rapport entre la sensibilité de détection obtenue avec une puissance d'excitation donnée et le coût de la source d'excitation nécessaire pour obtenir la puissance d'excitation en question. De manière avantageuse, l'ensemble de diagnostic selon l'invention permet d'optimiser ce facteur de mérite.

Selon un mode de réalisation particulièrement avantageux, la lecture des résultats, notamment dans le cadre d'une caractérisation qualitative de l'analyte, peut être opérée par une observation directe à l'œil nu, du dispositif de test à diffusion capillaire, en particulier de la zone de détection et, éventuellement, de la zone de contrôle, notamment en mettant en œuvre un filtre d'émission.

Le filtre d'émission permet de sélectionner la bande d'émission caractéristique des ions luminescents et d'exclure ainsi les signaux non spécifiques. A titre d'exemple, dans le cas de la mise en œuvre de nanoparticules Y₁₋ₓEuₓVO₄, l'intensité lumineuse émise peut être détectée à la longueur d'onde de luminescence de l'Eu³⁺ dans la matrice YVO₄, à savoir 617 nm. Le filtre d'émission peut être un filtre interférentiel ou un filtre passe haut.

Alternativement, la lecture du résultat peut être effectuée à l'aide d'un appareillage de détection simple. Celui-ci peut comprendre un filtre d'émission et un détecteur.

Le détecteur est un détecteur de photons.

Il peut s'agir d'un détecteur unique, en particulier de type photomultiplicateur, photodiode, photodiode à avalanche, ou un détecteur de type réseau de dispositifs photosensibles consistant en une surface 2D de pixels de détection tel qu'une caméra CCD ou EM-CCD ou une caméra CMOS.

De préférence, il s'agit d'un dispositif de détection, dit 2D, tel qu'une caméra. Il permet ainsi d'obtenir une image 2D de la bandelette mise en œuvre pour le test à flux latéral.

Par exemple, il peut s'agir du capteur CCD ou CMOS d'un smartphone.

De manière avantageuse, le test à diffusion capillaire selon l'invention présente une faible durée d'acquisition du signal de l'émission. En particulier, la mesure de la luminescence peut être réalisée en quelques secondes, en particulier en moins d'une seconde, en particulier en moins de 100 ms. De préférence, la durée d'acquisition du signal de l'émission doit être compatible avec le temps d'acquisition d'une image avec la caméra d'un smartphone.

### Analyse des résultats du test à flux latéral

Les résultats de luminescence peuvent ensuite être interprétés.

L'analyse des résultats du test à flux latéral peut consister en la simple détermination de la présence des sondes (mesure qualitative) au niveau de la zone de détection et/ou de contrôle, par exemple par simple observation visuelle à l'œil nu ou par lecture visuelle de l'image 2D de la bandelette, obtenue par exemple avec une caméra CCD, par exemple de la photographie enregistrée par un smartphone.

Elle permet de conclure à la présence, ou non, de la substance visée par le test au sein de l'échantillon analysé.

Par exemple, dans le cadre d'un test à flux latéral de type « sandwich », l'interprétation qualitative des résultats peut être la suivante :
. en cas de présence de deux bandes : le test est positif ;
. en cas de présence de l'unique bande de contrôle : le test est négatif ;
. en cas de présence de l'unique bande de test : le test est invalide ;
. en cas d'absence de bande : le test est invalide.

De manière avantageuse, le procédé de détection selon l'invention rend possible une mesure qualitative jusqu'à 10 fois, en particulier jusqu'à 100 fois, voire jusqu'à 1000 fois, en-dessous de la limite de détection d'un même test à diffusion capillaire mettant en œuvre, à titre de sondes, des nanoparticules d'or.

L'analyse des résultats du test à diffusion capillaire peut encore comprendre une caractérisation quantitative de la substance à analyser, autrement dit une détermination de la concentration de ladite substance au sein de l'échantillon, par interprétation des résultats de luminescence.

Le système de détection mis en œuvre selon l'invention peut alors comprendre, en outre, tout moyen permettant d'analyser l'émission de luminescence, par exemple un convertisseur permettant d'enregistrer et d'exploiter le signal de luminescence.

L'interprétation de la mesure de la luminescence peut être effectuée par référence à un étalon ou étalonnage préétablis.

Comme vu précédemment, dans le cadre d'un test de type sandwich, le signal de luminescence de la zone de détection augmente, en particulier est proportionnel, à la concentration de l'analyte, tandis qu'il pourra être inversement proportionnel dans le cadre d'un test par compétition.

Une quantification par référence à un étalonnage peut, par exemple, être réalisée grâce à plusieurs bandes de contrôle, appelées bandes de calibration, comportant différentes concentrations de la substance à analyser.

L'interprétation de la mesure de luminescence peut notamment exploiter le rapport entre le signal de luminescence de la zone de détection et celui de la zone de contrôle.

Ces moyens d'interprétation de la luminescence peuvent par exemple être réunis au sein d'une application d'un smartphone, permettant une analyse de l'image obtenue, et la donnée d'une valeur quantitative du résultat obtenu.

Alternativement, le système de détection mis en œuvre selon l'invention peut utiliser un détecteur 2D, un système d'enregistrement de l'image et un logiciel d'analyse de l'image.

Alternativement, le détecteur 2D peut être intégré dans le lecteur, et l'image enregistrée peut ensuite être transférée vers un smartphone ou un autre système permettant l'analyse de l'image.

Une analyse des résultats (*via* un logiciel d'analyse par exemple), en particulier pour une caractérisation quantitative de l'analyte, peut par exemple comprendre la détermination du signal correspondant à la zone de détection, la zone de contrôle et celle du signal de fond. La valeur de luminescence du signal de fond est soustraite à la valeur des deux autres zones. Ensuite, le rapport entre le signal de la zone de détection et le signal de la zone de contrôle est calculé.

Plus précisément, une analyse des résultats (*via* un logiciel d'analyse par exemple ou de façon avantageuse par l'intermédiaire d'une application embarquée sur le dispositif de détection (smartphone ou autres)), en particulier pour une caractérisation quantitative de l'analyte, peut par exemple comprendre (i) la détermination du niveau de luminescence dans la zone de détection, L_{D}, du niveau de luminescence dans la zone de contrôle, L_{C}, et du niveau de luminescence dans une zone sans marqueur L_{B} (signal de fond) identifiées par l'utilisateur, (ii) le calcul des signaux bruts S_{D} et S_{C} sous la forme S_{D/C}= (L_{D/C}-L_{B}), (iii) la mise en œuvre d'un algorithme de maximisation de S_{D/C} permettant une localisation optimale des zones de détection et de contrôle, (iv) le calcul du signal ratiométrique R=S_{D}/S_{C} ou R= S_{D}/(S_{D}+S_{C}) et (v) la comparaison de la valeur de R à une table d'étalonnage permettant la détermination de la concentration absolue d'analyte. Le positionnement automatisé des zones de détection (étape iii) permet de s'affranchir des biais introduits par l'utilisateur et d'obtenir une mesure quantitative reproductible. Alternativement, la position des bandes de détection et de contrôle peut être sélectionnée de manière totalement automatisée sans intervention de l'utilisateur. Dans ce dernier cas, il est important que le positionnement des bandes de détection et de contrôle sur la bandelette et le positionnement de la bandelette dans le lecteur soit toujours identique.

Dans le cadre d'un test de type « sandwich », l'analyse des résultats comprend de préférence le calcul du ratio R= S_{D}/S_{D}+S_{C}. De fait, dans le cadre d'un test de type « sandwich », plus la concentration de l'analyte est importante, plus le signal S_{D} est important et plus le signal S_{C} est faible (moins de sondes restent disponibles pour migrer jusqu'à la zone de contrôle).

L'ensemble des éléments d'excitation, de détection de la luminescence et d'analyse des résultats, peuvent être regroupés au sein d'un boitier, désigné comme le lecteur du dispositif de test à diffusion capillaire, par exemple un lecteur de bandelettes.

Une ouverture peut être par exemple être aménagée dans le lecteur de bandelettes pour insérer une ou plusieurs bandelettes à des fins de lecture du résultat du test.

Une ouverture contenant une connexion USB ou équivalent peut également être aménagée de sorte à pouvoir transférer les images enregistrées vers un appareil d'analyse des données.

Le procédé selon l'invention permet avantageusement de détecter une substance d'intérêt dans un échantillon, en une teneur strictement inférieure à 5 ng/mL, en particulier inférieure à 0,5 ng/mL, voire inférieure à 0,05 ng/mL. Ces performances dépendent bien sûr de l'analyte, ainsi que de l'efficacité du réactif de liaison spécifique utilisé.

De manière avantageuse, le procédé de détection selon l'invention rend possible une mesure quantitative jusqu'à 10 fois, en particulier jusqu'à 100 fois, voire jusqu'à 1000 fois, en-dessous de la limite de quantification d'un même test à diffusion capillaire mettant en œuvre, à titre de sondes, des nanoparticules d'or.

Les exemples et figures présentés ci-dessous sont uniquement donnés à titre illustratif et non limitatif de l'invention.

### FIGURES

Figure 1 : Représentation schématique, en vue de coupe, d'une bandelette de test à flux latéral ;
Figure 2 : Représentation schématique d'un test de type « sandwich », avant l'application d'un échantillon liquide à analyser comprenant l'analyte (11) au niveau de la zone de dépôt (1) (figure du haut) et à l'issue du test (figure du bas) ;
Figure 3 : Représentation schématique du déroulement d'un test « par compétition » suivant deux variantes, avant l'application d'un échantillon liquide à analyser comprenant l'analyte (11) au niveau de la zone de dépôt (1) (figure du haut) et à l'issue du test (figure du bas) ;
Figure 4 : Images obtenues par microscopie électronique à transmission (MET) des nanoparticules obtenues selon l'exemple 1.1.a. (Barre d'échelle : 60 nm (figure 4a) et 5 nm (figure 4b), respectivement) ;
Figure 5 : Histogramme de taille des nanoparticules déterminé à partir d'images de MET pour un ensemble d'environ 300 nanoparticules selon l'exemple 1.1.a. ;
Figure 6 : Photos de bandelettes, selon le test de l'exemple 3, suite à la migration d'une solution contenant l'antigène h-FABP à 5, 0,5, 0,05 ng/mL, illuminées par une lampe UV. On voit à gauche, la bande de détection et à droite la bande contrôle. Au bord droit des images, on voit le tampon absorbant. Les signaux de luminescence visibles sur les photos ont été analysés par ImageJ. Les résultats sont montrés sur la Figure 7.
Figure 7 : Résultats du rapport R= S_{D}/S_{D}+S_{C} mesuré par ImageJ pour des échantillons liquides contenant 5, 0,5, 0,05 et 0 ng/mL de h-FABP testés par les bandelettes de test selon l'exemple 3. Les points représentent la valeur moyenne de R et les barres d'erreur l'écart-type associé pour les 3 et 2 bandelettes, respectivement ;
Figure 8 : Représentation schématique en vue de dessus d'un boîtier comprenant une bandelette de test à flux latéral ;

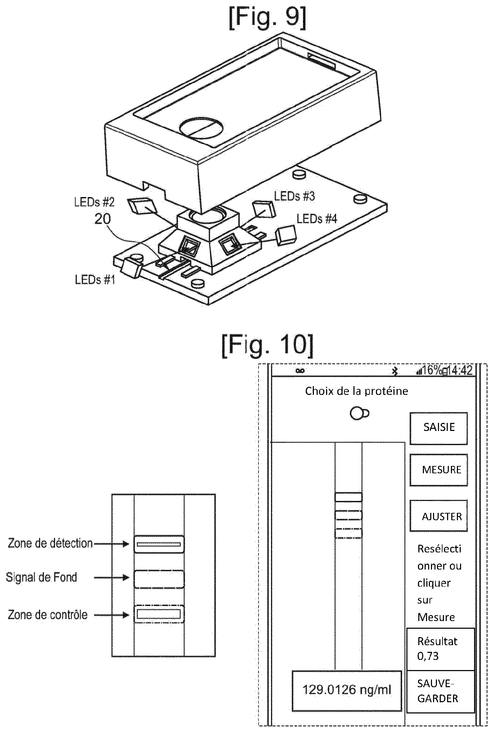

Figure 9 : Schéma du lecteur de bandelettes utilisant quatre groupes de quatre LED UV (« LEDs #1 » à (« LEDs #4 ») pour l'excitation des nanoparticules. La bandelette peut être insérée dans le lecteur au niveau du rail d'insertion (20). La lecture se fait à travers l'ouverture dans le capot, dans laquelle est positionné un filtre qui permet de sélectionner l'émission des nanoparticules (centrée à 617 nm dans le cas de l'exemple) et de rejeter la longueur d'onde d'excitation (centrée à 280 nm dans le cas de l'exemple). Il peut s'agir d'un filtre interférentiel ou d'un filtre passe-haut. Une caméra, par exemple la caméra CCD ou CMOS d'un téléphone portable, est positionnée devant cette ouverture pour l'enregistrement d'une image.
Figure 10 : Illustration de l'analyse du résultat d'une bandelette à l'aide d'une application dédiée fonctionnant sous Androïd (Samsung). Gauche : Image noir et blanc de la bandelette avec les rectangles à l'intérieur desquels sont calculés les niveaux cumulés de luminescence, de haut en bas, pour la zone de détection, la zone du signal de fond et la zone de contrôle. Droite : Photo de l'écran du téléphone portable sur lequel tourne l'application Androïd d'analyse. On distingue l'image en noir et blanc de la bandelette avec les rectangles à l'intérieur desquels est effectué le calcul du niveau cumulé de luminescence et les fonctions « Capture », «Measure », « Adjust » et « Save ».
Figure 11 : (A) Spectre d'absorbance d'une solution de nanoparticules Y_{0,6}Eu_{0,4}VO₄ synthétisées selon l'exemple. (B) Spectre d'émission d'une membrane de nitrocellulose collée sur une « backing card », telle qu'utilisée pour les tests à flux latéral de l'exemple, insérée dans une cuve de quartz, excitée à 280, 300 et 380 nm (largeur de la fente d'excitation : 5 nm). L'émission est bien plus intense suite à une excitation à 380 nm sur l'ensemble du spectre et plus particulièrement à 617 nm, la longueur d'onde où est détecté le signal des sondes à base de nanoparticules Y_{0,6}Eu_{0,4}VO₄.
Figure 12 : Spectre d'excitation des nanoparticules Y_{0,6}Eu_{0,4}VO₄ (partie gauche de la figure) avec la longueur d'onde d'émission fixée à 617 nm et spectre d'émission (partie droite de la figure) avec la longueur d'onde d'excitation fixée à 278 nm.
Figure 13 : Spectre d'excitation des nanoparticules YVO₄:Dy 5% (figure 13-a) avec la longueur d'onde d'émission fixée à 572 nm et spectre d'émission (figure 13-b) avec la longueur d'onde d'excitation fixée à 278 nm.
Figure 14 : Spectre d'excitation des nanoparticules YVO₄:Sm 3% (figure 14-a) avec la longueur d'onde d'émission fixée à 600 nm et spectre d'émission (figure 14-b) avec la longueur d'onde d'excitation fixée à 278 nm.
Figure 15 : Spectres d'excitation des nanoparticules Y_{0,6}Eu_{0,4}VO₄, Lu_{0,6}Eu_{0,4}VO₄, LuVO₄:Dy 10%, La_{0,6}Eu_{0,4}VO₄ et GdVO₄:Dy 20%, pour une longueur d'onde d'émission fixée à 617 nm pour les nanoparticules contenant des ions Eu³⁺ et à 573 nm pour les nanoparticules contenant des ions Dy³⁺.
Figure 16 : Spectre d'émission des nanoparticules Lu_{0,6}Eu_{0,4}VO₄ pour une longueur d'onde d'excitation à 278 nm (excitation de la matrice LuVO₄). L'émission présente un pic principal à 617 nm et deux autres pics à 593 et 700 nm.
Figure 17 : Spectre d'émission des nanoparticules LuVO₄:Dy 10 % pour une longueur d'onde d'excitation à 278 nm (excitation de la matrice LuVO₄). L'émission présente deux pics principaux à 483 et à 573 nm.
Figure 18 : Spectre d'émission des nanoparticules La_{0,6}Eu_{0,4}VO₄ pour une longueur d'onde d'excitation à 278 nm (excitation de la matrice LaVO₄). L'émission présente un pic principal à 617 et deux autres pics à 593 et 700 nm.
Figure 19 : Spectre d'émission des nanoparticules GdVO₄:Dy 20 % pour une longueur d'onde d'excitation à 278 nm (excitation de la matrice GdVO₄). L'émission présente deux pics principaux à 483 et à 573 nm.
Figure 20 : Spectres d'absorbance des nanoparticules Y(VO₄)_{1-y}(PO₄)_{y}:Eu 20% pour y=0, y=0,05, y=0,2, y=0,5 et y=1. Les concentrations initiales avant dilution sont de l'ordre de 50 mM en ions vanadate.
Figure 21 : Spectres d'émission des nanoparticuless Y(VO₄)_{1-y}(PO₄)_{y}:Eu 20% pour y=0, y=0,05, y=0,2, y=0,5 et y=1 pour une longueur d'onde d'excitation fixée à 278 nm. Les concentrations initiales avant dilution sont de l'ordre de 50 mM en ions vanadate.

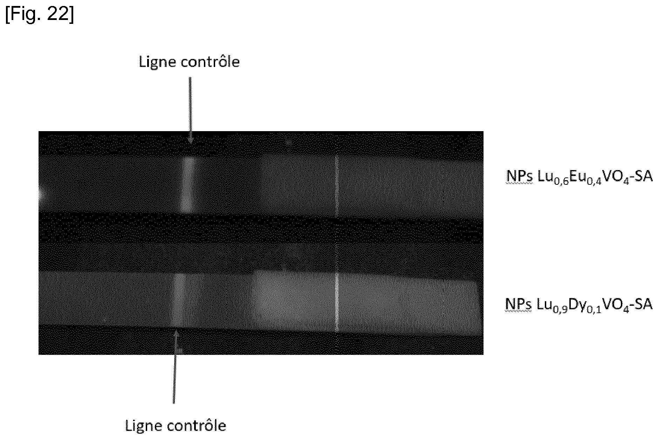

Figure 22 : Migration des nanoparticules Lu_{0,6}Eu_{0,4}VO₄-SA et Lu_{0,9}Dy_{0,1}VO₄-SA sur des bandelettes « dipstick » contenant de la BSA-Biotine immobilisée sur la ligne contrôle, en l'absence d'antigène. Les bandelettes sont observées sous illumination par lampe UV à 312 nm. Emission détectée à travers un filtre interférentiel (Semrock FF01-620/14-25 et FF03-575/25 pour l'émission des ions Eu³⁺ et Dy³⁺, respectivement) ; image prise par un smartphone Iphone 6. Deux bandes claires sont observées sur la ligne contrôle. A droite de l'image, est visible l'émission des nanoparticules ayant migré jusqu'à l'« absorbant pad ».

### EXEMPLE

### 1. Préparation des sondes photoluminescences

### 1.1. Synthèse de nanoparticules inorganiques photoluminescentes

### 1.1.a Synthèse de nanoparticules Y_{0,6}Eu_{0,4}VO₄

Comme source d'ions de métavanadate VO₃⁻, on utilise du métavanadate d'ammonium NH₄VO₃, l'orthovanadate YO₄³⁻ étant obtenu *in situ* suite à une réaction avec une base, ici l'hydroxyde de tétraméthylammonium, N(CH₃)₄OH. Des nitrates d'yttrium et d'europium ont été utilisés comme sources d'ions Y³⁺ et Eu³⁺.

Une solution aqueuse de 10 mL de NH₄VO₃ à 0,1M et 0,2 M de N(CH₃)₄OH (solution 1) est fraichement préparée.

Un volume de 10 mL d'une autre solution (solution 2) de Y(NO₃)₃ et de Eu(NO₃)₃ à 0,1 M en ions (Y³⁺ + Eu³⁺) est ajoutée goutte à goutte à l'aide d'un pousse seringue dans la solution 1 à un flux de 1 mL/min.

Le rapport en concentration molaire entre Y(NO₃)₃ et Eu(NO₃)₃ est choisi en fonction du rapport entre ions Y³⁺ et Eu³⁺ souhaité dans la nanoparticule, typiquement le rapport molaire Y³⁺ : Eu³⁺ est de 0,6 : 0,4.

Dès l'ajout de la solution Y(NO₃)₃/Eu(NO₃)₃, la solution devient diffusante et apparaît blanche/laiteuse sans formation de précipité. La synthèse se poursuit jusqu'à l'ajout total de la solution Y(NO₃)₃/Eu(NO₃)₃.

La solution finale de 20 mL doit à présent être purifiée pour éliminer l'excès de contre-ions. Pour ce faire, des centrifugations (typiquement trois) à 11000 g (Sigma 3K10, Bioblock Scientific) pendant 80 minutes suivies chacune d'une redispersion par sonification (Bioblock Scientific, Ultrasonic Processor, puissance maximale de 130 W fonctionnant à 50 % pendant 40 s) sont utilisées jusqu'à atteindre une conductivité strictement inférieure à 100 µS.cm⁻¹. La conductivité est mesurée à l'aide d'un conductimètre de chimie.

La synthèse des nanoparticules Y_{0,6}Eu_{0,4}VO₄ à la surface desquelles sont immobilisés les cations tétraméthylammonium peut être décrite comme suit :

NH₄VO₃ + 2 (N(CH₃)₄)OH ⇆ VO₄³⁻ +2 N(CH₃)₄⁺ + NH₄⁺ VO₄³⁻ + 2 N(CH₃)₄⁺ + NH₄⁺ + 0.6 Y(NO₃)₃ +0.4 Eu(NO₃)₃ →Y_{0.6}Eu_{0.4}VO₄ + 2 N(CH₃)₄⁺ + NH₄⁺ + 3NO₃⁻

L'observation visuelle de la solution de nanoparticules, après avoir été laissée au repos pendant 16 heures dans un flacon, montre une solution uniformément diffusante.

La solution finale reste très stable dans l'eau, même après plusieurs mois dans le pH final de la synthèse (environ pH 5). La solution reste stable y compris dans le milieu de synthèse (avant l'élimination de l'excès de contre-ions), de force ionique pourtant élevée (>0,1 M).

Après élimination des contre-ions, le potentiel zêta des nanoparticules, déterminé avec un appareil DLS-Zeta Potential (Zetasizer Nano ZS90, Malvern), est de -38,4 mV à pH 7.

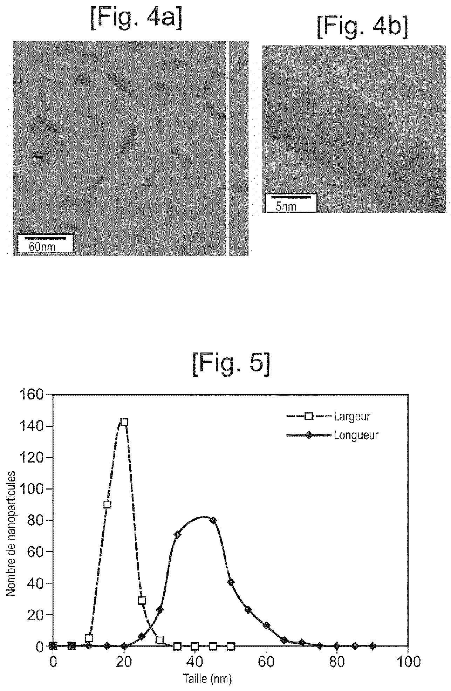

L'observation des nanoparticules par MET (figure 4) montre que les nanoparticules sont de forme éllipsoïde allongée. Les dimensions des nanoparticules sont déterminées à partir d'images de MET pour un ensemble d'environ 300 nanoparticules (figure 5). Les nanoparticules présentent une longueur du grand axe, notée *a*, comprise entre 20 et 60 nm, avec une valeur moyenne d'environ 40 nm, et une longueur du petit axe, notée b, comprise entre 10 et 30 nm, avec une valeur moyenne d'environ 20 nm.

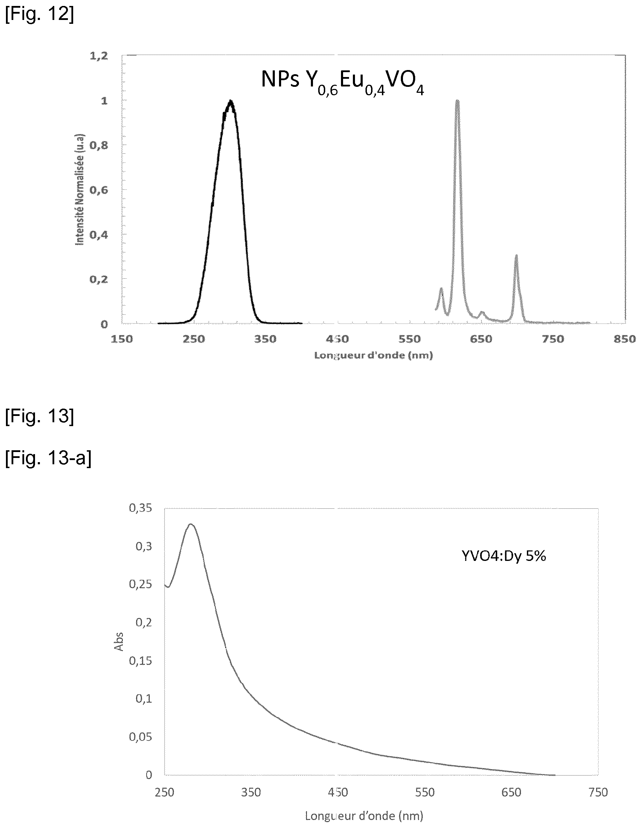

Le spectre d'excitation et d'émission des nanoparticules_Y_{0,6}Eu_{0,4}VO₄ est montré en figure 12. Le spectre d'excitation présente un pic à 278 nm et le spectre d'émission présente un pic principal à 617 nm et deux pics à 593 et 700 nm.

Les ions Eu³⁺ dans la matrice YVO₄ peuvent être remplacés par d'autres ions lanthanides luminescents. Dans ce cas, le spectre d'excitation et d'absorption autour du pic d'absorption des ions vanadate VO₄³⁻ lié à une transition de transfert de charge V-O reste inchangé. Le spectre d'émission est caractéristique du spectre d'émission de chaque ion lanthanide.

### 1.1.b Synthèse de nanoparticules Y_{0,95}Dy_{0,05} VO₄ (YVO₄:Dy (à titre illustratif seulement)

La synthèse est identique à celle de l'exemple 1.1.a. à l'exception de la solution 2 qui est constituée de Y(NO₃)₃ et de Dy(NO₃)₃ à 0,1 M en ions (Y³⁺ + Dy³⁺). La solution 2 est ajoutée goutte à goutte à l'aide d'un pousse seringue dans la solution 1 à un flux de 1 mL/min.

Le rapport en concentration molaire entre Y(NO₃)₃ et Dy(NO₃)₃ est choisi en fonction du rapport entre ions Y³⁺ et Dy³⁺ souhaité dans la nanoparticule, ici le rapport molaire Y³⁺ : Dy⁺ est de 0,95 : 0,05.

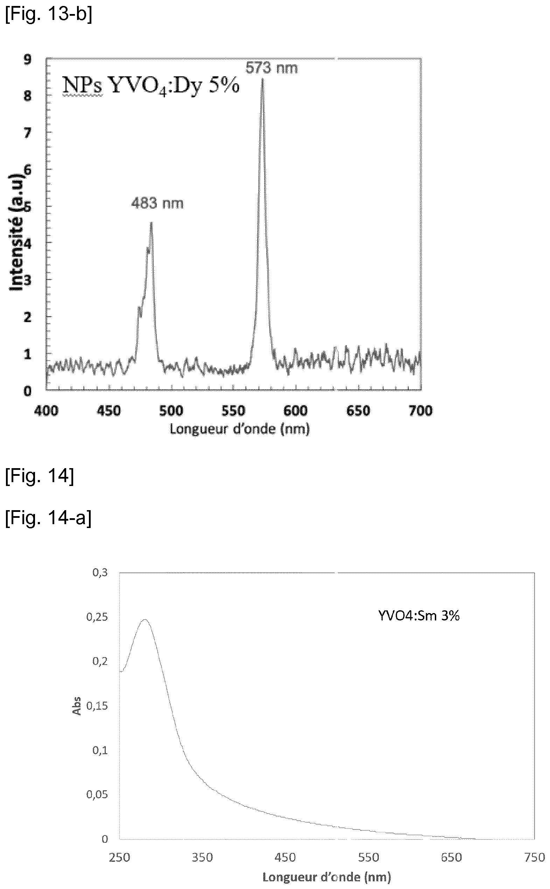

Les spectres d'excitation et d'émission de ces nanoparticules sont montrées dans la Figure 13. L'émission des ions Dy³⁺ présente deux pics principaux à 483 et à 573 nm.

### 1.1.c Synthèse de nanoparticules Y_{0,97}Sm_{0,03}VO₄ (YVO₄:Sm 3%) (à titre illustratif seulement)

La synthèse est identique à celle de l'exemple 1.1.a à l'exception de la solution 2 qui est constituée de Y(NO₃)₃ et de Sm(NO₃)₃ à 0,1 M en ions (Y³⁺ + Sm³⁺). La solution 2 est ajoutée goutte à goutte à l'aide d'un pousse seringue dans la solution 1 à un flux de 1 mL/min.

Le rapport en concentration molaire entre Y(NO₃)₃ et Sm(NO₃)₃ est choisi en fonction du rapport entre ions Y³⁺ et Sm³⁺ souhaité dans la nanoparticule, ici le rapport molaire Y³⁺ : Sm³⁺ est de 0,97 : 0,03.

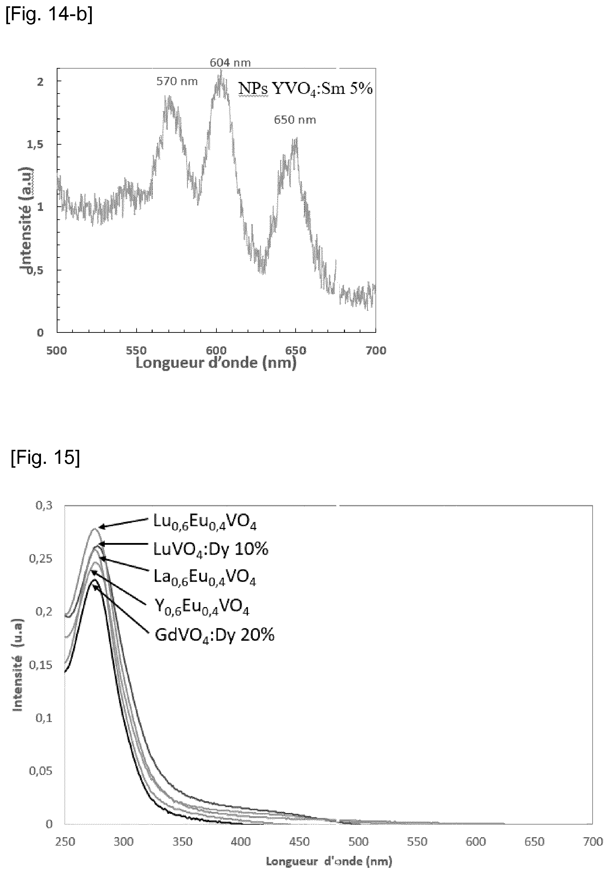

Les spectres d'excitation et d'émission de ces nanoparticules sont montrées dans la Figure 14.

Par ailleurs, les ions Y³⁺ de la matrice YVO₄ peuvent être remplacés par d'autres ions tels que Gd³⁺, Lu³⁺ et La³⁺ (voir exemples suivants). Pour toutes ces matrices GdVO₄, LuVO₄ et LaVO₄, le spectre d'excitation et d'absorption autour du pic d'absorption des ions vanadate VO₄³⁻ lié à une transition de transfert de charge V⁵⁺-O²⁻ reste inchangé par rapport à la matrice YVO₄. De plus, dans ces matrices GdVO₄, LuVO₄ et LaVO₄, les ions Eu³⁺ peuvent être remplacés par d'autres ions lanthanides luminescents. Le spectre d'émission est caractéristique du spectre d'émission de chaque ion lanthanide. Différentes combinaisons représentatives des matrices et ions lanthanides luminescents sont présentées ci-dessous.

### 1.1.d Synthèse de nanoparticules Lu_{0,6}Eu_{0,4}VO₄

La synthèse est identique à celle de l'exemple 1.1.a à l'exception de la solution 2 qui est constituée de Lu(NO₃)₃ et de Eu(NO₃)₃ à 0,1 M en ions (Lu³⁺ + Eu³⁺). La solution 2 est ajoutée goutte à goutte à l'aide d'un pousse seringue dans la solution 1 à un flux de 1 mL/min.

Le rapport en concentration molaire entre Lu(NO₃)₃ et Eu(NO₃)₃ est choisi en fonction du rapport entre ions Lu³⁺ et Eu³⁺ souhaité dans la nanoparticule, ici le rapport molaire Lu³⁺ : Eu³⁺ est de 0,6 : 0,4.

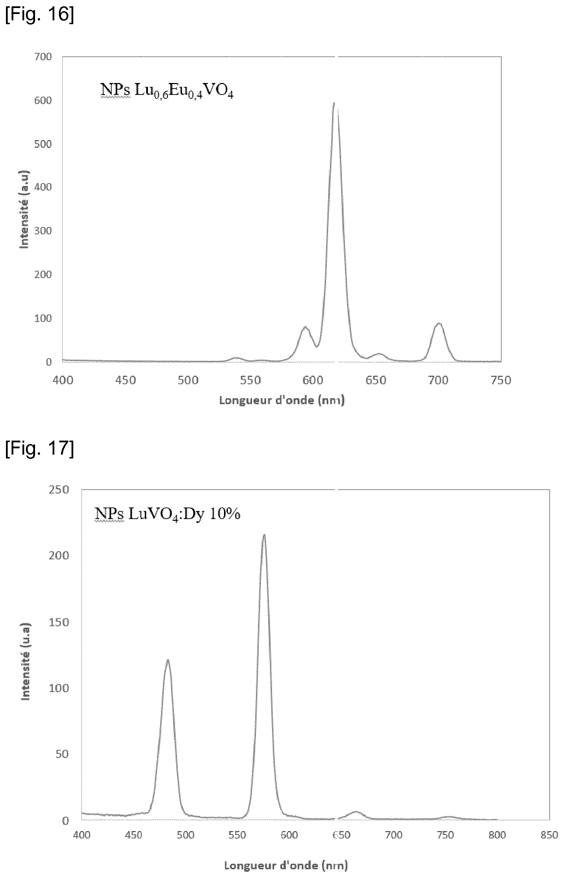

Le spectre d'excitation des nanoparticules Lu_{0,6}Eu_{0,4}VO₄ est montré dans la figure 15 et le spectre d'émission est présenté dans la figure 16. Le spectre d'émission des ions Eu³⁺ dans la matrice LuVO₄ est pratiquement inchangé par rapport à celui dans la matrice YVO₄ (figure 12) et présente un pic principal à 617 nm et deux pics à 593 et à 700 nm.

### 1.1.e Synthèse de nanoparticules LuVO₄:Dy 10%

La synthèse est identique à celle de l'exemple 1.1.a à l'exception de la solution 2 qui est constituée de Lu(NO₃)₃ et de Dy(NO₃)₃ à 0,1 M en ions (Lu³⁺ + Dy³⁺). La solution 2 est ajoutée goutte à goutte à l'aide d'un pousse seringue dans la solution 1 à un flux de 1 mL/min.

Le rapport en concentration molaire entre Lu(NO₃)₃ et Dy(NO₃)₃ est choisi en fonction du rapport entre ions Lu³⁺ et Dy³⁺ souhaité dans la nanoparticule, ici le rapport molaire Lu³⁺ : Dy³⁺ est de 0,9 : 0,1.

Le spectre d'excitation des nanoparticules LuVO₄:Dy 10% est montré dans la figure 16 et le spectre d'émission est présenté dans la figure 17. Le spectre d'émission des ions Dy³⁺ dans la matrice LuVO₄ est pratiquement inchangé par rapport à celui dans la matrice YVO₄ (figure 13) et présente deux pics d'émission à 483 et à 573 nm.

### 1.1.f Synthèse de nanoparticules La_{0,6}Eu_{0,4}VO₄

La synthèse est identique à celle de l'exemple 1.1.a à l'exception de la solution 2 qui est constituée de La(NO₃)₃ et de Eu(NO₃)₃ à 0,1 M en ions (La³⁺ + Eu³⁺). La solution 2 est ajoutée goutte à goutte à l'aide d'un pousse seringue dans la solution 1 à un flux de 1 mL/min.

Le rapport en concentration molaire entre La(NO₃)₃ et Eu(NO₃)₃ est choisi en fonction du rapport entre ions La³⁺ et Eu³⁺ souhaité dans la nanoparticule, ici le rapport molaire La³⁺ : Eu³⁺ est de 0,6 : 0,4.

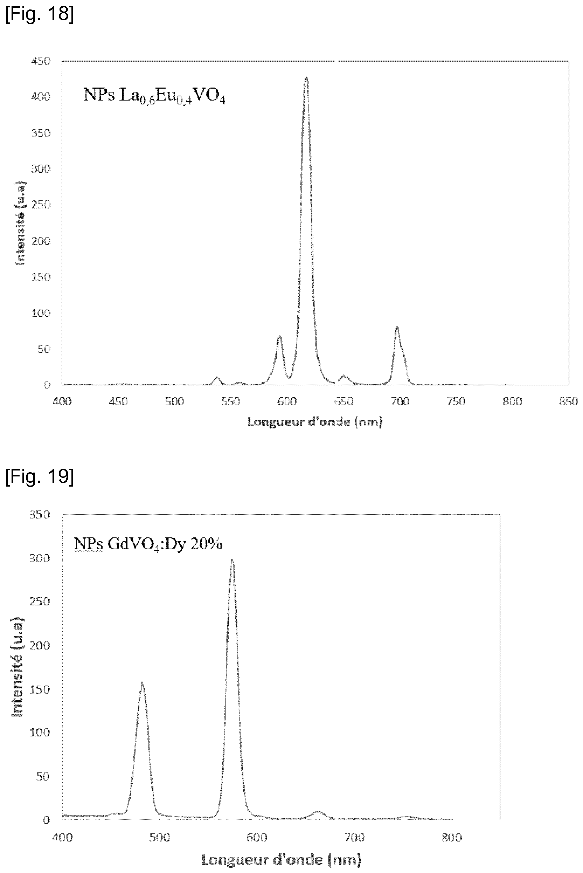

Le spectre d'excitation des nanoparticules La_{0,6}Eu_{0,4}VO₄ est montré dans la figure 15 et le spectre d'émission est présenté dans la figure 18. Le spectre d'émission des ions Eu³⁺ dans la matrice LaVO₄ est pratiquement inchangé par rapport à celui dans la matrice YVO₄ (figure 12) et présente un pic principal à 617 nm et deux pics à 593 et à 700 nm.

### 1.1.g Synthèse de nanoparticules GdVO₄:Dy 20%

La synthèse de ces nanoparticules est effectuée à partir d'un précurseur orthovanadate comme suit. Une solution aqueuse de 10 mL de NaVO₄ à 0,1M (solution 1) est fraichement préparée et son pH est ajusté entre 12.6 et 13 avec une solution de NaOH à 1 M.

Un volume de 10 mL d'une autre solution (solution 2) de Gd(NO₃)₃ et de Dy(NO₃)₃ à 0,1 M en ions (Gd³⁺ + Dy³⁺) est ajoutée goutte à goutte sous agitation à l'aide d'un pousse seringue dans la solution 1 à un flux de 1 mL/min.

Le rapport en concentration molaire entre Gd(NO₃)₃ et Dy(NO₃)₃ est choisi en fonction du rapport entre ions Gd³⁺ et Dy³⁺ souhaité dans la nanoparticule, ici le rapport molaire Gd³⁺ : Dy³⁺ est de 0,8 : 0,2.

Dès l'ajout de la solution Gd(NO₃)₃/Dy(NO₃)₃, un précipité laiteux se forme. La synthèse se poursuit jusqu'à l'ajout total de la solution Y(NO₃)₃/Eu(NO₃)₃. La solution est laissée sous agitation pendant 30 min jusqu'à ce que le pH se stabilise à 8-9.

La solution finale de 20 mL doit être purifiée, comme dans l'exemple 1.1.a pour éliminer l'excès de contre-ions. Pour ce faire, des centrifugations (typiquement trois) à 11000 g (Sigma 3K10, Bioblock Scientific) pendant 15 minutes suivies chacune d'une redispersion par sonification (Bioblock Scientific, Ultrasonic Processor avec une puissance maximale de 130 W fonctionnant à 50 % pendant 40 s) sont utilisées jusqu'à atteindre une conductivité strictement inférieure à 100 µS.cm⁻¹.

Le spectre d'excitation des nanoparticules GdVO₄:Dy 20% est montré dans la figure 15 et le spectre d'émission est présenté dans la figure 19. Le spectre d'émission des ions Dy³⁺ dans la matrice GdVO₄ est pratiquement inchangé par rapport à celui dans la matrice YVO₄ (figure 13) et présente deux pics d'émission à 483 et à 573 nm.

### 1.1.h Synthèse de nanoparticules Y(VO₄)_{1-y}(PO₄)_{y}:En 20%

Des nanoparticules contenant un mélange d'ions VO₄³⁻ et de PO₄³⁻ dans la matrice à différents ratios VO₄³⁻ : PO₄³⁻ ont également été synthétisées.

La synthèse est identique à celle de l'exemple 1.1.a à l'exception de la solution 1 qui est constituée de 0,1·y M de Na₃PO₄, de 0,1·(1-y) M NH₄VO₃ à une concentration totale de 0,1M en ions (VO₃⁻+ PO₄³⁻) et de 0,2·(1-y) M de N(CH₃)₄OH. Une solution aqueuse de 10 mL avec les concentrations ci-dessus (solution 1) est fraichement préparée. Des NPs avec y=0, y=0,05, y=0,2, y=0,5 et y=1 ont été préparées.

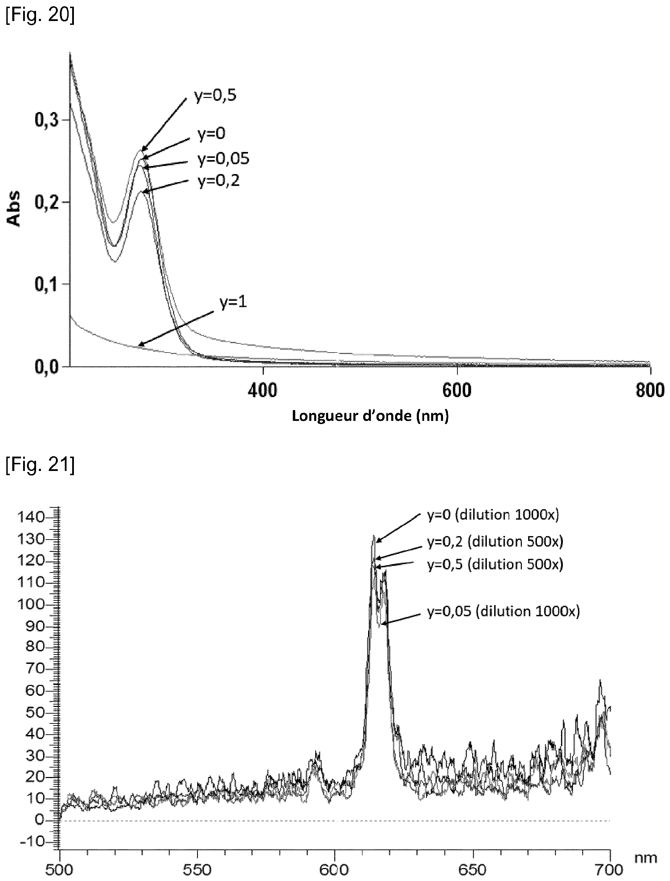

Les ions PO₄³⁻ ne présentent pas d'absorption à 278 nm. Ainsi, les nanoparticules contenant 100% d'ions PO₄³⁻ ne présentent pas de pic d'absorption à 278 nm (voir figure 20). Les spectres d'émission de ces nanoparticules sont présentés dans la figure 21 et sont identiques pour toutes les valeurs de y différentes de 1 (pas d'émission observée pour y=1). Ils présentent un pic principal à 617 et deux pics supplémentaires à 593 et 700 nm. Ces spectres d'émission sont pratiquement identiques.

### 1.2. Couplage covalent des nanoparticules avec des protéines (anticorps anti-h-FABP)

Les nanoparticules Y_{0,6}Eu_{0,4}VO₄, obtenues comme décrit au point 1.1.a., sont couplées avec des anticorps selon le protocole suivant.

### 1.2.1. Recouvrement des nanoparticules par une couche de silice

A l'issue de la synthèse des nanoparticules, on centrifuge la solution de nanoparticules à 17 000 g pendant 3 minutes, pour précipiter les éventuels agrégats de nanoparticules et on récupère le surnageant. Une sélection en taille est réalisée. Pour ce faire, plusieurs centrifugations à 1900 g pendant 3 min sont réalisées. Chaque centrifugation est suivie d'une redispersion des nanoparticules au sonificateur, puis la taille des nanoparticules est déterminée à l'aide d'un appareil DLS-Zeta Potentiel (Zetasizer Nano ZS90, Malvern).

Un volume de 25 mL de particules Y_{0,6}Eu_{0,4}VO₄ de concentration 20 mM en ions vanadate, est préparé. Un volume de 2,5 mL d'une autre solution de silicate de sodium pur (Merck Millipore 1.05621.2500) est ajouté goutte à goutte à l'aide d'une pipette afin d'enrober la surface des particules. Nous laissons agir cette solution sous agitation au moins cinq heures.

La solution est ensuite purifiée afin d'enlever l'excédent de silicate et les contre-ions de sodium. La solution est centrifugée à 11000 g (Sigma 3K10, Bioblock Scientific) pendant 60 minutes puis redispersée par sonification (Bioblock Scientific, Ultrasonic Processor, fonctionnant à 50 % à une puissance de 400 W). Cette étape est répétée jusqu'à ce que la conductivité de la solution soit inférieure à 100 µS/cm.

### 1.2.2. Greffage d'amines à la surface des nanoparticules

Dans un ballon de type tricol de 500 mL, mettre 225 mL d'éthanol absolu et ajouter 265 µL de APTES (3-Aminopropyltriethoxysilane) (Mw 221,37 g/mol Sigma Aldrich), ce qui correspond à une concentration finale de 1,125 mM. Cette quantité correspond à 5 équivalents de vanadate qui sont introduits. Un réfrigérant est ensuite branché au ballon. L'ensemble est posé sur un chauffe ballon et mis sous une hotte. Le mélange est chauffé à reflux à 90°C. Sur une des entrées du tricol une solution colloïdale de nanoparticules (concentration en ions vanadate [V] = 3 mM) dans 75 mL d'eau à pH 9 est ajoutée goutte à goutte à l'aide d'une pompe péristaltique avec un débit de 1 mL/min. L'ensemble est chauffé sous agitation pendant 24 h.

Après 48 heures, nous utilisons un évaporateur rotatif (rotavapor R-100, BUCHI) afin de concentrer partiellement les nanoparticules. La solution est mise en rotation dans un ballon adapté, et chauffé dans un bain à 50°C.

La solution récupérée est purifiée par plusieurs centrifugations dans un solvant éthanol : eau (3 :1). Après la purification, un tri en taille est réalisé en suivant le protocole décrit ci-dessus.

### 1.2.3. Greffage de carboxyles à la surface des nanoparticules aminées

Avant de démarrer le greffage un transfert de solvant est réalisé.

Le protocole de greffage est le suivant.

Transférer les NPs aminées du tampon EtOH : H₂O vers le DMF ou DMSO en réalisant plusieurs centrifugations (13000 g, 90 min). Le culot est redispersé par sonification entre chaque centrifugation (20 s à 75%). Mesurer et déterminer la concentration des NPs.

Récupérer les NPS dans 5 mL de DMF puis rajouter 10% de l'acide succinique anhydride dans un bécher en verre (i.e. 0,5 g dans les 5 mL). Laisser réagir au moins toute une nuit sous atmosphère inerte tout en agitant.

Laver les NPs carboxylées au moins 2 fois par des centrifugations (13000 g pendant 60 min, Legend Micro 17R, Thermo Scientific) afin d'enlever le DMF et l'excès de l'acide succinique anhydride.

Resuspendre les particules carboxylées dans de l'eau ou du tampon MES à pH6 par sonification (Bioblock Scientific, Ultrasonic processor).

### 1.2.4. Couplage des nanoparticules avec les anticorps anti-h-FABP

Le couplage des nanoparticules greffées en surface avec du COOH est opéré suivant le protocole ci-dessous :
1. Préparer fraichement une solution mixte de EDC/Sulfo-NHS (concentration 500 et 500 mg/mL, respectivement) dans du tampon MES (pH 5-6).
2. Dans 3 mL de solution préparée précédemment, rajouter 90 nM de NPs (ici il s'agit de la concentration en nanoparticules calculée à partir de la concentration en ions vanadate selon la référence Casanova *et al.* [37]) et laisser réagir 25 min à température ambiante tout en agitant.
3. Laver rapidement les NPs par au moins 2 centrifugations (13000 g pendant 60 min, Legend Micro 17R, Thermo Scientific) avec de l'eau MilliQ pour enlever l'excès de réactifs.
4. Récupérer le dernier culot après sonification dans du tampon phosphate de sodium à pH 7,3. Rajouter la quantité de protéine (Anticorps anti h-FABP, *Ref* 4F29, 10 E1, Hytest) nécessaire en fonction du ratio recherché (Protéine : Nps), typiquement 2 µM pour un ratio de 20 :1, et 5 mg/mL de mPEG-silane (MW : 10 kD, Laysan Bio 256-586-9004).
5. Laisser réagir cette solution entre 2 et 4h à température ambiante tout en agitant.
6. Rajouter l'agent bloquant (glycine à 1%) pour qu'il réagisse avec les COOH libres et qu'il bloque les sites de réaction résiduels à la surface des NPs. Laisser réagir 30 min.
7. Laver les Nps couplées aux protéines plusieurs fois par centrifugation en utilisant des filtres à centrifuger (Amicon Ultra 0,5mL, *Ref*UFC501096, Millipore) avec du PBS pH 7,2. Transférer les NPs dans leur milieu de stockage : tampon phosphate + Tween 20 (0.05%)+ 0.1% glycin+10% glycérol. Prélever 100 µL pour les tests BCA. Le reste de la solution est aliquoté et congelé à -80°C.

De même, l'ensemble des nanoparticules synthétisées selon les exemples 1.1.b à 1.1.h peuvent être couplées avec des anticorps, de la même façon que pour les nanoparticules Y_{0,6}Eu_{0,4}VO₄.

### 1.3. Couplage passif des nanoparticules avec des protéines (anticorps anti-h-FABP)

Un couplage passif des nanoparticules avec des anticorps, à la place du couplage covalent de l'exemple 1.2, peut également être réalisé comme suit.
- Centrifuger une solution d'1 mL de nanoparticules (concentration de 5 mM en ions vanadates) 15 min à 15 000 g.
- Reprendre le culot par 800 µL d'eau MilliQ puis redisperser par sonification (Bioblock Scientific, Ultrasonic Processor, puissance maximale de 130 W fonctionnant à 50 % pendant 40 s).
- Ajouter 100 µL d'une solution d'anticorps à 250 µg/mL en tampon phosphate de potassium 2 mM pH 7.4.
- Incuber sous rotation pendant 1 heure.
- Ajouter 100 µL de tampon phosphate de potassium 20 mM pH 7.4 / 1% BSA.
- Centrifuger 15 min à 15 000 g et éliminer le surnageant.
- Reprendre le culot par 1 mL de tampon phosphate de potassium 2 mM pH 7.4 / 0.1% BSA. Redisperser par sonification (Bioblock Scientific, Ultrasonic Processor, puissance maximale de 130 W fonctionnant à 50 % pendant 40 s).
- Centrifuger 15 min à 15 000 g et éliminer le surnageant.
- Reprendre le culot par 250 µL de tampon phosphate de potassium 2 mM pH 7.4 / 0.1% BSA. Redisperser par sonification (Bioblock Scientific, Ultrasonic Processor, puissance maximale de 130 W fonctionnant à 50 % pendant 40 s).

### 2. Préparation du test sur bandelettes de type « sandwich »

Pour développer des tests rapides afin de déterminer la présence d'une protéine d'une manière qualitative ou quantitative, il faut optimiser les différents paramètres et faire des compromis entre le temps de réaction et la sensibilité du test.

La fabrication du test bandelette, tel que représenté en Figure 1, se fait grâce à la combinaison de quatre parties essentielles :
• La fibre de verre essentiellement inerte est utilisée comme zone de marquage (2) (« Conjugate Pad » en terminologie anglo-saxonne) (GFDX 103000, Millipore).
• Comme zone de dépôt (« Sample Pad » en terminologie anglo-saxonne) (1) (*Ref* CFSP173000, Millipore), nous utilisons des polyesters dont les surfaces sont modifiées. Ils ont l'avantage d'avoir de faibles interactions non-spécifique avec les protéines, une excellente force de traction et aussi une bonne maniabilité.
• La membrane de nitrocellulose (NC) (HF180MC100, Millipore) est utilisée comme moyen de diffusion capillaire (10). Elle possède des propriétés optimales pour la migration des fluides et pour l'immobilisation des protéines. La membrane de NC est collée sur un support (6) en plastique adhésif non poreux (« backing card »).
• La cellulose (CFSP173000, Millipore) est quant à elle utilisée comme tampon absorbant (5) (« Absorbent Pad » en terminologie anglo-saxonne) pour sa forte capacité absorbante.

Pour la détection de la h-FABP (« human fatty-acid binding protein ») qui est un biomarqueur cardiaque :
Avant l'assemblage des différents composants, il faut déposer les anticorps sur la membrane de NC.
1. Une solution d'anticorps monoclonaux de souris dirigés contre la h-FABP (ref 4F29, 9F3, Hytest) est diluée dans du PBS (pH 7.4) à une concentration de 1 mg/mL. Cette solution sera utilisée pour la bande de test (3). Une autre solution d'anticorps IgG polyclonal de chèvre (*Ref* ab6708, Abcam) dirigé contre les anticorps de la souris est diluée dans du PBS (pH7.4) à une concentration de 1 mg/mL. Cette dernière est utilisée pour la bande de contrôle (4).
2. Les solutions d'anticorps sont déposées sur la membrane NC en utilisant un « dispenser » (Claremont Bio Automated Lateral Flow Reagent Dispenser (ALFRD)). A l'aide d'un pousse-seringue, un volume de 0,7 µL/2 mm est déposé pour chaque bande tout au long de la membrane NC (environ 30 cm de longueur, à partir de laquelle plusieurs bandelettes seront fabriquées). Laisser sécher pendant 1 h à 37°C.
3. Après le dépôt des anticorps, incuber la membrane NC avec 1% BSA dilué dans du PBS (pH 7.4) + du tween 20 à 0.04% pendant 30 min à 37°C pour passiver les sites de fixation non occupés par des anticorps.
4. Déposer les Ac couplés aux NPs, sur la zone de marquage (« conjugate pad »), en utilisant le dispenser. Un volume de 3 µL/4 mm est appliqué tout au long de la membrane en fibre de verre. Laisser sécher 1h à température ambiante avant de bloquer avec de la BSA 1% dilué dans du PBS (pH7.4). Laisser sécher à température ambiante.

### Assemblage de la bandelette

1. Assembler les différentes structures (la cellulose qui sert de tampon absorbant et de zone de dépôt, la zone de marquage sur laquelle est déposé Ac+NPs,) sur les parties adhésives de la NC sur laquelle les Ac sont déjà immobilisés. Les composants sont fixés sur le support en plastique de la NC en suivant l'ordre suivant : zone de marquage (« conjugate pad », zone de dépôt (« sample pad ») et enfin tampon absorbant (« absorbent pad »). Pour une meilleure migration par capillarité du fluide, les différents composants sont montés de sorte à se chevaucher les uns sur les autres comme le montre l'image d'illustration (Figure 1).
2. Couper la membrane assemblée à l'aide d'un massicot en pièces individuelles de 4 mm de largeur.
3. Les bandelettes sont ensuite conservées dans des poches en aluminium en présence d'un absorbeur d'humidité (« desiccant » en anglais) dans une atmosphère avec une humidité inférieure à 30%.

### 3. Procédure des tests de bandelette

La bandelette est préparée en mettant en œuvre les nanoparticules Y_{0,6}Eu_{0,4}VO₄ couplées aux anticorps préparées en exemple 1.2.

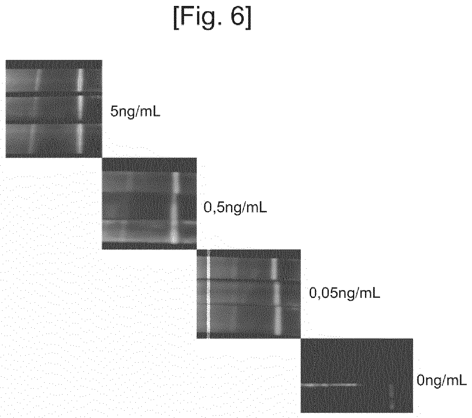

Plusieurs concentrations de h-FABP (Ref. 8F65, Hytest) ont été mesurées de 5 ng/mL à 0,05 ng/mL. Le recombinant h-FABP est dilué aux concentrations voulues avec du tampon ou avec du sérum.
1. Ramener la bandelette préparée comme décrit au point 2. ci-dessus, et les échantillons à doser à température ambiante avant la réalisation du test.
2. Déposer 400 µL de l'échantillon dans un flacon en position verticale
3. Immerger la bandelette dans le flacon en orientant la zone de dépôt (« sample pad ») vers le bas. Tapoter la bandelette au fond pour démarrer la migration. Maintenir la bandelette en position verticale dans le tube pendant 10 min.
4. Lire les résultats des bandelettes à l'aide d'une lampe UV (Vilber Lourmat, VL-8.MC 8W à 312 nm et 8 W à 254 nm) (prise de photo numérique et analyse par ImageJ, voir Figures 6 et 7) ou à l'aide du lecteur présenté dans les Figures 8 et 9. La Figure 10 illustre l'analyse du résultat d'une bandelette par une application dédiée sur un téléphone portable fonctionnant sous Androïd. Le lecteur utilise 4 groupes de 4 LED à 278 nm et un filtre interférentiel (620/15, Semrock) pour la détection. Un filtre passe-haut tel qu'un filtre RG605 (Schott) peut également être utilisé pour la détection.

Le spectre d'absorption des nanoparticules est présenté dans la Figure 11 (A). Le pic d'absorption de situe à 280 nm avec une largeur à mi-hauteur d'environ 50 nm. Le spectre d'émission de la lampe UV est centré à 310 nm avec une largeur à mi-hauteur de 40 nm. Le spectre d'émission des LED UV est centré à 278 nm avec une largeur à mi-hauteur de 10 nm.

### Interprétation qualitative des résultats

L'interprétation qualitative des résultats est la suivante :
. en cas de présence de deux bandes : le test est positif
. en cas de présence de l'unique bande de contrôle : le test est négatif
. en cas de présence de l'unique bande de test : le test est invalide
. en cas d'absence de bande : le test est invalide.

### Interprétation quantitative des résultats

La figure 10 montre un exemple d'analyse quantitative à partir d'une photo numérique prise avec un téléphone portable, à l'aide d'une application dédiée. Lors de l'appui sur « Capture » sur l'écran du téléphone, l'enregistrement d'une image noir et blanc et déclenchée. Ensuite, suite à un appui sur « Measure », l'application demande à l'utilisateur de pointer avec le doigt sur l'écran du téléphone la zone de détection puis la zone contrôle pour que l'application calcule le niveau de luminescence cumulé à l'intérieur d'un rectangle contenant la zone de détection, L_{D}, et le niveau de luminescence cumulé à l'intérieur d'un rectangle de même taille contenant la zone de contrôle, L_{C}. Un appui du « Adjust » déclenche l'optimisation de la position des deux rectangles correspondant à la zone de détection et à la zone de contrôle de sorte à maximiser le signal mesuré. Le niveau d'émission cumulé à l'intérieur d'un rectangle de même taille situé au milieu de l'espace entre la zone de détection et la zone de contrôle sert à déterminer le signal de fond, L_{B}, et à calculer les signaux S_{D/C}= L_{D/C}-L_{B}. L'application calcule et affiche ensuite le rapport R=S_{D}/S_{C} ou R= S_{D}/(S_{D}+S_{C}). La valeur de R peut être comparée à une table d'étalonnage pour fournir également une valeur de concentration en ng/mL. Le résultat peut être sauvegardé avec la fonction « Save » pour une comparaison ultérieure avec des résultats suivants.

Pour le test sur bandelettes de chacun des échantillons comprenant l'antigène h-FABP, trois bandelettes ont été préparées. Pour le cas de l'échantillon ne contenant pas l'antigène, seules deux bandelettes ont été préparées.

Le graphe de la figure 7 représente les résultats obtenus pour le rapport R=S_{D}/(S_{D}+S_{C}), mesuré par ImageJ pour les différents échantillons liquides contenant 5 ; 0,5 ; 0,05 et 0 ng/mL de la h-FABP. Les points sur la figure 7 représentent les valeurs moyennes de R, et les barres d'erreur, l'écart-type associé pour les différentes bandelettes testées (trois bandelettes dans le cas des échantillons contenant de la h-FABP ; deux bandelettes dans le cas de l'échantillon n'en contenant pas).

Ainsi, le procédé selon l'invention permet ainsi avantageusement de détecter la h-FABP dans un échantillon, en une teneur inférieure ou égale à 5 ng/mL, en particulier inférieure ou égale à 0,5 ng/mL, voire jusqu'à une valeur aussi faible que 0,05 ng/mL. Autrement dit, la h-FABP peut être détectée en une teneur inférieure ou égale à 330 pM, en particulier inférieure ou égale à 33 pM, voire jusqu'à une teneur aussi faible que 3,3 pM.

### 4. Migration des nanoparticules Lu_{0,6}Eu_{0,4}VO₄-SA et Lu_{0,9}Dy_{0,1}VO₄-SA sur bandelettes « dipstick » de type « sandwich »

Des nanoparticules Lu_{0,6}Eu_{0,4}VO₄ et Lu_{0,9}Dy_{0,1}VO₄ synthétisées selon les exemples 1.1.d et 1.1.e, respectivement, ont été couplées avec de la streptavidine (SA) selon l'exemple 1.3. (couplage passif). Des bandelettes « dipstick » ont été préparées selon l'exemple 2 en immobilisant de la BSA-Biotine sur la membrane de nitrocellulose pour reconnaître les NPs couplées avec la streptavidine. Des tests bandelettes ont été réalisés avec les nanoparticules Lu_{0,6}Eu_{0,4}VO₄-SA et Lu_{0,9}Dy_{0,1}VO₄-SA selon l'exemple 3, en l'absence d'antigène. Les bandelettes ont été visualisées sous excitation par lampe UV (312 nm). Deux bandes claires et intenses sont formées au niveau de la ligne contrôle (Figure 22).

### Références

[1] Sajid et al., « Designs, formats and applications of lateral flow assay: A literature review », Journal of Saudi Chemical Society (2015) 19, 689-705;
[2] Kavosi et al., Biosens. Biolectron. 59 (2014) 389-396;
[3] http://www.rapidtest.com/pdf/Troponin_I_Serum%20_WB%20166772-1_%20(05-01-2017).pdf
[4] Huang et al., Biosensors and Bioelectronics 75 (2016) 166-180;
[5] Tang et al., 2009, Biosens. Bioelectron. 25, 514-518;
[6] Xu et al., 2014, Anal. Chem. 86, 7351-7359;
[7] Ji et al. 2015, Talanta 142, 206-2012;
[8] Der-Jiang et al, Journal of Chromatography B, 809 (2004) 37-41;
[9] Khreich et al., Anal. Biochem. 377 (2008) 182-188;
[10] Ahn et al., Clin. Chim. Acta 332 (2003) 51-59;
[11] Biosens. Bioelectron. 63 (2014) 255-261;
[12] Yang et al., Biosens. Bioelectron. 30 (2011), 145-150;
[13] Taranova et al., Biosens. Bioelectron. 63 (2015), 255-261;
[14] Wang et al., Biosens. Bioelectron. 68 (2015), 156-162;
[15] Li et al., ACS Appl. Mater Interfaces 6 (2014), 6406-6414;
[16] Duan et al., Talanta 132 (2015), 126-131;
[17] Ren et al., ACS Appl. Mater Interfaces 6 (2014), 14215-14222;
[18] Medintz et al., Nat. Mat., 2005, 4, pp. 435-446;
[19] Zhang et al., Biosensors and Bioelectronics 51 (2014) 29-35;
[20] Xia et al., Clinical Chemistry 55 :1, 179-182 (2009));
[21] Liang et al., Analytica Chimica Acta 891 (2015) 277-283;
[22] Juntunen et al., Analytical Biochem. 428 (2012) 31-38;
[23] Zhiquiang Ye et al., J. Mater. Chem., 2004, 14, 851-856;
[24] DaCosta et al., Anal.Chimica Acta 832 (2014) 1-33;
[25] Achatz et al., Top Curr Chem (2011) 300 :29-50;
[26] Lin et al., Biotechnology Advances 30 (2012) 1551-1561;
[27] Dev K. Chatterjee et al., small 2010, 6, No. 24, 2781-2795;
[28] Corstjens et al., Ann. N. Y. Acad. Sci. 1098 : 437-445;
[29] Wang et al., Analyst, 2010, 135, 1839-1854;
[30] Niedbala et al., Anal. Biochem. 293, 22-30 (2011);
[31] Maldiney *et al*., American Chemical Society : Washington, DC, 2012, Vol. 2, pp 1-2;
[32] Paterson et al., Anal. Chem. 2014, 86, 9481-9488);
[33] Min Luo et al., Materials Research Bulletin 48 (2013) 4454-4459;
[34] Takeshitaa et al., Journal of Luminescence 128 (2008), 1515-1522;
[35] Beaurepaire et al., Nano Letters 4, 2079 (2004);
[36] Yuan et al., Trends in Analytical Chemistry, 2006, 2515, pp. 490-500;
[37] Casanova et al. Appl. Phys. Lett., 2006, 89, 253103;
[38] Casanova et al., J. Am. Chem. Soc., 2007, 129, 12592;
[39] Giaume et al., Langmuir, 2008, 24, pp. 11018-11026 ;
[40] Jan W. Stouwdam et al., Langmuir 2005, 21, 7003-7008 ;
[41] Wahid et al., J. Solid State Chem. 2016, 245, 89-97 et références qui y sont citées
[42] Riwotzki et al. J. Phys. Chem. B, 1998, 102, 10129 ;
[43] Meza et al. J. Phys. Chem. A, 2014, 118, 1390 ;
[44] Dordevic et al. J. Res. Phys., 2013, 37, 47 ;
[45] Riwotzki et al., J. Phys. Chem. B, 1998, 105, pp. 12709-127 ;
[46] Huignard et al., Chem. Mater., 2000, 10, pp.1090-1094 ;
[47] Huhtinen et al., Anal. Chem. 2005, 77, 2643-2648 ;
[48] Wolfbeis et al., Angew. Chem. Int. Ed. 2002, 41, No. 23, 4495-4498;
[49] Bouzigues et al., ACS Nano 5, 8488-8505 (2011) ;
[50] Savin et al., Talanta 178, 910-915 (2018) ;
[51] Rahman et al., Journal of Colloid and Interface Science 254 (2011), 592-596.

## Revendications

1. Procédé *in vitro* de détection et/ou de quantification d'une substance d'intérêt biologique ou chimique dans un échantillon liquide, par un test à diffusion capillaire de type bandelette de migration mettant en œuvre, à titre de sondes, des nanoparticules inorganiques photoluminescentes, de formule (II) suivante :
A₁₋ₓLnₓVO_{4(1-y)}(PO₄)_{y} (II)
dans laquelle :
. A est choisi parmi l'yttrium (Y), le gadolinium (Gd), le lanthane (La), le lutécium (Lu), et leurs mélanges ;
. Ln est choisi parmi l'europium (Eu), le dysprosium (Dy), le samarium (Sm), le néodyme (Nd), l'erbium (Er), l'ytterbium (Yb), le thulium (Tm), le praséodymium (Pr), l'holmium (Ho) et leurs mélanges ;
. 0,1 ≤ x ≤ 0,9, en particulier 0,2 ≤ x ≤ 0,6 et plus particulièrement x vaut 0,4 ; et
. 0 ≤ y < 1, en particulier y vaut 0 ;
ledit procédé mettant en œuvre la détection de la luminescence, d'une durée de vie d'émission plus brève que 100 ms, des nanoparticules, après une absorption à un photon, par excitation de la matrice à une longueur d'onde inférieure ou égale à 320 nm.

2. Procédé selon la revendication précédente, dans lequel la détection de la luminescence est opérée par excitation de la matrice à une longueur d'onde comprise inférieure ou égale à 300 nm, en particulier comprise entre 250 nm et 300 nm.

3. Procédé selon l'une quelconque des revendications précédentes, dans lequel l'échantillon liquide est un échantillon biologique, en particulier un échantillon de prélèvement humain, et plus particulièrement un échantillon choisi parmi le sang, le sérum, le plasma, la salive, l'urine, le frottis nasal, le frottis vaginal, l'expectoration, la matière fécale diluée et le liquide cérébro-spinal.

4. Procédé selon l'une quelconque des revendications précédentes, pour la détection et/ou la quantification de molécules, de protéines, d'acides nucléiques, de toxines, de virus, de bactéries ou de parasites dans un échantillon, en particulier dans un échantillon biologique.

5. Procédé selon l'une quelconque des revendications précédentes, dans lequel lesdites nanoparticules photoluminescentes présentent une taille moyenne supérieure ou égale à 5 nm et strictement inférieure à 1 µm, en particulier comprise entre 10 nm et 500 nm et de préférence entre 20 nm et 200 nm.

6. Procédé selon l'une quelconque des revendications précédentes, dans lequel Ln est choisi parmi Eu, Dy, Sm, Yb, Er, Nd et leurs mélanges, en particulier parmi Eu, Dy, Sm et leurs mélanges, et plus particulièrement est Eu ;et/ou A est choisi parmi Y, Gd, La et leurs mélanges, en particulier A représente Y ou Gd, en particulier A représente Y.

7. Procédé selon l'une quelconque des revendications précédentes, dans lequel lesdites nanoparticules présentent en surface des cations tétraalkylammonium en une quantité telle que lesdites nanoparticules présentent un potentiel zêta, noté ζ, inférieur ou égal à - 28 mV, dans un milieu aqueux de pH ≥ 5, et de conductivité ionique strictement inférieure à 100 µS.cm⁻¹.

8. Procédé selon l'une quelconque des revendications précédentes, dans lequel lesdites nanoparticules sont de formule A₁₋ₓLnₓVO₄ (III), dans laquelle A, Ln et x sont tels que définis selon l'une quelconque des revendications 1, 6 et 7, en particulier de formule Y₁₋ₓEuₓVO₄ (IV), dans laquelle 0,1≤ x ≤ 0,9, en particulier 0,2≤x≤0,6 et plus particulièrement x vaut 0,4.

9. Procédé selon l'une quelconque des revendications précédentes, ledit procédé mettant en œuvre un dispositif de test à diffusion capillaire de type bandelette de migration, dans lequel lesdites nanoparticules inorganiques photoluminescentes sont couplées avec au moins un réactif de liaison spécifique de la substance à analyser, en particulier un anticorps ou fragments d'anticorps, un peptide, un acide nucléique modifié chimiquement ou un aptamère, lesdites nanoparticules inorganiques photoluminescentes étant en particulier fonctionnalisées en surface avec un ou plusieurs agents destinés à faciliter leur migration au sein du dispositif de test à diffusion capillaire, en particulier choisis parmi des agents de furtivité ou de passivation, et plus particulièrement choisis parmi les chaines PEG silanisées, les poloxamères et les acides polylactiques (PLA)..

10. Procédé selon l'une quelconque des revendications précédentes, mettant en œuvre un dispositif de test à diffusion capillaire, comprenant :
- une zone (1) de dépôt de l'échantillon liquide, et éventuellement d'un diluant ;
- une zone (2), disposée en aval de la zone de dépôt, dite « zone de marquage », chargée avec lesdites nanoparticules inorganiques photoluminescentes couplées avec au moins un réactif de liaison spécifique de la substance à analyser ;
- une zone réactionnelle (3), dite encore « zone de détection », disposée en aval de la zone (2) de marquage, dans laquelle est immobilisé au moins un réactif de capture spécifique de la substance à analyser ;
- une zone de contrôle (4), située en aval de la zone de détection, dans laquelle est immobilisé au moins un deuxième réactif de capture spécifique du réactif de liaison spécifique de la substance à analyser ; et
optionnellement, un tampon absorbant (5), disposé en aval de la zone réactionnelle et de la zone de contrôle,
ledit procédé comprenant au moins les étapes suivantes :
(i) l'application de l'échantillon liquide à analyser, et éventuellement d'un diluant, au niveau de la zone de dépôt (1) du dispositif de test à diffusion capillaire ;
(ii) l'incubation du dispositif jusqu'à la détection dans la zone réactionnelle (3) de la luminescence générée par les nanoparticules photoluminescences et/ou jusqu'à la détection de la luminescence dans la zone de contrôle de migration (4) ; et
(iii) la lecture et l'interprétation des résultats.

11. Procédé selon l'une quelconque des revendications précédentes, dans lequel la lecture des résultats du test à diffusion capillaire est opérée par détection de la luminescence générée par les sondes immobilisées, à l'issue du test, au niveau du dispositif de test à diffusion capillaire, en particulier immobilisées au niveau de la zone de détection (3) et, éventuellement au niveau de la zone de contrôle (4), du dispositif tel que défini en revendication 10, la lecture des résultats du test à diffusion capillaire étant plus particulièrement opérée par observation directe à l'œil nu du dispositif de test à diffusion capillaire, en particulier de la zone de détection et, éventuellement, de la zone de contrôle du dispositif de test à diffusion capillaire tel que défini en revendication 10, en particulier en mettant en œuvre un filtre d'émission ;
ou à l'aide d'un appareillage de détection comprenant un filtre d'émission et un détecteur de photons, en particulier une caméra CCD ou CMOS..

12. Procédé selon l'une quelconque des revendications précédentes, dans lequel lesdites nanoparticules sont de formule Y₁₋ₓEuₓVO₄, (IV), la détection de la luminescence étant opérée par excitation de la matrice YVO₄ à une longueur d'onde comprise entre 230 et 320 nm, en particulier entre 250 et 310 nm et plus particulièrement entre 265 et 295 nm.

13. Procédé selon l'une quelconque des revendications précédentes, dans lequel l'interprétation des résultats, en particulier pour obtenir une caractérisation quantitative de ladite substance d'intérêt, comprend la détermination du signal correspondant à la zone de détection, la zone de contrôle et du signal de fond du dispositif de test à diffusion capillaire tel que défini en revendication 10, la soustraction de la valeur de luminescence du signal de fond puis la détermination du rapport entre le signal de la zone de détection et le signal de la zone de contrôle.

14. Dispositif de test à diffusion capillaire de type bandelette de migration, utile pour la détection et/ou quantification d'une substance d'intérêt biologique ou chimique dans un échantillon liquide, ledit dispositif comprenant,
- une zone (1) de dépôt de l'échantillon liquide, et éventuellement d'un diluant ;
- une zone (2), disposée en aval de la zone de dépôt, dite « zone de marquage », chargée avec lesdites nanoparticules inorganiques photoluminescentes couplées avec au moins un réactif de liaison spécifique de la substance à analyser ;
- une zone réactionnelle (3), dite encore « zone de détection », disposée en aval de la zone (2) de marquage, dans laquelle est immobilisé au moins un réactif de capture spécifique de la substance à analyser ;
- une zone de contrôle (4), située en aval de la zone de détection, dans laquelle est immobilisé au moins un deuxième réactif de capture spécifique du réactif de liaison spécifique de la substance à analyser , et comprenant, à titre de sondes, des nanoparticules inorganiques photoluminescentes de formule (II) suivante :
A₁₋ₓLnₓVO_{4(1-y)}(PO₄)_{y} (II)
dans laquelle :
. A est choisi parmi l'yttrium (Y), le gadolinium (Gd), le lanthane (La), le lutécium (Lu), et leurs mélanges ;
. Ln est choisi parmi l'europium (Eu), le dysprosium (Dy), le samarium (Sm), le néodyme (Nd), l'erbium (Er), l'ytterbium (Yb), le thulium (Tm), le praséodymium (Pr), l'holmium (Ho) et leurs mélanges ;
. 0,1≤ x ≤ 0,9, en particulier 0,2 ≤ x ≤ 0,6 et plus particulièrement x vaut 0,4 ; et
. 0 ≤ y < 1, en particulier y vaut 0 ;
lesdites nanoparticules étant aptes à émettre de la luminescence, d'une durée de vie d'émission plus brève que 100 ms, après absorption à un photon, par excitation de la matrice à une longueur d'onde inférieure ou égale à 320 nm, en particulier inférieure ou égale à 300 nm et plus particulièrement entre 250 et 300 nm.

15. Dispositif selon la revendication précédente, dans lequel lesdites nanoparticules sont telles que définies selon l'une quelconque des revendications 5 à 9.

16. Ensemble de diagnostic *in vitro,* comprenant au moins :
- un dispositif de test à diffusion capillaire tel que défini selon l'une quelconque des revendications 14 à 15 ; et
- un dispositif de détection de la luminescence générée par les sondes immobilisées au niveau du dispositif, à l'issue du test, en particulier au niveau de la zone de détection et/ou de la zone de contrôle du dispositif de test à diffusion capillaire.

17. Utilisation d'un procédé tel que défini selon l'une quelconque des revendications 1 à 13 ou d'un dispositif de test à diffusion capillaire tel que défini selon l'une quelconque des revendications 14 à 15, à des fins de diagnostic *in vitro.*

18. Utilisation d'un procédé tel que défini selon l'une quelconque des revendications 1 à 13 ou d'un dispositif de test à diffusion capillaire de type bandelette de migration tel que défini selon l'une quelconque des revendications 14 à 15, pour la détection et/ou quantification d'une substance d'intérêt, notamment d'un pathogène, dans un produit agroalimentaire ou dans l'environnement.

19. Utilisation d'un procédé tel que défini selon l'une quelconque des revendications 1 à 13 ou d'un dispositif de test à diffusion capillaire de type bandelette de migration tel que défini selon l'une quelconque des revendications 14 à 15, pour la détection et/ou quantification *in vitro* d'une substance chimique illicite, notamment d'une drogue, ou de toute autre substance d'intérêt pour la police ou la défense.

## Patentansprüche

1. *In*-*vitro*-Verfahren zum Nachweisen und/oder Quantifizieren einer interessierenden biologischen oder chemischen Substanz in einer flüssigen Probe mittels Kapillardiffusionstest vom Lateral-Flow-Teststreifen/Migrationsstreifen-Typ unter Verwendung von photolumineszenten anorganischen Nanopartikeln der nachstehenden Formel (II) als Sonden:
A₁₋ₓLnₓVO_{4(1-y)}(PO₄)_{y} (II)
wobei:
. A aus Yttrium (Y), Gadolinium (Gd), Lanthan (La), Lutetium (Lu) und Mischungen davon ausgewählt ist;
. Ln aus Europium (Eu), Dysprosium (Dy), Samarium (Sm), Neodym (Nd), Erbium (Er), Ytterbium (Yb), Thulium (Tm), Praseodym (Pr), Holmium (Ho) und Mischungen davon ausgewählt ist;
. 0,1 ≤ x ≤ 0,9, insbesondere 0,2 ≤ x ≤ 0,6 und im Besonderen x gleich 0,4 ist; und
. 0 ≤ y < 1, insbesondere y gleich 0 ist;
wobei das Verfahren den Nachweis der Lumineszenz, bei einer kürzeren Emissionslebensdauer als 100 ms, der Nanopartikel nach Einzelphotonenabsorption durch Anregung der Matrix bei einer Wellenlänge kleiner oder gleich 320 nm umfasst.

2. Verfahren nach dem vorhergehenden Anspruch, wobei der Nachweis der Lumineszenz durch Anregung der Matrix bei einer Wellenlänge kleiner oder gleich 300 nm, insbesondere zwischen 250 nm und 300 nm, erfolgt.

3. Verfahren nach einem der vorhergehenden Ansprüche, wobei es sich bei der flüssigen Probe um eine biologische Probe, insbesondere eine menschliche Testprobe, und im Besonderen eine Probe, ausgewählt aus Blut, Serum, Plasma, Speichel, Urin, Nasenabstrich, Vaginalabstrich, Sputum, verdünnter Fäkalsubstanz und Zerebrospinalflüssigkeit, handelt.

4. Verfahren nach einem der vorhergehenden Ansprüche zum Nachweisen und/oder Quantifizieren von Molekülen, Proteinen, Nukleinsäuren, Toxinen, Viren, Bakterien oder Parasiten in einer Probe, insbesondere in einer biologischen Probe.

5. Verfahren nach einem der vorhergehenden Ansprüche, wobei die photolumineszenten Nanopartikel eine mittlere Größe größer oder gleich 5 nm und strikt kleiner als 1 µm, insbesondere zwischen 10 nm und 500 nm und bevorzugt zwischen 20 nm und 200 nm aufweisen.

6. Verfahren nach einem der vorhergehenden Ansprüche, wobei Ln aus Eu, Dy, Sm, Yb, Er, Nd und Mischungen davon, insbesondere aus Eu, Dy, Sm und Mischungen davon, ausgewählt ist, und insbesondere Eu ist; und/oder A aus Y, Gd, La und Mischungen davon ausgewählt ist, insbesondere wobei A für Y oder Gd steht, insbesondere wobei A für Y steht.

7. Verfahren nach einem der vorhergehenden Ansprüche, wobei die Nanopartikel an der Oberfläche Tetraalkylammoniumkationen in einer solchen Menge aufweisen, dass die Nanopartikel in einem wässrigen Medium mit einem pH-Wert ≥ 5 ein Zetapotenzial, bezeichnet als ζ, kleiner oder gleich -28 mV und eine Ionenleitfähigkeit von strikt weniger als 100 µS.cm⁻¹ aufweisen.

8. Verfahren nach einem der vorhergehenden Ansprüche, wobei die Nanopartikel die Formel A₁₋ₓLnₓ VO₄ (III), wobei A, Ln und x nach einem der Ansprüche 1, 6 und 7 definiert sind, insbesondere die Formel Y₁₋ₓEuₓVO₄ (IV), wobei 0, 1≤ x ≤ 0,9, insbesondere 0,2≤x≤0,6 und im Besonderen x gleich 0,4 ist, aufweisen.

9. Verfahren nach einem der vorhergehenden Ansprüche, wobei das Verfahren eine Kapillardiffusionstestvorrichtung vom Migrationsstreifen-Typ verwendet, bei der die photolumineszenten anorganischen Nanopartikel mit mindestens einem für die zu analysierende Substanz spezifischen Bindungsreagenz, insbesondere einem Antikörper oder Antikörperfragmenten, einem Peptid, einer chemisch modifizierten Nukleinsäure oder einem Aptamer, gekoppelt sind, wobei im Besonderen die photolumineszenten anorganischen Nanopartikel an der Oberfläche mit einem oder mehreren Mitteln, die ihre Migration innerhalb der Kapillardiffusionstestvorrichtung erleichtern sollen, insbesondere ausgewählt aus Maskierungs- oder Passivierungsmitteln, und im Genaueren ausgewählt aus silanisierten PEG-Ketten, Poloxameren und Polymilchsäuren (PLA), funktionalisiert sind.

10. Verfahren nach einem der vorhergehenden Ansprüche unter Verwendung einer Kapillardiffusionstestvorrichtung, umfassend:
- eine Zone (1) für die Abscheidung der flüssigen Probe und gegebenenfalls eines Verdünnungsmittels;
- eine stromabwärts der Abscheidungszone angeordnete Zone (2), die sogenannte "Markierungszone", die mit den photolumineszenten anorganischen Nanopartikeln, die mit mindestens einem für die zu analysierende Substanz spezifischen Bindungsreagenz gekoppelt sind, beladen ist;
- eine stromabwärts der Markierungszone (2) angeordnete Reaktionszone (3), auch "Detektionszone" genannt, in der mindestens ein für die zu analysierende Substanz spezifisches Einfangreagenz immobilisiert ist;
- eine stromabwärts der Detektionszone gelegene Kontrollzone (4), in der mindestens ein zweites Einfangreagenz immobilisiert ist, das für das für die zu analysierende Substanz spezifische Bindungsreagenz spezifisch ist; und
optional ein Absorptionspad (5), das stromabwärts der Reaktionszone und der Kontrollzone angeordnet ist, wobei das Verfahren zumindest die folgenden Schritte umfasst:
(i) das Auftragen der zu analysierenden flüssigen Probe und gegebenenfalls eines Verdünnungsmittels auf Höhe der Abscheidungszone (1) der Kapillardiffusionstestvorrichtung;
(ii) das Inkubieren der Vorrichtung, bis zum Nachweis der von den photolumineszenten Nanopartikeln erzeugten Lumineszenz in der Reaktionszone (3) und/oder bis zum Nachweis der Lumineszenz in der Migrationskontrollzone (4); und
(iii) das Ablesen und Interpretieren der Ergebnisse.

11. Verfahren nach einem der vorhergehenden Ansprüche, wobei das Ablesen der Ergebnisse des Kapillardiffusionstests durch Nachweisen der Lumineszenz, die erzeugt wird von den Sonden, die am Ende des Tests in der Kapillardiffusionstestvorrichtung immobilisiert worden sind, insbesondere in Höhe der Detektionszone (3) und gegebenenfalls in Höhe der Kontrollzone (4) der Vorrichtung nach Anspruch 10 immobilisiert worden sind, erfolgt, wobei das Ablesen der Ergebnisse des Kapillardiffusionstests im Besonderen durch direkte Beobachtung der Kapillardiffusionstestvorrichtung, insbesondere der Detektionszone und gegebenenfalls der Kontrollzone der Kapillardiffusionstestvorrichtung nach Anspruch 10, mit bloßem Auge, insbesondere unter Verwendung eines Emissionsfilters, erfolgt;
oder mithilfe eines Nachweisgeräts, umfassend einen Emissionsfilter und einen Photonendetektor, insbesondere eine CCD- oder CMOS-Kamera, erfolgt.

12. Verfahren nach einem der vorhergehenden Ansprüche, wobei die Nanopartikel die Formel Y₁₋ₓEuₓVO₄ (IV) aufweisen, wobei der Nachweis der Lumineszenz durch Anregung der YVO₄-Matrix bei einer Wellenlänge zwischen 230 und 320 nm, insbesondere zwischen 250 und 310 nm und insbesondere zwischen 265 und 295 nm erfolgt.

13. Verfahren nach einem der vorhergehenden Ansprüche, wobei die Interpretation der Ergebnisse, insbesondere zum Erhalten einer quantitativen Charakterisierung der interessierenden Substanz, das Bestimmen des der Detektionszone bzw. der Kontrollzone entsprechenden Signals und des Hintergrundsignals der Kapillardiffusionstestvorrichtung nach Anspruch 10, das Subtrahieren des Hintergrundsignals vom Lumineszenzwert und anschließend das Bestimmen des Verhältnisses zwischen dem Signal der Detektionszone und dem Signal der Kontrollzone umfasst.

14. Kapillardiffusionstestvorrichtung vom Migrationsstreifen-Typ, verwendet zum Nachweisen und/oder Quantifizieren einer interessierenden biologischen oder chemischen Substanz in einer flüssigen Probe, wobei die Vorrichtung umfasst:
- eine Zone (1) für die Abscheidung der flüssigen Probe und gegebenenfalls eines Verdünnungsmittels;
- eine stromabwärts der Abscheidungszone angeordnete Zone (2), die sogenannte "Markierungszone", die mit den photolumineszenten anorganischen Nanopartikeln, die mit mindestens einem für die zu analysierende Substanz spezifischen Bindungsreagenz gekoppelt sind, beladen ist;
- eine stromabwärts der Markierungszone (2) angeordnete Reaktionszone (3), auch "Detektionszone" genannt, in der mindestens ein für die zu analysierende Substanz spezifisches Einfangreagenz immobilisiert ist;
- eine stromabwärts der Detektionszone gelegene Kontrollzone (4), in der mindestens ein zweites Einfangreagenz immobilisiert ist, spezifisch für das für die zu analysierende Substanz spezifische Bindungsreagenz, und umfassend, als Sonden, photolumineszente anorganische Nanopartikel der nachstehenden Formel (II):
A₁₋ₓLnₓVO_{4(1-y)}(PO₄)_{y} (II)
wobei:
. A aus Yttrium (Y), Gadolinium (Gd), Lanthan (La), Lutetium (Lu) und Mischungen davon ausgewählt ist;
. Ln aus Europium (Eu), Dysprosium (Dy), Samarium (Sm), Neodym (Nd), Erbium (Er), Ytterbium (Yb), Thulium (Tm), Praseodym (Pr), Holmium (Ho) und Mischungen davon ausgewählt ist;
. 0,1≤ x ≤ 0,9, insbesondere 0,2 ≤ x ≤ 0,6 und im Besonderen x gleich 0,4 ist; und
. 0 ≤ y < 1, insbesondere y gleich 0 ist;
wobei die Nanopartikel geeignet sind zum Emittieren von Lumineszenz, bei einer kürzeren Emissionslebensdauer als 100 ms, nach Einzelphotonenabsorption durch Anregung der Matrix bei einer Wellenlänge kleiner oder gleich 320 nm, insbesondere kleiner oder gleich 300 nm und insbesondere zwischen 250 und 300 nm.

15. Vorrichtung nach dem vorhergehenden Anspruch, wobei die Nanopartikel wie nach einem der Ansprüche 5 bis 9 definiert beschaffen sind.

16. Baugruppe für die In-vitro-Diagnostik, die zumindest Folgendes umfasst:
- eine Kapillardiffusionstestvorrichtung, wie nach einem der Ansprüche 14 bis 15 definiert; und
- eine Vorrichtung zum Nachweisen der Lumineszenz, die von den in der Vorrichtung immobilisierten Sonden, am Ende des Tests, insbesondere in Höhe der Detektionszone und/oder der Kontrollzone der Kapillardiffusionstestvorrichtung, erzeugt wird.

17. Verwendung eines Verfahrens nach einem der Ansprüche 1 bis 13 oder einer Kapillardiffusionstestvorrichtung nach einem der Ansprüche 14 bis 15 für Zwecke der *In-*vitro-Diagnostik.

18. Verwendung eines Verfahrens nach einem der Ansprüche 1 bis 13 oder einer Kapillardiffusionstestvorrichtung vom Migrationsstreifen-Typ nach einem der Ansprüche 14 bis 15 zum Nachweis und/oder Quantifizieren einer interessierenden Substanz, insbesondere eines Krankheitserregers, in einem Lebensmittelprodukt oder in der Umwelt.

19. Verwendung eines Verfahrens nach einem der Ansprüche 1 bis 13 oder einer Kapillardiffusionstestvorrichtung vom Migrationsstreifen-Typ nach einem der Ansprüche 14 bis 15 zum *In-vitro*-Nachweis und/oder -Quantifizieren einer illegalen chemischen Substanz, insbesondere einer Droge oder einer anderen Substanz von Interesse für die Polizei oder Verteidigung.

## Claims

1. *In-vitro* method for detecting and/or quantifying a biological or chemical substance of interest in a liquid sample, by a capillary action test of lateral flow strip type using, as probes, photoluminescent inorganic nanoparticles of the following formula (II):
A₁₋ₓLnₓVO_{4(1-y)}(PO₄)_{y} (II)
in which:
. A is selected from yttrium (Y), gadolinium (Gd), lanthanum (La), lutetium (Lu), and mixtures thereof;
. Ln is selected from europium (Eu), dysprosium (Dy), samarium (Sm), neodymium (Nd), erbium (Er), ytterbium (Yb), thulium (Tm), praseodymium (Pr), holmium (Ho) and mixtures thereof;
. 0.1 ≤ x ≤ 0.9, in particular 0.2 ≤ x ≤ 0.6 and more particularly x has a value of 0.4; and
. 0 ≤ y < 1, in particular y has a value of 0;
said method employing detection of the luminescence, with an emission lifetime shorter than 100 ms, of the nanoparticles, after one-photon absorption, by excitation of the matrix at a wavelength less than or equal to 320 nm.

2. Method according to the preceding claim, in which detection of the luminescence is effected by excitation of the matrix at a wavelength of less than or equal to 300 nm, in particular between 250 nm and 300 nm.

3. Method according to either one of the preceding claims, in which the liquid sample is a biological sample, in particular a sample taken from a human, and more particularly a sample selected from blood, serum, plasma, saliva, urine, nasal swab, vaginal smear, expectoration, diluted faecal matter and cerebrospinal fluid.

4. Method according to any one of the preceding claims, for detecting and/or quantifying molecules, proteins, nucleic acids, toxins, viruses, bacteria or parasites in a sample, in particular in a biological sample.

5. Method according to any one of the preceding claims, in which said photoluminescent nanoparticles have an average size greater than or equal to 5 nm and strictly less than 1 µm, in particular between 10 nm and 500 nm and preferably between 20 nm and 200 nm.

6. Method according to any one of the preceding claims, in which Ln is selected from Eu, Dy, Sm, Yb, Er, Nd and mixtures thereof, in particular from Eu, Dy, Sm and mixtures thereof, and more particularly is Eu; and/or A is selected from Y, Gd, La and mixtures thereof, in particular A represents Y or Gd, in particular A represents Y.

7. Method according to any one of the preceding claims, in which said nanoparticles have tetraalkylammonium cations on their surface in an amount such that said nanoparticles have a zeta potential, denoted ζ, less than or equal to - 28 mV, in an aqueous medium of pH ≥ 5, and with ionic conductivity strictly less than 100 µS.cm⁻¹.

8. Method according to any one of the preceding claims, in which said nanoparticles are of formula A₁₋ₓLnₓVO₄ (III), in which A, Ln and x are as defined according to any one of Claims 1, 6 and 7, in particular of formula Y₁₋ₓEuₓVO₄ (IV), in which 0.1 ≤ x ≤ 0.9, in particular 0.2 ≤ x ≤ 0.6 and more particularly x has a value of 0.4.

9. Method according to any one of the preceding claims, said method using a capillary action test device of lateral flow strip type, in which said photoluminescent inorganic nanoparticles are coupled to at least one binding reagent specific to the substance to be analysed, in particular an antibody or antibody fragments, a peptide, a chemically modified nucleic acid or an aptamer, said photoluminescent inorganic nanoparticles being in particular functionalized on the surface with one or more agents intended to facilitate their migration within the capillary action test device, in particular selected from stealth agents or passivating agents, and more particularly selected from silanized PEG chains, poloxamers and polylactic acids (PLA).

10. Method according to any one of the preceding claims, using a capillary action test device, comprising:
- a zone (1) for deposition of the liquid sample, and optionally of a diluent;
- a zone (2), arranged downstream of the deposition zone, called "labelling zone", loaded with said photoluminescent inorganic nanoparticles coupled to at least one binding reagent specific to the substance to be analysed;
- a reaction zone (3), also called "detection zone", arranged downstream of the labelling zone (2), in which at least one capturing reagent specific to the substance to be analysed is immobilized;
- a control zone (4), located downstream of the detection zone, in which at least one second capturing reagent specific to the binding reagent specific to the substance to be analysed is immobilized; and optionally, an absorbent pad (5), arranged downstream of the reaction zone and of the control zone,
said method comprising at least the following steps:
(i) applying the liquid sample to be analysed, and optionally a diluent, at the level of the deposition zone (1) of the capillary action test device;
(ii) incubating the device until the luminescence generated by the photoluminescent nanoparticles is detected in the reaction zone (3) and/or until the luminescence is detected in the migration control zone (4); and
(iii) reading and interpreting the results.

11. Method according to any one of the preceding claims, in which reading of the results of the capillary action test is effected by detecting the luminescence generated by the probes immobilized, at the end of the test, at the level of the capillary action test device, in particular immobilized at the level of the detection zone (3), and optionally at the level of the control zone (4), of the device as defined in Claim 10, the reading of the results of the capillary action test being more particularly effected by direct, naked eye observation of the capillary action test device, in particular of the detection zone and, optionally, of the control zone of the capillary action test device as defined in Claim 10, in particular using an emission filter;
or using detection equipment comprising an emission filter and a photon detector, in particular a CCD or CMOS camera.

12. Method according to any one of the preceding claims, in which said nanoparticles are of formula Y₁₋ₓEuₓVO₄, (IV), detection of the luminescence being effected by excitation of the YVO₄ matrix at a wavelength between 230 and 320 nm, in particular between 250 and 310 nm and more particularly between 265 and 295 nm.

13. Method according to any one of the preceding claims, in which interpretation of the results, in particular for obtaining a quantitative characterization of said substance of interest, comprises determining the signal corresponding to the detection zone, the control zone and the background signal of the capillary action test device as defined in Claim 10, subtracting the value of luminescence of the background signal and then determining the ratio of the signal from the detection zone to the signal from the control zone.

14. Capillary action test device of lateral flow strip type, useful for detecting and/or quantifying a biological or chemical substance of interest in a liquid sample, said device comprising
- a zone (1) for deposition of the liquid sample, and optionally of a diluent;
- a zone (2), arranged downstream of the deposition zone, called "labelling zone", loaded with said photoluminescent inorganic nanoparticles coupled to at least one binding reagent specific to the substance to be analysed;
- a reaction zone (3), also called "detection zone", arranged downstream of the labelling zone (2), in which at least one capturing reagent specific to the substance to be analysed is immobilized;
- a control zone (4), located downstream of the detection zone, in which at least one second capturing reagent specific to the binding reagent specific to the substance to be analysed is immobilized, and comprising, as probes, photoluminescent inorganic nanoparticles of the following formula (II):
A₁₋ₓLnₓVO_{4(1-y)}(PO₄)_{y} (II)
in which:
. A is selected from yttrium (Y), gadolinium (Gd), lanthanum (La), lutetium (Lu), and mixtures thereof;
. Ln is selected from europium (Eu), dysprosium (Dy), samarium (Sm), neodymium (Nd), erbium (Er), ytterbium (Yb), thulium (Tm), praseodymium (Pr), holmium (Ho) and mixtures thereof;
. 0.1 ≤ x ≤ 0.9, in particular 0.2 ≤ x ≤ 0.6 and more particularly x has a value of 0.4; and
. 0 ≤ y < 1, in particular y has a value of 0;
said nanoparticles being able to emit luminescence, with an emission lifetime shorter than 100 ms, after one-photon absorption, by excitation of the matrix at a wavelength less than or equal to 320 nm, in particular less than or equal to 300 nm and more particularly between 250 and 300 nm.

15. Device according to the preceding claim, in which said nanoparticles are as defined according to any one of Claims 5 to 9.

16. *In-vitro* diagnostic kit, comprising at least:
- a capillary action test device as defined according to either one of Claims 14 and 15; and
- a device for detecting the luminescence generated by the probes immobilized at the level of the device, at the end of the test, in particular at the level of the detection zone and/or control zone of the capillary action test device.

17. Use of a method as defined according to any one of Claims 1 to 13 or of a capillary action test device as defined according to either one of Claims 14 and 15, for purposes of *in-vitro* diagnostics.

18. Use of a method as defined according to any one of Claims 1 to 13 or of a capillary action test device of lateral flow strip type as defined according to either one of Claims 14 and 15, for detecting and/or quantifying a substance of interest, in particular a pathogen, in an agri-food product or in the environment.

19. Use of a method as defined according to any one of Claims 1 to 13 or of a capillary action test device of lateral flow strip type as defined according to either one of Claims 14 and 15, for detecting and/or quantifying *in-vitro* an illegal chemical substance, in particular a drug, or any other substance of interest for the police or defence.
